# EUROPEAN PATENT APPLICATION

(11) **EP 4 455 294 A1**
(43) Date of publication of application: **30.10.2024**
(21) Application number: 22912010.0
(22) Date of filing: 23.12.2022
(51) Int. Cl.: C12N 15/85

(54) **NOVEL PROMOTER AND USE THEREOF**

(30) Priority: 24.12.2021 KR 20210187163
(71) Applicant: Toolgen Incorporated, Seoul 07789 (KR); Seoul National University R&DB Foundation, Seoul 08826 (KR)
(72) Inventor: YEOM, Su Cheong, Pyeongchang-gun, Gangwon-do 25354 (KR); SONG, Dong Woo, Seoul 07789 (KR); KIM, Seokjoong, Seoul 07789 (KR); OH, Hye Kyung, Seoul 07789 (KR)
(74) Representative: HGF
(86) International application number: PCT/KR2022/021148
(87) International publication number: WO 2023/121377

(57) **Abstract**

The present application relates to a novel promoter and a use thereof. In one embodiment of the present application, provided is a nucleic acid construct comprising a novel promoter and a transgene of the present application. At this time, on the nucleic acid construct, the novel promoter of the present application can be operably linked to the transgene.

## Description

### Technical Field

The present application relates to a novel promoter and a use thereof.

### Background Art

A promoter generally refers to a region of DNA located immediately upstream of a gene to be transcribed, which is involved in the initiation of transcription of the gene. In general, transcription of a gene located downstream of a promoter begins through RNA polymerase bound to the promoter. It is known that transcription of DNA into mRNA in eukaryotes is initiated by a promoter and is regulated with the help of enhancers, silencers, and transcription factors.

The APOC3 gene refers to a gene encoding apolipoprotein C-III. The APOC3 gene is located on chromosome 11 in humans. There is the following literature regarding the function of apolipoprotein C-III: Mendivil, Carlos O., et al. "Metabolism of very-low-density lipoprotein and low-density lipoprotein containing apolipoprotein C-III and not other small apolipoproteins." Arteriosclerosis, thrombosis, and vascular biology 30.2 (2010): 239-245.

### Detailed Description of Invention

### Technical Problem

The present application provides a novel promoter. The novel promoter provided by the present application is new, not a conventional promoter. The novel promoter provided by the present application can be appropriately used in various technologies and various situations where a promoter is required.

### Solution to Problem

The present application relates to a novel promoter and a use thereof.

In one embodiment of the present application, there is provided a nucleic acid construct comprising the promoter of the present application.

In one embodiment of the present application, there is provided a nucleic acid construct for expressing transgene comprising:
a promoter comprising any one of the sequences of SEQ ID NOs: 01 to 08 and sequences having at least 80% sequence identity thereto; and
a transgene,
wherein the promoter is operably linked to the 5' terminus of the transgene.

In certain embodiments, the promoter may comprise any one of the sequence of SEQ ID NO: 01 and a sequence having at least 80% sequence identity thereto.

In certain embodiments, the promoter may comprise any one of the sequence of SEQ ID NO: 02 and a sequence having at least 80% sequence identity thereto.

In certain embodiments, the promoter may comprise any one of the sequence of SEQ ID NO: 03 and a sequence having at least 80% sequence identity thereto.

In certain embodiments, the promoter may comprise any one of the sequence of SEQ ID NO: 04 and a sequence having at least 80% sequence identity thereto.

In certain embodiments, the promoter may comprise any one of the sequence of SEQ ID NO: 05 and a sequence having at least 80% sequence identity thereto.

In certain embodiments, the promoter may comprise any one of the sequence of SEQ ID NO: 06 and a sequence having at least 80% sequence identity thereto.

In certain embodiments, the promoter may comprise any one of the sequence of SEQ ID NO: 07 and a sequence having at least 80% sequence identity thereto.

In certain embodiments, the promoter may comprise any one of the sequence of SEQ ID NO: 08 and a sequence having at least 80% sequence identity thereto.

In certain embodiments, the promoter may have a length of 1000 bp or less.

In certain embodiments, the nucleic acid construct may have a length of 10000 bp or less.

In certain embodiments, the transgene may be a nucleic acid encoding the human FIX protein.

In certain embodiments, the nucleic acid construct may further comprise any one of an upstream homology arm, a downstream homology arm, a splicing acceptor, and a Poly A sequence.

In one embodiment of the present application, there is provided a nucleic acid construct for expressing transgene comprising:
a promoter comprising any one of the sequences of SEQ ID NOs: 01 to 08 and sequences having at least 80% sequence identity thereto;
a transgene; and
an upstream homology arm designed to insert the promoter and the transgene into a target site within the genome of a cell; and
a downstream homology arm designed to insert the promoter and the transgene into a target site within the genome of a cell,
wherein the promoter is operably linked to the 5' terminus of the transgene.

In certain embodiments, on the nucleic acid construct, the promoter, the transgene, the upstream homology arm, and the downstream homology arm may be arranged in a 5' to 3' direction in the order of the upstream homology arm, the promoter, the transgene, and the downstream homology arm.

In certain embodiments, each of the upstream homology arm and the downstream homology arm may have a length of 300 bp to 1500 bp.

In certain embodiments, the target site may be located within a safe harbor gene on the genome.

In certain embodiments, the safe harbor gene may be any one selected from ALB gene, FTL gene, FTH1 gene, ACTB gene, HP gene, APOC3 gene, SOD2 gene, ORM1 gene, AAVS1 gene, Rosa gene, HPRT gene, and CCR5 gene.

In one embodiment of the present application, there is provided a nucleic acid construct for expressing transgene comprising:
an upstream homology arm comprising a promoter comprising any one of the sequences of SEQ ID NOs: 01 to 08 and sequences having at least 80% sequence identity thereto;
a transgene; and
a downstream homology arm designed to insert the transgene into a target site on the genome of a cell,
wherein the promoter is operably linked to the 5' terminus of the transgene.

In certain embodiments, the upstream homology arm may comprise a promoter comprising any one of the sequences of SEQ ID NOs: 01 to 06 and sequences having at least 80% sequence identity thereto, and the downstream homology arm may comprise any one of the sequences of SEQ ID NOs: 07 to 08 and sequences having at least 80% sequence identity thereto.

In certain embodiments, on the nucleic acid construct, the upstream homology arm, the transgene, and the downstream homology arm may be arranged in a 5' to 3' direction in the order of the upstream homology arm, the transgene, and the downstream homology arm.

In certain embodiments, the target site may be located within intron 1 of the APOC3 gene.

### Effects of Invention

The novel promoter provided by the present application allows transcription and/or expression of a transgene within a cell, regardless of whether the transgene is inserted into the genome of a cell. Furthermore, through this, a target protein can be produced.

### Brief Description of Drawings

Figure 01 shows an example of the novel promoter of the present application and the corresponding region on the genome.
Figure 02 shows an example of an HDR-mediated repair process involving the HDR template of a CRISPR/Cas-based gene editing process.
Figure 03 shows an example of a usage aspect of the promoter of the present application. As shown, a genome comprising the promoter of the present application and a transgene can be engineered by an HDR-mediated repair process involving an HDR template comprising UHA, the promoter of the present application, a transgene, and DHA.
Figure 04 shows an example of a usage aspect of the promoter of the present application. As shown, a genome comprising a transgene can be engineered by an HDR-mediated repair process involving an HDR template comprising UHA comprising the promoter of the present application, a transgene, and DHA.
Figure 05 shows an example of a target site, an upstream homology arm, and a downstream homology arm that can be designed in a usage aspect (3) of the promoter of the present application.
Figure 06 shows an example of a target site, an upstream homology arm, and a downstream homology arm that can be designed in a usage aspect (3) of the promoter of the present application.
Figure 07 shows an example of a target site, an upstream homology arm, and a downstream homology arm that can be designed in a usage aspect (3) of the promoter of the present application.
Figure 08 shows the region on the genome corresponding to seq1 and the region on the genome corresponding to seq2 in an experimental example.
Figure 09 shows the AAV-ITR plasmid (plasmid 1) comprising the nucleic acid UHA (seq1)-SA-EGFP-bGHpA-DHA (seq2) in an experimental example.
Figure 10 shows an exemplary structure of the nucleic acid seq1-SA-EGFP-bGHpA-seq2 in an experimental example.
Figure 11 shows the AAV-ITR plasmid (plasmid 2) comprising seq1-SA-EGFP-bGHpA in an experimental example.
Figure 12 shows an exemplary structure of the nucleic acid seq1-SA-EGFP-bGHA in an experimental example.
Figure 13 shows a result obtained by confirming GFP expression in HepG2 cells. In Figure 13, UHA-GFP-DHA shows a result observed from HepG2 into which the nucleic acid seq1-SA-EGFP-bGHpA-seq2 was introduced. UHA-GFP shows a result observed from HepG2 into which the nucleic acid seq1-SA-EGFP-bGHpA was introduced.
Figure 14 shows a result obtained by confirming GFP expression in HepG2 cells. In Figure 14, UHA-GFP-DHA shows a result observed from HepG2 into which the nucleic acid seq1-SA-EGFP-bGHpA-seq2 was introduced. UHA-GFP shows a result observed from HepG2 into which the nucleic acid seq1-SA-EGFP-bGHpA was introduced.

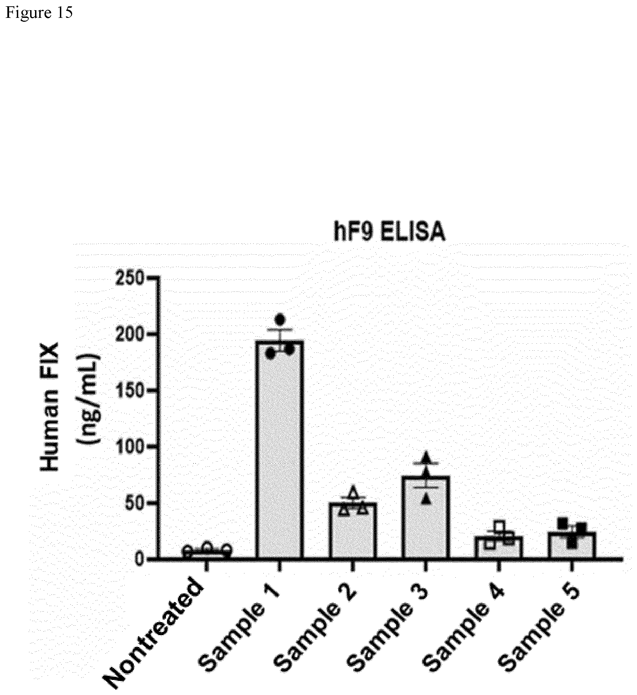

Figure 15 shows a result obtained by analyzing hF9 disclosed in Experimental Example 2 by ELISA. In Figure 15, a result for the sample treated with the AAV vector related to nucleic acid 1 is shown in sample 1, a result for the sample treated with the AAV vector related to nucleic acid 2 is shown in sample 2, a result for the sample treated with the AAV vector related to nucleic acid 3 is shown in sample 3, a result for the sample treated with the AAV vector related to nucleic acid 4 is shown in sample 4, and a result for the sample treated with the AAV vector related to nucleic acid 5 is shown in sample 5.
Figure 16 shows an exemplary structure of the nucleic acid seq5-SA-hF9-pA-seq6 in an Experimental Example.
Figure 17 shows a result obtained by measuring the hFIX concentration in mouse blood as disclosed in Experimental Example 3. Figure 17a shows a result obtained by observing the hFIX concentration in blood for a certain period (14 weeks) after injection of the AAV vector (AAV-UHA-hF9-DHA) comprising the nucleic acid (seq5-SA-hF9-pA-seq6) through the intravenous route in mice. Figure 17b shows a result obtained by measuring the hFIX concentration in mouse blood after intravenous or intraperitoneal injection of the AAV vector.

### Best Mode for Carrying out the Invention

### Definition of Terms

Unless otherwise defined, all technical and scientific terms used herein have meanings commonly understood by those of ordinary skill in the art to which the present invention pertains. The following references provide those of ordinary skill in the art with general definitions of many terms used herein: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, the Harper Collins Dictionary of Biology (1991). The following terms used herein have the meanings assigned to them unless otherwise specified.

### Nucleic acid

As used herein, the term "nucleic acid" is used to mean a portion of a region within a molecule or the entire molecule consisting of DNA (double-stranded or single-stranded), RNA (double-stranded or single-stranded), or a hybrid of DNA and RNA (double-stranded or single-stranded). Nucleic acid is used to mean a set of nucleotides (either a portion of a region within a molecule or the entire molecule). The terms nucleic acid or nucleic acid region may be used to refer to a portion of a region within a molecule. The terms nucleic acid or nucleic acid molecule may be used to refer to the entire molecule. The term "nucleic acid" should be interpreted appropriately depending on the context, and the content of each context including the description of the term "nucleic acid" will help those of ordinary skill in the art understand the meaning of the term nucleic acid.

### Upstream and downstream

As used herein, the terms "upstream" and "downstream" are relative terms that define the linear position of at least two elements located in a nucleic acid molecule (whether single-stranded or double-stranded) oriented in a 5' to 3' direction. For example, a first element is described as being upstream of a second element in a nucleic acid molecule, where the first element is located somewhere in the 5' direction with respect to the second element. For example, if the promoter is on the 5' side of the transgene, the promoter may be described as being located upstream of the transgene. As another example, a first element is described as being downstream of a second element in a nucleic acid molecule, where the first element is located somewhere in the 3' direction with respect to the second element. For example, if a transgene is on the 3' side of a promoter, the transgene may be described as being located downstream of the promoter. A nucleic acid molecule may be DNA (double-stranded or single-stranded), RNA (double-stranded or single-stranded), or a hybrid of DNA and RNA.

### Operably linked

As used herein, the term "operably linked" refers to a variety of possible arrangements of nucleic acid sequence elements that are linked so that each nucleic acid sequence element may perform its respective function and each nucleic acid sequence element may interact with one another. Examples of nucleic acid sequence elements include, but are not limited to, promoters, polyadenyl sequences, introns, exons, genes (target genes or transgenes), and the like. Nucleic acid sequence elements may be operably linked to control the presence or absence of expression, or the level of expression of the transgene.

### "Linked" or "linkage"

As used herein, the term "linked" or "linkage" means that two or more elements present within one conceptualizable construct are linked directly or indirectly (e.g., through another element such as a linker), and it does not intend that other additional elements are not be present between the above two or more elements. For example, a description such as "element B linked to element A" is intended to include instances where one or more other elements are present between element A and element B (i.e. element A is linked to element B through one or more other elements), and instances where one or more other elements are not present between element A and element B (i.e. element A is directly linked to element B), and is not to be interpreted in a limited manner.

### Administration

"Administration" of a substance, compound, nucleic acid, vector, composition, or preparation may be performed using any one of a variety of methods known to those of ordinary skill in the art. For example, the substance, compound, nucleic acid, vector, composition, or preparation may be administered intravenously, intraarterially, intradermally, intramuscularly, intraperitoneally, subcutaneously, intraocularly, sublingually, orally (by ingestion), intranasally (by inhalation), intrathecally, intracelebellar, intracerebrally, or transdermally (e.g., by absorption through the dermal tract).

### Expression

As used herein, the term "expression" refers to producing a product (e.g., RNA, including pre-mRNA and mature-mRNA, peptide, or protein, etc.) from genetic information (e.g., genomic DNA and transgene, etc.). For example, as used herein, "expression of a transgene" may mean "transcription of a transgene into mRNA" and/or "translation of an mRNA associated with a transgene." For example, when used herein as "regulating the expression of a transgene," the above phrase "regulating the expression of a transgene" may be interpreted as "regulating the transcription of a transgene," and/or "producing a target protein from a transgene," etc.

### Sequence identity

As used herein, the term "sequence identity" is a term used in relation to the degree of similarity between two or more nucleotide sequences. For example, the term "sequence identity" is used with terms referring to a reference sequence and terms indicating a ratio (e.g., percentage). For example, the term "sequence identity" may be used to describe a sequence that is similar or substantially identical to a reference nucleotide sequence. When described as "a sequence having at least 90% sequence identity to sequence A," the reference sequence here is sequence A. For example, the percentage of sequence identity may be calculated by aligning the sequence that is the subject of the percent sequence identity measurement with a reference sequence, and the percentage of sequence identity may be calculated including all mismatches, deletions, and insertions of one or more nucleotides. The method of calculating and/or determining the percentage of sequence identity is not otherwise limited, and may be calculated and/or determined through a reasonable method or algorithm that may be used by those of ordinary skill in the art.

### Target sequence

As used herein, the term "target sequence" refers to a specific sequence recognized by the guide RNA or Cas/gRNA complex used in a CRISPR/Cas gene editing system in order to cleave the target gene or target nucleic acid. The target sequence may be appropriately selected depending on the purpose. Specifically, "target sequence" is a sequence included within a target gene or target nucleic acid sequence, and may be used to refer to a sequence having complementarity to the spacer sequence included in the guide RNA provided herein, to refer to a sequence having substantial identity to the spacer sequence included in the guide RNA, or to refer to a double-stranded region comprising a sequence having complementarity to the spacer sequence and a sequence complementary thereto. In general, the spacer sequence is determined considering the sequence of the target gene or target nucleic acid and the PAM sequence recognized by the Cas protein of the CRISPR/Cas system. The target sequence may refer only to a sequence on a specific strand that binds complementarily to the guide RNA of the CRISPR/Cas complex, may refer only to a sequence on a specific strand that does not bind complementarily to the guide RNA, or may also refer to the entire target double strand including a portion of the specific strand, and is interpreted appropriately depending on the context. In addition, the above term includes all meanings that may be recognized by those of ordinary skill in the art, and may be appropriately interpreted depending on the context.

### Vector

As used herein, the term "vector" refers collectively to all substances capable of transporting genetic material into a cell, unless otherwise specified. For example, the vector may be a DNA molecule comprising the genetic material of interest, such as a nucleic acid encoding the Cas protein of the CRISPR/Cas system, and/or a nucleic acid encoding a guide RNA, but is not limited thereto. The above term includes all meanings recognized by those of ordinary skill in the art, and may be appropriately interpreted depending on the context.

### About

As used herein, the term "about" means a degree of approximation to a quantity, and refers to an amount, level, value, number, frequency, percent, dimension, size, quantity, weight, or length that varies by about 30, 25, 20, 25, 10, 9, 8, 7, 6, 5, 4, 3, 2 or 1% relative to the reference amount, level, value, number, frequency, percent, dimension, size, quantity, weight, or length.

### Orientation of the disclosed sequences

The nucleotide sequences disclosed herein (e.g., DNA sequences, RNA sequences, DNA/RNA hybrid sequences) are to be understood as disclosed in a 5' to 3' direction, unless otherwise specified. The amino acid sequences disclosed herein are to be understood as disclosed in the direction from the N terminus to the C terminus, unless otherwise specified. For the sequences disclosed in an orientation different from the above-mentioned orientation, the orientation in the other direction is separately specified in the paragraph related to the sequence.

### Overview of promoter

As used herein, the term promoter is used broadly to refer to a region consisting of a DNA sequence or a set of nucleotides that is essential for transcription of the desired DNA sequence and is located near (for example, upstream) the desired DNA sequence, controlling the expression of the desired DNA sequence. A promoter may refer to a DNA sequence or region to which a protein (e.g., transcription factor, RNA polymerase, etc.) binds in order to initiate transcription of the DNA sequence near the promoter (e.g., downstream of the promoter). A promoter is generally located upstream of the DNA sequence that is the target of transcription. The DNA sequence that is the target of transcription is transcribed into the corresponding RNA. It is noted that a promoter is essential for expressing the desired DNA sequence (e.g., transgene). For example, even when a transgene is introduced into a cell, it is difficult for the transgene to be expressed within the cell if there is no promoter cis-linked to the transgene. For this reason, researchers conducting research aimed at the expression of a transgene in a cell either (i) induce the expression of a transgene using an endogenous promoter present in the cell, or (ii) induce the expression of a transgene using an exogenous promoter. As in (i), the transgene is inserted into the genome of a cell in order to use the endogenous promoter present in the cell, wherein the insertion site of the transgene is designed to be near the endogenous promoter region present on the genome (e.g., downstream of the endogenous promoter region). As in (ii), in order to induce the expression of a transgene using an exogenous promoter, a nucleic acid comprising an exogenous promoter and a transgene (at this time, the exogenous promoter may be operably linked to the transgene) is introduced into a cell. As in (ii), when using an exogenous promoter, the expression of a transgene may be induced within the cell without inserting the transgene into the genome of a cell. In addition, alternatively, the expression of a transgene may be induced by inserting the transgene into the genome of a cell; in this case, the insertion site of the transgene is not limited to the neighborhood of the endogenous promoter as in (i). This is because an exogenous promoter operably linked to the transgene is inserted into the genome of a cell together with the transgene. As such, a promoter is an essential element for the expression of a desired DNA sequence.

Hereinafter, the novel promoter provided herein is disclosed in detail.

### Novel promoter provided by the present application

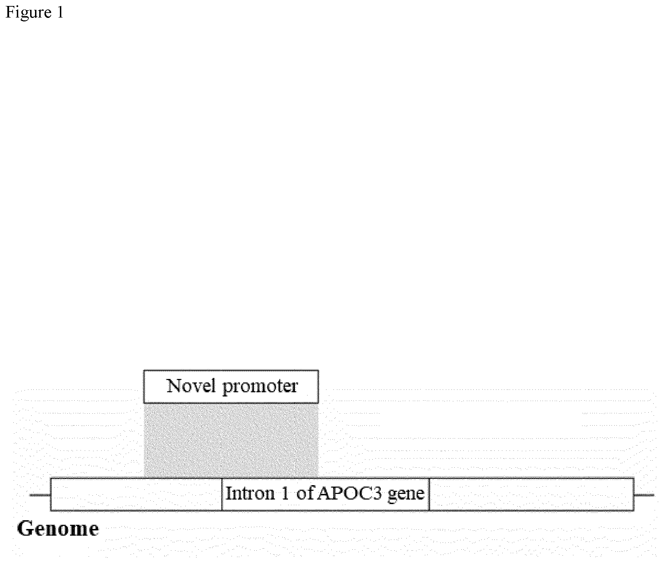

In one embodiment of the present application, there is provided a novel promoter. The novel promoter provided by the present application includes a sequence derived from part or all of intron 1 of the APOC3 gene, which encodes the protein apolipoprotein C-III. Furthermore, the novel promoter provided by the present application may include a portion of the DNA sequence located linked to the 5' terminus of the gene (APOC3 gene) encoding APOC3 (apolipoprotein C-III) and a sequence derived from part or all of intron 1 of the APOC3 gene. Examples of the novel promoter of the present application and the corresponding region near the APOC3 gene on the genome and the region of the APOC3 gene are shown in Figure 01, but are not limited thereto.

The APOC3 gene refers to the gene encoding apolipoprotein C-III. The APOC3 gene is present on chromosome 11 in humans, is present on chromosome 9 in mice (house mice), and is present on chromosome 8 in rats (Norway rats). In one embodiment, the novel promoter of the present application may comprise a sequence derived from part or all of intron 1 of the APOC3 gene. In one embodiment, the novel promoter of the present application may further comprise, in addition to a sequence derived from part or all of intron 1 of the APOC3 gene, a sequence derived from one or more of part or all of the 5' flanking region of the APOC3 gene, part or all of the 5' UTR, and part or all of exon 1. In one embodiment, the novel promoter of the present application may comprise a sequence derived from one or more of part or all of intron 1 of the APOC3 gene, part or all of the 5' flanking region, part or all of the 5' UTR, and part or all of exon 1. In certain embodiments, the novel promoter of the present application may comprise a sequence derived from a portion of the 5' flanking region of the APOC3 gene, a sequence derived from the entire 5' UTR, a sequence derived from the entire exon 1 of the APOC3 gene, and a sequence derived from part of intron 1 of the APOC3 gene. The novel promoter of the present application is derived from the APOC3 gene and/or a sequence located near the APOC3 gene, and may be referred to as an APOC3 gene-derived promoter. In one embodiment, the APOC3 gene-derived promoter may comprise a sequence having identity to the sequence of a portion of the APOC3 gene (for example, the sequence of part or all of intron 1 of the APOC3 gene). In one embodiment, the APOC3 gene-derived promoter may comprise a sequence having identity to the sequence of the region linked to the 5' terminus of the APOC3 gene on the genome and a sequence having identity to the sequence of a portion of the APOC3 gene (for example, the sequence of part or all of intron 1 of the APOC3 gene).

In one embodiment, the novel promoter of the present application may be a liver-specific promoter. For example, a liver-specific promoter (for example, hepatocyte-specific promoter) may refer to a sequence or region that may function as a promoter within cells found in the liver (for example, hepatocytes). A liver-specific promoter may not function as a promoter in cells other than hepatocytes, or may function as a promoter. The term liver-specific promoter refers to a sequence or region that functions as a promoter in hepatocytes, but a liver-specific promoter should not be interpreted as being limited to being inactive in cells other than hepatocytes. In one embodiment, a liver-specific promoter may function as a promoter in cells other than hepatocytes. In another embodiment, a liver-specific promoter may not function as a promoter in cells other than hepatocytes.

In one embodiment, the present application provides a novel promoter. The novel promoter of the present application may function as a promoter within cells. In one embodiment, the cell may be a prokaryotic cell or a eukaryotic cell. In one embodiment, the eukaryotic cell may be a plant cell, an animal cell, a non-human animal cell, and/or a human cell. In certain embodiments, the human cell may be an immune cell (for example, T cell, B cell, NK cell, and macrophage, etc.), a stem cell (for example, IPS cell), an enterocyte, and/or a hepatocyte.

In one embodiment, the novel promoter of the present application may comprise any one of the sequences of SEQ ID NOs: 01 to 08.

In one embodiment, the novel promoter of the present application may comprise any one of sequences having at least 80%, 81%, 82%, 83%, 84%, 85%, 86%, 87%, 88%, 89%, 90%, 91%, 92%, 93%, 94%, 95%, 96%, 97%, 98%, 99%, and 99.5% sequence identity to the sequences of SEQ ID NOs: 01 to 08.

In one embodiment, the novel promoter of the present application may comprise any one of the sequences of SEQ ID NOs: 01 to 08, and sequences having at least 80%, 81%, 82%, 83%, 84%, 85%, 86%, 87%, 88%, 89%, 90%, 91%, 92%, 93%, 94%, 95%, 96%, 97%, 98%, 99%, and 99.5% sequence identity thereto.

In one embodiment, the novel promoter of the present application may comprise any one sequence selected from the sequences of SEQ ID NOs: 01 to 08 and complementary sequences thereof, and sequences having at least 80%, 81%, 82%, 83%, 84%, 85%, 86%, 87%, 88%, 89%, 90%, 91%, 92%, 93%, 94%, 95%, 96%, 97%, 98%, 99%, and 99.5% sequence identity to the sequences of SEQ ID NOs: 01 to 08 and complementary sequences thereof.

Hereinafter, the sequences of SEQ ID NOs: 01 to 08 are disclosed in Table 01.

**Table 01. Sequences of SEQ ID NOs: 01 to 08**

| **SEQ ID NO** | **Sequence** |
|---|---|
| 01 | TGAGAGCTCAGCCCTGTAACCAGGCCTTGCCGGAGCCACTGATGCCTGGTCTTCTGTGCCTT TACTCCAAACACCCCCCAGCCCAAGCCACCCACTTGTTCTCAAGTCTGAAGAAGCCCCTCAC CCCTCTACTCCAGGCTGTGTTCAGGGCTTGGGGCTGGTGGAGGGAGGGGCCTGAAATTCCAG TGTGAAAGGCTGAGATGGGCCCGAGGCCCCTGGCCTATGTCCAAGCCATTTCCCCTCTCACC AGCCTCTCCCTGGGGAGCCAGTCAGCTAGGAAGGAATGAGGGCTCCCCAGGCCCACCCCCAG TTCCTGAGCTCATCTGGGCTGCAGGGCTGGCGGGACAGCAGCGTGGACTCAGTCTCCTAGGG ATTTCCCAACTCTCCCGCCCGCTTGCTGCATCTGGACACCCTGCCTCAGGCCCTCATCTCCA CTGGTCAGCAGGTGACCTTTGCCCAGCGCCCTGGGTCCTCAGTGCCTGCTGCCCTGGAGATG ATATAAAACAGGTCAGAACCCTCCTGCCTGTCTGCTCAGTTCATCCCTAGAGGCAGCTGCTC CAGGTAATGCCCTCTGGGGAGGGGAAAGAGGAGGGGAGGAGGATGAAGAGGGGCAAGAGGAG CTCCCTGCCCAGCCCAGCCAGCAAGCCTGGAGAAGCACTTGCTAGAGCTAAGGAAGCCTCGG AGCTGGACGGGTGCCCCCCACCCCTCATCATAACCTGAAGAACATGGAGGCCCGGGAGGGGT GTCACTTGCCCAAAGCTACACAGGGGGTGGGGCTGGAAGTGGCTCCAAGTGCAGGTTCCCCC CTCATTCTTCAGGCTTAGGGCTGGAGGAAGCCTTAGACAGCCCAGTCCTACCCCAGACAGGG AAACTGAGGCCTGGAGAGGGCCAGAAATCACC |
| 02 | CGCCCTGGGTCCTCAGTGCCTGCTGCCCTGGAGATGATATAAAACAGGTCAGAACCCTCCTG CCTGTCTGCTCAGTTCATCCCTAGAGGCAGCTGCTCCAGGTAATGCCCTCTGGGGAGGGGAA AGAGGAGGGGAGGAGGATGAAGAGGGGCAAGAGGAGCTCCCTGCCCAGCCCAGCCAGCAAGC CTGGAGAAGCACTTGCTAGAGCTAAGGAAGCCTCGGAGCTGGACGGGTGCCCCCCACCCCTC ATCATAACCTGAAGAACATGGAGGCCCGGGAGGGGTGTCACTTGCCCAAAGCTACACAGGGG GTGGGGCTGGAAGTGGCTCCAAGTGCAGGTTCCCCCCTCATTCTTCAGGCTTAGGGCTGGAG GAAGCCTTAGACAGCCCAGTCCTACCCCAGACAGGGAAACTGAGGCCTGGAGAGGGCCAGAA ATCACC |
| 03 | TCCTGAGCTCATCTGGGCTGCAGGGCTGGCGGGACAGCAGCGTGGACTCAGTCTCCTAGGGA TTTCCCAACTCTCCCGCCCGCTTGCTGCATCTGGACACCCTGCCTCAGGCCCTCATCTCCAC TGGTCAGCAGGTGACCTTTGCCCAGCGCCCTGGGTCCTCAGTGCCTGCTGCCCTGGAGATGA TATAAAACAGGTCAGAACCCTCCTGCCTGTCTGCTCAGTTCATCCCTAGAGGCAGCTGCTCC AGGTAATGCCCTCTGGGGAGGGGAAAGAGGAGGGGAGGAGGATGAAGAGGGGCAAGAGGAGC TCCCTGCCCAGCCCAGCCAGCAAGCCTGGAGAAGCACTTGCTAGAGCTAAGGAAGCCTCGGA GCTGGACGGGTGCCCCCCACCCCTCATCATAACCTGAAGAACATGGAGGCCCGGGAGGGGTG TCACTTGCCCAAAGCTACACAGGGGGTGGGGCTGGAAGTGGCTCCAAGTGCAGGTTCCCCCC TCATTCTTCAGGCTTAGGGCTGGAGGAAGCCTTAGACAGCCCAGTCCTACCCCAGACAGGGA AACTGAGGCCTGGAGAGGGCCAGAAATCACC |
| 04 | CGCCCTGGGTCCTCAGTGCCTGCTGCCCTGGAGATGATATAAAACAGGTCAGAACCCTCCTG CCTGTCTGCTCAGTTCATCCCTAGAGGCAGCTGCTCCAGGTAATGCCCTCTGGGGAGGGGAA AGAGGAGGGGAGGAGGATGAAGAGGGGCAAGAGGAGCTCCCTG |
| 05 | TCCTGAGCTCATCTGGGCTGCAGGGCTGGCGGGACAGCAGCGTGGACTCAGTCTCCTAGGGA TTTCCCAACTCTCCCGCCCGCTTGCTGCATCTGGACACCCTGCCTCAGGCCCTCATCTCCAC TGGTCAGCAGGTGACCTTTGCCCAGCGCCCTGGGTCCTCAGTGCCTGCTGCCCTGGAGATGA TATAAAACAGGTCAGAACCCTCCTGCCTGTCTGCTCAGTTCATCCCTAGAGGCAGCTGCTCC AGGTAATGCCCTCTGGGGAGGGGAAAGAGGAGGGGAGGAGGATGAAGAGGGGCAAGAGGAGC TCCCTG |
| 06 | TGCCCAGCTACGGCAGAGGAGACTGTTGGGATCACAGGTGGAGGTCAGTGTGGGGGCATGTA ACCTTCACTCTGCCCCCTCCCACCCCACTTCCTCCGTTGCTCCACAGTGGCACCACAGATTC CCCATTCCATTCTACTGGAAGGCTTTCAGGTGGCTTCTGTGAAGGAGCCTGTCACTGGCTCT AGGCTGTAAAGCAGGCCTGAGGTCCAAGGCTTGTCCCCTGTCTTTTCCTGTCTCACCGACCT CATCTAGGTGAGCCGGTAGCTGATCCCTTGGTGGCGGAGGTGGCCGTGAAAAGCATGGGCAA TCTCAGAGGGATTTCTCAACTCCTCTGGCAGATGGCTGCATGGCCTTCCTGCCTCAGGCTCT GGTCTGGACTGCTCAGCAGGTGACCTTTGCCCAGCTCACTGGGCCTTCTGTGCCCGCTGTCC CATCCTGGAGCCAATATAAAACAGGTCACAGCCCTCCCCGCTTGCCTGCTCAGTTTTATCCC TAGAAGCAGCTAGCTACTCCAGGTAATGCCCCTGGGGAGGAGAGGAAGGAAGGGAAGAAACA AAGAGCTGGAGGGAGAAGCTCTCACCACCCAGCCATCTAGCCCACAGAAGGCTTGGGACTCA TGGTCTCCACCCTTGGGTTCCTGGTGTGGGGGGGGCTAGAAGCTGGGGAAAGGGAGGCTATG GGCCAAAAGCCTTGCACTGGGTAGTGGCAGAGCTGCCTGCAATTGGGTCATGCTGTCCCTTG TTTAGCTTGCTCTGAGCTATTAGAAGCCTTTGGTATCCATGCCTACCCAGGACTGAAAAAAC TGAGTCCTAGCGAGGAAAAGAAACCTATTGTTTCTCAGCATATTAGAGTAGATGTCCCGTTC TGCAGAGTATTTCTATACTCCACCTTCCAGGTTCCCCCCTCATTCTTCAGGCTTAGGGCTGG AGGAAGCCTTAGACAGCCCAGTCCTACCCCAGACAGGGAAACTGAGGCCTGGAGAGGGCCAG AAATCACC |
| 07 | CAAAGACACACAGCATGTTGGCTGGACTGGACGGAGATCAGTCCAGACCGCAGGTGCCTTGA TGTTCAGTCTGGTGGGTTTTCTGCTCCATCCCACCCACCTCCCTTTGGGCCTCGATCCCTCG CCCCTCACCAGTCCCCCTTCTGAGAGCCCGTATTAGCAGGGAGCCGGCCCCTACTCCTTCTG GCAGACCCAGCTAAGGTTCTACCTTAGGGGCCACGCCACCTCCCCAGGGAGGGGTCCAGAGG CATGGGGACCTGGGGTGCCCCTCACAGGACACTTCCTTGCAGGAACAGAGGTGCCATGCAGC CCCGGGTACTCCTTGTTGTTGCCCTCCTGGCGCTCCTGGCCTCTGCCCGTAAGCACTTGGTG GGACTGGGCTGGGGGCAGGGTGGAGGCAACTTGGGGATCCCAGTCCCAATGGGTGGTCAAGC AGGAGCCCAGGGCTCGTCCAGAGGCCGATCCACCCCACTCAGCCCTGCTCTTTCCTCAGGAG CTTCAGAGGCCGAGGATGCCTCCCTTCTCAGCTTCATGCAGGGTTACATGAAGCACGCCACC AAGACCGCCAAGGATGCACTGAGCAGCGTGCAGGAGTCCCAGGTGGCCCAGCAGGCCAGGTA CACCCGCTGGCCTCCCTCCCCATCCCCCCTGCCAGCTGCCTCCATTCCCACCCGCCCCTGCC CTGGTGAGATCCCAACAATGGAATGGAGGTGCTCCAGCCTCCCCTGGGCCTGTGCCTCTTCA GCCTCCTCTTTCCTCACAGGGCCTTTGTCAGGCTGCTGCGGGAGAGATGACAGAGTTGAGAC TGCATTCCTCCCAGGTCCCTCCTTTCTCCCCGGAGCAGTCCTAGGGCGTGCCGTTTTAGCCC TCATTTCCATTTTCCTTTCCTTTCCCTTTCTT |
| 08 | CAAAGACACACAGACCACTTGGGGGGCTGGGTCTACTGTAGTTCTCTATCTAATATCAGTGG GAGAAAGGCTTGGGATTCATGACCCAGGAGTCATGGCATTCTCAAGGGGAGGACCTAGATGT GGCAGATCCACAGTACCTTCTCACGAGATGCTTTCCCACAGGTACGTAGGTGCCATGCAGCC CCGGACGCTCCTCACTGTGGCCCTCTTGGCTCTCCTGGCATCTGCCCGTAAGATGGTGGGAG TGGGGCTGGGTTTCAGTGGGAGCTGGTCAGGGGGCCCTGCCCTGAGAAGTGGTGGCTGGGCA GGAGCTGAGGGCTGGCGTGGCCCAACTCTGCTCTCTCGCAGGAGCTGAAGAGGTAGAGGGAT CCTTGCTGCTGGGCTCTGTACAGGGCTACATGGAACAAGCCTCCAAGACGGTCCAGGATGCG CTAAGTAGCGTGCAGGAGTCCGATATAGCTGTGGTGGCCAGGTAGGCACACTGGCCTCCTTT CCTGTGTCTTCTCCTCTCATGCCCTGGTGCTAAGACCCTAGCACAGAGACTGTGGTCCACCA AGTTTACCAGGGCCTGGGACCCGCCCGAGCCTCTGGTCCCTCATGAGGCCTTTCGGTACTGT GACCAAGATGACAGGCAGGGCAAGCAGCCTTCTCCTATCTTCTCTGCTTTTCCTCAGAGCAG |

In one embodiment, the novel promoter of the present application may have a length of 10 nt to 2000 nt or 10 bp to 2000 bp. In one embodiment, the promoter of the present application may have a length of 30 nt to 1500 nt or 30 bp to 1500 bp. In one embodiment, the promoter of the present application may have a length of 50 nt to 1000 nt or 50 bp to 1000 bp. In one embodiment, the promoter of the present application may have a length of 100 nt to 800 nt or 100 bp to 800 bp. In one embodiment, the promoter of the present application may have a length of 50 nt to 200 nt or 50 bp to 200 bp. In one embodiment, the promoter of the present application may have a length of 200 nt to 400 nt or 200 bp to 400 bp. In one embodiment, the promoter of the present application may have a length of 400 nt to 600 nt or 400 bp to 600 bp. In one embodiment, the promoter of the present application may have a length of 600 nt to 800 nt or 600 bp to 800 bp. In one embodiment, the promoter of the present application may have a length of 800 nt to 1000 nt or 800 bp to 1000 bp. In one embodiment, the promoter of the present application may have a length of 1000 nt to 1200 nt or 1000 bp to 1200 bp. In one embodiment, the promoter of the present application may have a length of 1200 nt to 1400 nt or 1200 bp to 1400 bp. In one embodiment, the promoter of the present application may have a length of 1400 nt to 1600 nt or 1400 bp to 1600 bp. In one embodiment, the promoter of the present application may have a length of 1600 nt to 1800 nt or 1600 bp to 1800 bp. In one embodiment, the promoter of the present application may have a length of 1800 nt to 2000 nt or 1800 bp to 2000 bp. In one embodiment, the promoter of the present application may have a length of about 20 nt, 22 nt, 24nt, 26nt, 28 nt, 30 nt, 32 nt, 34nt, 36nt, 38 nt, 40 nt, 42 nt, 44nt, 46nt, 48 nt, 50 nt, 55 nt, 60 nt, 65 nt, 70 nt, 75 nt, 80 nt, 85 nt, 90 nt, 95 nt, 100 nt, 110 nt, 120 nt, 130 nt, 140 nt, 150 nt, 160 nt, 170 nt, 180 nt, 190 nt, 200 nt, 220 nt, 240 nt, 260 nt, 280 nt, 300 nt, 320 nt, 340 nt, 360 nt, 380 nt, 400 nt, 420 nt, 440 nt, 460 nt, 480 nt, 500 nt, 520 nt, 540 nt, 560 nt, 580 nt, 600 nt, 620 nt, 640 nt, 660 nt, 680 nt, 700 nt, 720 nt, 740 nt, 760 nt, 780 nt, 800 nt, 820 nt, 840 nt, 860 nt, 880 nt, 900 nt, 920 nt, 940 nt, 960 nt, 980 nt, 1000 nt, 1100 nt, 1200 nt, 1300 nt, 1400 nt, 1500 nt, 1600 nt, 1800 nt, 1900 nt, or 2000 nt, or a length of about 20 bp, 22 bp, 24bp, 26bp, 28 bp, 30 bp, 32 bp, 34bp, 36bp, 38 bp, 40 bp, 42 bp, 44bp, 46bp, 48 bp, 50 bp, 55 bp, 60 bp, 65 bp, 70 bp, 75 bp, 80 bp, 85 bp, 90 bp, 95 bp, 100 bp, 110 bp, 120 bp, 130 bp, 140 bp, 150 bp, 160 bp, 170 bp, 180 bp, 190 bp, 200 bp, 220 bp, 240 bp, 260 bp, 280 bp, 300 bp, 320 bp, 340 bp, 360 bp, 380 bp, 400 bp, 420 bp, 440 bp, 460 bp, 480 bp, 500 bp, 520 bp, 540 bp, 560 bp, 580 bp, 600 bp, 620 bp, 640 bp, 660 bp, 680 bp, 700 bp, 720 bp, 740 bp, 760 bp, 780 bp, 800 bp, 820 bp, 840 bp, 860 bp, 880 bp, 900 bp, 920 bp, 940 bp, 960 bp, 980 bp, 1000 bp, 1100 bp, 1200 bp, 1300 bp, 1400 bp, 1500 bp, 1600 bp, 1800 bp, 1900 bp, or 2000 bp.

In one embodiment, the promoter of the present application may comprise any one or more of a TATA box, a CCAAT box, a NF-κB binding site, an AP-1 binding site, and a HNFJARP-1/RXR1. In one embodiment, the promoter of the present application may comprise any one or more of a TATA box and a CCAAT box. In one embodiment, the promoter of the present application may comprise a TATAbox. In one embodiment, the promoter of the present application may comprise a CCAAT box. For example, a TATAbox may comprise the sequence of TATAAAA (SEQ ID NO: 12). For example, a CCAAT box may comprise the sequence of CAATCT (SEQ ID NO: 13) or CAGTCT (SEQ ID NO: 14). For example, an AP-1 binding site may comprise TGCCTCA (SEQ ID NO: 15) or TGCCTCG (SEQ ID NO: 16). For example, a HNFJARP-1/RXR1 may comprise AGGTGACCTTTC (SEQ ID NO: 17).

As described above, the novel promoter provided by the present application may comprise a sequence derived from the APOC3 gene and/or a region near the APOC3 gene. At this time, the APOC3 gene includes, for example, the human APOC3 gene, the mouse APOC3 gene, and the rat APOC3 gene. In one embodiment, the novel promoter provided by the present application may comprise a sequence derived from the region linked to the 5' terminus of intron 1 of the hAPOC3 gene (human APOC3 gene intron 1) and/or a sequence derived from part or all of intron 1 of the hAPOC3 gene. In one embodiment, the novel promoter provided by the present application may comprise a sequence derived from the region linked to the 5' terminus of intron 1 of the mAPOC3 gene (mouse APOC3 gene intron 1) and/or a sequence derived from part or all of intron 1 of the mAPOC3 gene. In one embodiment, the novel promoter provided by the present application may comprise a sequence derived from the region linked to the 5' terminus of intron 1 of the rAPOC3 gene (rat APOC3 gene intron 1) and/or a sequence derived from part or all of intron 1 of the rAPOC3 gene. The sequence of each APOC3 intron 1 is disclosed in Table 02 below.

**Table 02. Entire sequence of intron 1 of APOC3 gene**

| | |
|---|---|
| Intron 1 of human AP0C3 gene | GTAATGCCCTCTGGGGAGGGGAAAGAGGAGGGGAGGAGGATGAAGAGGGGCAAGAGGAG CTCCCTGCCCAGCCCAGCCAGCAAGCCTGGAGAAGCACTTGCTAGAGCTAAGGAAGCCT CGGAGCTGGACGGGTGCCCCCCACCCCTCATCATAACCTGAAGAACATGGAGGCCCGGG AGGGGTGTCACTTGCCCAAAGCTACACAGGGGGTGGGGCTGGAAGTGGCTCCAAGTGCA GGTTCCCCCCTCATTCTTCAGGCTTAGGGCTGGAGGAAGCCTTAGACAGCCCAGTCCTA CCCCAGACAGGGAAACTGAGGCCTGGAGAGGGCCAGAAATCACCCAAAGACACACAGCA TGTTGGCTGGACTGGACGGAGATCAGTCCAGACCGCAGGTGCCTTGATGTTCAGTCTGG TGGGTTTTCTGCTCCATCCCACCCACCTCCCTTTGGGCCTCGATCCCTCGCCCCTCACC AGTCCCCCTTCTGAGAGCCCGTATTAGCAGGGAGCCGGCCCCTACTCCTTCTGGCAGAC CCAGCTAAGGTTCTACCTTAGGGGCCACGCCACCTCCCCAGGGAGGGGTCCAGAGGCAT GGGGACCTGGGGTGCCCCTCACAGGACACTTCCTTGCAG (SEQ ID NO: 09) |
| Intron 1 of mouse AP0C3 gene | GTAATGCCCCTGGGGAGGAGAGGAAGGAAGGGAAGAAACAAAGAGCTGGAGGGAGAAGC TCTCACCACCCAGCCATCTAGCCCACAGAAGGCTTGGGACTCATGGTCTCCACCCTTGG GTTCCTGGTGTGGGGGGGGCTAGAAGCTGGGGAAAGGGAGGCTATGGGCCAAAAGCCTT GCACTGGGTAGTGGCAGAGCTGCCTGCAATTGGGTCATGCTGTCCCTTGTTTAGCTTGC TCTGAGCTATTAGAAGCCTTTGGTATCCATGCCTACCCAGGACTGAAAAAACTGAGTCC TAGCGAGGAAAAGAAACCTATTGTTTCTCAGCATATTAGAGTAGATGTCCCGTTCTGCA GAGTATTTCTATACTCCACCTTCCACCCCACTTGGGGGGCTGGGTCTACTGTAGTTCTC TATCTAATATCAGTGGGAGAAAGGCTTGGGATTCATGACCCAGGAGTCATGGCATTCTC AAGGGGAGGACCTAGATGTGGCAGATCCACAGTACCTTCTCACGAGATGCTTTCCCACA G (SEQ ID NO: 10) |
| Intron 1 of rat AP0C3 gene | GTGGGTGGGGGAGGCTAGAAGCTGAGGAAAGGGAGGCCACTGGGCCAAAAAAGCCTTGC ACTGGGTAGTGGCAGAGCTGATGGGAACTGGGTCATGCTGCCCCTTGTTCGTCTTGCTC TGAGCTATTAGAAGCCTTTGATATCCGTGCCTACCCCAGACTGAAAAGCCTGAAGCCTA GAGAGAAAAAGAAACCTGTTGTTTCTCCATATATTGGGGCAGAGGCTGGGTAGATGTTC TGCTTTGCAGAGGATTCCCACATTCCACCTTCCAGCCCCCTTGGGGTGGGTCTGGGTCT TCTATAGTTCTCTCTCCGAGAGCTGATATCAGTAGGAGAAAGGCTTGGGGATTCATGAC CCAGGCAGGGGCCGTGGTGTTCTCCAGGGAAGGGCTTAGATGTGGCAGATCCACAATAC CCTCTCACAAGATGGTCTCCCGCAG (SEQ ID NO: 11) |

As described above, a portion of any one of the sequences of SEQ ID NOs: 01 to 08, which is a sequence that may be included in the novel promoter of the present application, may be identical to the sequence of a portion of the APOC3 gene or a region located near the APOC3 gene. For example, a portion of any one of the sequences of SEQ ID NO: 01 to SEQ ID NO: 05 may be identical to the sequence of a portion of intron 1 of the human APOC3 gene. For example, a portion of any one of the sequences of SEQ ID NOs: 06 and 08 may be identical to the sequence of a portion of intron 1 of the mouse APOC3 gene.

A promoter is generally present in a DNA molecule in which the promoter is present, and serves to induce and/or initiate transcription of a DNA sequence that is the target of transcription located near the region of the promoter (for example, upstream or downstream of the region of the promoter). The promoter provided by one embodiment of the present application has promoter activity (for example, functions as a promoter). The promoter provided by one embodiment of the present application also serves to induce and/or initiate transcription of a DNA sequence that is the target of transcription located near the promoter. It is noted that in the absence of an appropriate promoter, it is difficult to transcribe the DNA sequence that is the target of transcription. The promoter of the present application may be used in conjunction with the DNA that is the target of transcription to initiate or induce transcription of the DNA sequence that is the target of the transcription. For example, the promoter of the present application may be used in conjunction with a transgene to induce and/or initiate transcription of the transgene and to produce a target protein (encoded by the transgene). Hereinafter, a nucleic acid construct comprising the promoter of the present application and a transgene is disclosed. When a nucleic acid construct comprising the promoter of the present application and a transgene is introduced into a cell, the transgene may be expressed, regardless of whether the transgene is integrated into the genome of a cell (i.e., regardless of the use of an endogenous promoter).

### Nucleic acid construct comprising the novel promoter provided by the present application

### Overview of nucleic acid construct comprising the novel promoter of the present application

In one embodiment of the present application, there is provided a nucleic acid or nucleic acid construct comprising the promoter of the present application. The nucleic acid or nucleic acid construct comprises the promoter of the present application described above. In one embodiment, the nucleic acid or nucleic acid construct comprises the promoter of the present application and a transgene. At this time, the transgene may be operably linked to the promoter of the present application. At this time, the promoter of the present application may be operably linked to a transgene. In one embodiment, the nucleic acid construct may further comprise additional elements in addition to the promoter of the present application and a transgene. For example, additional elements included in the nucleic acid construct may be any one or more selected from homology arms (for example, an upstream homology arm and/or a downstream homology arm), nucleotide linkers, and additional functional elements. Hereinafter, a nucleic acid construct according to one embodiment of the present application is disclosed in detail. The nucleic acid construct disclosed in this section is a concept encompassing all nucleic acid constructs disclosed in usage aspects 1, 2, and 3 of the promoter of the present application, which will be described below.

### Element 1 included in nucleic acid construct - the novel promoter of the present application

A nucleic acid construct according to one embodiment of the present application may comprise the promoter of the present application. In one embodiment, the promoter of the present application may be referred to as an APOC3 gene-derived promoter. In one embodiment, the promoter of the present application may be referred to as a liver-specific promoter. The promoter of the present application is disclosed in detail in the section "Novel promoter provided by the present application". In one embodiment, the promoter of the present application may comprise any one sequence selected from the sequences of SEQ ID NOs: 01 to 08 and complementary sequences thereof, and sequences having at least 80%, 81%, 82%, 83%, 84%, 85%, 86%, 87%, 88%, 89%, 90%, 91%, 92%, 93%, 94%, 95%, 96%, 97%, 98%, 99%, and 99.5% sequence identity to the sequences of SEQ ID NOs: 01 to 08 and complementary sequences thereof.

### Element 2 included in nucleic acid construct - transgene

A nucleic acid construct according to one embodiment of the present application may comprise a transgene in addition to the promoter of the present application. In one embodiment, the promoter of the present application may be operably linked to a transgene. In one embodiment, a transgene may be operably linked to the promoter of the present application.

A transgene refers to a gene inserted into a cell for transcription and/or expression. In one embodiment, a transgene may be a gene encoding a target protein. At this time, the target protein may be a protein that is not produced in wild type target cells (for example, unmanipulated target cells). At this time, the target protein may be a protein that is produced in wild type target cells. In other words, a transgene refers to a gene inserted from outside the target cell, regardless of whether it is expressed within wild type target cells, and is not otherwise limited.

In one embodiment, a target protein may be any one selected from FVII (factor VII) (for example, human FVII), FVIII (factor VIII) (for example, human FVIII), FIX (Factor IX) (for example, human FIX), IDUA, I2S, SGSH, NAGLU, HGSNAT, GNS, GALNS, GLB1, ARSB, GUSB, HYAL, NEU, GNPTAB, MCOLN1, SAH1, GALC, CTSA, GLA, NAGA, beta-galactosidase, hexosaminidase, GBA, SMPD1, ARSA, NPC, PPT, TPP1, CLN3, CLN6, PPT1, DNAJC5, CTSF, CLN7, CLN8, CTSD, GAA, LAMP2, CTNS, CTSK, SLC17A5, MAN2B, MAN2C, MANBA, AGA, FUCA1, LAL, methylmalonic aciduria CbIA Type (MMAA) protein, methylmalonic aciduria CbIB Type (MMAB) protein, methylmalonic aciduria CbIC Type (MMADHC) protein, 5-Methyltetrahydrofolate-Homocysteine Methyltransferase Reductase (MTRR) protein, lysosomal membrane protein domain (LMBRD 1) protein, 5-Methyltetrahydrofolate-Homocysteine Methyltransferase (MTR) protein, propionyl-CoA protein, glucose-6-phosphate transporter (G6PT) protein, glucose-6-phosphatase (G6Pase) protein, low density lipoprotein receptor (LDLR) protein, low density lipoprotein receptor adaptor protein 1 (LDLRAP-1 protein), N-acetylglutamate synthetase (NAGS) protein, carbamoyl phosphate synthetase 1 (CPS1) protein, ornithine transcarbamylase (OTC) protein, argininosuccinic acid synthetase (ASS) protein, argininosuccinase acid lyase (ASL) protein, arginase (ARG1) protein, solute carrier family 25 protein, UDP glucuronosyltransferase 1 family, polypeptide A1 (UGT1A1) protein, fumarylacetoacetate hydrolase (FAH), alanine-glyoxylate aminotransferase (AGXT) protein, glyoxylate reductase/hydroxypyruvate reductase (GRHPR) protein, APTase Cu(2+) transporting beta (ATP7B) protein, phenylalanine hydroxylase (PAH) protein, and lipoprotein lyase (LPL) protein. In certain embodiments, a target protein may be FIX.

In one embodiment, a transgene may be a gene encoding any one or more selected from FVII (for example, human VII), FVIII (for example, human VIII), FIX (for example, human FIX), IDUA, I2S, SGSH, NAGLU, HGSNAT, GNS, GALNS, GLB1, ARSB, GUSB, HYAL, NEU, GNPTAB, MCOLN1, SAH1, GALC, CTSA, GLA, NAGA, beta-galactosidase, hexosaminidase, GBA, SMPD1, ARSA, NPC, PPT, TPP1, CLN3, CLN6, PPT1, DNAJC5, CTSF, CLN7, CLN8, CTSD, GAA, LAMP2, CTNS, CTSK, SLC17A5, MAN2B, MAN2C, MANBA, AGA, FUCA1, LAL, methylmalonic aciduria CbIA Type (MMAA) protein, methylmalonic aciduria CbIB Type (MMAB) protein, methylmalonic aciduria CbIC Type (MMADHC) protein, 5-Methyltetrahydrofolate-Homocysteine Methyltransferase Reductase (MTRR) protein, lysosomal membrane protein domain (LMBRD 1) protein, 5-Methyltetrahydrofolate-Homocysteine Methyltransferase (MTR) protein, propionyl-CoA protein, glucose-6-phosphate transporter (G6PT) protein, glucose-6-phosphatase (G6Pase) protein, low density lipoprotein receptor (LDLR) protein, low density lipoprotein receptor adaptor protein 1 (LDLRAP-1 protein), N-acetylglutamate synthetase (NAGS) protein, carbamoyl phosphate synthetase 1 (CPS1) protein, ornithine transcarbamylase (OTC) protein, argininosuccinic acid synthetase (ASS) protein, argininosuccinase acid lyase (ASL) protein, arginase (ARG1) protein, solute carrier family 25 protein, UDP glucuronosyltransferase 1 family, polypeptide A1 (UGT1A1) protein, fumarylacetoacetate hydrolase (FAH), alanine-glyoxylate aminotransferase (AGXT) protein, glyoxylate reductase/hydroxypyruvate reductase (GRHPR) protein, APTase Cu(2+) transporting beta (ATP7B) protein, phenylalanine hydroxylase (PAH) protein, and lipoprotein lyase (LPL) protein. In certain embodiments, a transgene may be a gene encoding FIX.

In one embodiment, a transgene may have a length of, but is not limited to, 10 nt to 10000 nt or 10 bp to 10000 bp. In one embodiment, a transgene may have a length of 100 nt to 5000 nt or 100 bp to 5000 bp. In one embodiment, a transgene may have a length of about 100 nt, 200 nt, 300 nt, 400 nt, 500 nt, 600 nt, 700 nt, 800 nt, 900 nt, 1000 nt, 1100 nt, 1200 nt, 1300 nt, 1400 nt, 1500 nt, 1600 nt, 1700 nt, 1800 nt, 1900 nt, 2000 nt, 2100 nt, 2200 nt, 2300 nt, 2400 nt, 2500 nt, 2600 nt, 2700 nt, 2800 nt, 2900 nt, 3000 nt, 3100 nt, 3200 nt, 3300 nt, 3400 nt, 3500 nt, 3600 nt, 3700 nt, 3800 nt, 3900 nt, 4000 nt, 4100 nt, 4200 nt, 4300 nt, 4400 nt, 4500 nt, 4600 nt, 4700 nt, 4800 nt, 4900 nt, 5000 nt, 5100 nt, 5200 nt, 5300 nt, 5400 nt, 5500 nt, 5600 nt, 5700 nt, 5800 nt, 5900 nt, 6000 nt, 6100 nt, 6200 nt, 6300 nt, 6400 nt, 6500 nt, 6600 nt, 6700 nt, 6800 nt, 6900 nt, 7000 nt, 7100 nt, 7200 nt, 7300 nt, 7400 nt, 7500 nt, 7600 nt, 7700 nt, 7800 nt, 7900 nt, 8000 nt, 8100 nt, 8200 nt, 8300 nt, 8400 nt, 8500 nt, 8600 nt, 8700 nt, 8800 nt, 8900 nt, 9000 nt, 9100 nt, 9200 nt, 9300 nt, 9400 nt, 9500 nt, 9600 nt, 9700 nt, 9800 nt, 9900 nt, 10000 nt, 100 bp, 200 bp, 300 bp, 400 bp, 500 bp, 600 bp, 700 bp, 800 bp, 900 bp, 1000 bp, 1100 bp, 1200 bp, 1300 bp, 1400 bp, 1500 bp, 1600 bp, 1700 bp, 1800 bp, 1900 bp, 2000 bp, 2100 bp, 2200 bp, 2300 bp, 2400 bp, 2500 bp, 2600 bp, 2700 bp, 2800 bp, 2900 bp, 3000 bp, 3100 bp, 3200 bp, 3300 bp, 3400 bp, 3500 bp, 3600 bp, 3700 bp, 3800 bp, 3900 bp, 4000 bp, 4100 bp, 4200 bp, 4300 bp, 4400 bp, 4500 bp, 4600 bp, 4700 bp, 4800 bp, 4900 bp, 5000 bp, 5100 bp, 5200 bp, 5300 bp, 5400 bp, 5500 bp, 5600 bp, 5700 bp, 5800 bp, 5900 bp, 6000 bp, 6100 bp, 6200 bp, 6300 bp, 6400 bp, 6500 bp, 6600 bp, 6700 bp, 6800 bp, 6900 bp, 7000 bp, 7100 bp, 7200 bp, 7300 bp, 7400 bp, 7500 bp, 7600 bp, 7700 bp, 7800 bp, 7900 bp, 8000 bp, 8100 bp, 8200 bp, 8300 bp, 8400 bp, 8500 bp, 8600 bp, 8700 bp, 8800 bp, 8900 bp, 9000 bp, 9100 bp, 9200 bp, 9300 bp, 9400 bp, 9500 bp, 9600 bp, 9700 bp, 9800 bp, 9900 bp, or 10000 bp. In one embodiment, the length of the transgene may be within a range determined by two values selected from the foregoing values.

In one embodiment, a transgene may be for expressing a protein in a human or non-human animal. In one embodiment, a transgene may be derived from a human or non-human animal.

In one embodiment, a transgene may be derived from a different species than the subject. In one embodiment, a transgene may be a wild type gene. In another embodiment, a transgene may be a mutated gene. In certain embodiments, a mutant may be a form in which one or more nucleotides of a wild type gene are deleted, substituted, or added.

### Relationship between the promoter of the present application and a transgene

As described above, on the nucleic acid construct, the promoter of the present application and a transgene are operably linked. In one embodiment, the promoter of the present application is operably linked to a transgene. In one embodiment, a transgene is operably linked to the promoter of the present application. In one embodiment, on the nucleic acid construct, the promoter may be located upstream or downstream of a transgene. In certain embodiments, the promoter may be located upstream of a transgene. In one embodiment, the promoter may be located at the 5' or 3' terminus of a transgene. In certain embodiments, the promoter may be located at the 5' terminus of a transgene. The promoter may be linked to a transgene through additional elements. For example, any one or more of a linker and a splicing acceptor may be present between the promoter and a transgene.

### Elements that may be included in a nucleic acid construct

The nucleic acid construct of the present application may comprise one or more additional elements in addition to the promoter of the present application and a transgene.

Additional elements may be, for example, an upstream homology arm, a downstream homology arm, a splicing acceptor (SA), a bovine growth hormone polyadenylation signal (bGHpA), or poly A (PA).

For example, the nucleic acid construct of the present application may further comprise an upstream homology arm. For example, the nucleic acid construct of the present application may further comprise a downstream homology arm. For example, the nucleic acid construct of the present application may further comprise SA. SA refers to a sequence required in the RNA splicing process. For example, the nucleic acid construct of the present application may further comprise a poly A (PA) sequence. The PA sequence may function to maintain the stability of mRNA during the transgene expression process. For example, the nucleic acid construct of the present application may further comprise a bovine growth hormone polyadenylation signal (bGHpA). bGHpA refers to the poly A sequence within the bovine growth hormone gene, and like poly A, it may function to maintain the stability of mRNA during the transgene expression process.

The promoter of the present application, or a nucleic acid comprising the promoter and a transgene, may be used in a variety of situations. Hereinafter, various usage aspects of the promoter of the present application are exemplified. Furthermore, a nucleic acid construct that may be suitable for each usage aspect is also exemplified. The following description of exemplary usage aspects of the promoter of the present application is intended to aid the understanding of those of ordinary skill in the art and does not limit the scope of the present application.

### Use (1) of the promoter of the present application

### Overview of use (1) of the promoter of the present application

As described above, the promoter of the present application may be used in conjunction with a desired DNA sequence for expression (for example, transcription) of the desired DNA sequence. For example, the promoter of the present application may be used in conjunction with a transgene. In one embodiment, there is provided a nucleic acid comprising the promoter of the present application and a transgene. In one embodiment, there is provided a nucleic acid for expressing transgene comprising the promoter of the present application and a transgene. When a nucleic acid for expressing transgene is introduced into a cell (e.g., into the nucleus of a cell), the transgene may be transcribed within the cell, regardless of whether the transgene is integrated into the genome of a cell. Since a nucleic acid for expressing transgene comprises the promoter of the present application that functions as a promoter, the transgene may be transcribed through the transcription tool present in the cell and the promoter of the present application introduced from outside. The transcription of a transgene may be performed regardless of whether the transgene is integrated into the genome. For example, a transgene may be expressed within a cell without being integrated into the genome. For example, a transgene may be integrated into the genome of a cell and expressed. At this time, the location where a transgene is inserted into the genome may be within intron 1 of the APOC3 gene, but is not otherwise limited.

### Promoter of the present application

A nucleic acid for expressing transgene comprises the promoter of the present application. The promoter of the present application is disclosed in detail in the sections "Novel promoter provided by the present application" and "Nucleic acid construct comprising the novel promoter provided by the present application".

### Transgene

A nucleic acid for expressing transgene comprises the promoter of the present application. A transgene is disclosed in detail in the section "Nucleic acid construct comprising the novel promoter provided by the present application".

### Nucleic acid comprising the promoter of the present application and a transgene

In one embodiment of the present application, there is provided a nucleic acid comprising the promoter of the present application and a transgene. In one embodiment of the present application, there is provided a nucleic acid for expressing transgene comprising the promoter of the present application and a transgene. As described above, the nucleic acid for expressing transgene provided according to one embodiment may comprise: the promoter of the present application; and a transgene. At this time, the promoter of the present application may be operably linked to a transgene. For example, the promoter of the present application may be located upstream of a transgene and may be operably linked to a transgene.

### Introduction of nucleic acid into cell

A nucleic acid for expressing transgene comprising the promoter of the present application and a transgene may be introduced into a cell. Introduction of the nucleic acid into a cell may be performed through methods well known to those of ordinary skill in the art. In one embodiment, a vector comprising a nucleic acid for expressing transgene may be introduced into a cell. For example, introduction of the nucleic acid into a cell may be achieved through methods disclosed in Panyam, Jayanth, and Vinod Labhasetwar. "Biodegradable nanoparticles for drug and gene delivery to cells and tissue." Advanced drug delivery reviews 55.3 (2003): 329-347, the contents of which are incorporated herein by reference in its entirety. Introduction of the nucleic acid into a cell is disclosed in detail in the section "Introduction of nucleic acid or nucleic acid construct" below.

### Expression of transgene in cell

A transgene may be expressed from a nucleic acid for expressing transgene introduced into a cell. For the expression of a transgene, the promoter of the present application and tools for performing transcription present in the cell may be used. In one embodiment, some of the tools for performing transcription may be introduced externally.

A transgene may be transcribed regardless of whether it is inserted into the genome of a cell. This is because the promoter of the present application operably linked to a transgene is introduced in conjunction with the transgene. In one embodiment, a transgene may be transcribed and/or expressed without being inserted into the genome and/or with being inserted into the genome of a cell. When a gene editing system for inserting a transgene into the genome is used in conjunction with a nucleic acid for expressing transgene, the expression of a transgene may be achieved both through a transgene (operably linked to the promoter of the present application) that is not inserted into the genome of a cell and a transgene that is inserted into the genome of a cell.

### Exemplary embodiment of use (1) of the promoter of the present application - nucleic acid construct 1

In one embodiment of the present application, there is provided a nucleic acid construct comprising:
a first nucleic acid comprising the promoter of the present application; and
a second nucleic acid comprising a transgene.

In certain embodiments, a first nucleic acid comprising the promoter of the present application may be operably linked to a second nucleic acid comprising a transgene. In certain embodiments, a first nucleic acid comprising the promoter of the present application may be operably linked to the 5' terminus of a second nucleic acid comprising a transgene. In certain embodiments, a first nucleic acid comprising the promoter of the present application may be operably linked to the 3' terminus of a second nucleic acid comprising a transgene.

In certain embodiments, a first nucleic acid may have a length of 30 nt to 2000 nt or 30 bp to 2000 bp. In certain embodiments, a first nucleic acid may have a length of 100 nt to 1500 nt or 100 bp to 1500 bp. In certain embodiments, a first nucleic acid may have a length of 300 nt to 1000 nt or 300 bp to 1000 bp. In certain embodiments, a first nucleic acid may have a length of about 50 nt, 55 nt, 60 nt, 65 nt, 70 nt, 75 nt, 80 nt, 85 nt, 90 nt, 100 nt, 110 nt, 120 nt, 130 nt, 140 nt, 150 nt, 160 nt, 170 nt, 180 nt, 190 nt, 200 nt, 220 nt, 240 nt, 260 nt, 280 nt, 300 nt, 320 nt, 340 nt, 340 nt, 360 nt, 380 nt, 400 nt, 420 nt, 440 nt, 460 nt, 480 nt, 500 nt, 550 nt, 600 nt, 650 nt, 700 nt, 750 nt, 800 nt, 850 nt, 900 nt, 950 nt, 1000 nt, 1100 nt, 1200 nt, 1300 nt, 1400 nt, 1500 nt, 50 bp, 55 bp, 60 bp, 65 bp, 70 bp, 75 bp, 80 bp, 85 bp, 90 bp, 100 bp, 110 bp, 120 bp, 130 bp, 140 bp, 150 bp, 160 bp, 170 bp, 180 bp, 190 bp, 200 bp, 220 bp, 240 bp, 260 bp, 280 bp, 300 bp, 320 bp, 340 bp, 340 bp, 360 bp, 380 bp, 400 bp, 420 bp, 440 bp, 460 bp, 480 bp, 500 bp, 550 bp, 600 bp, 650 bp, 700 bp, 750 bp, 800 bp, 850 bp, 900 bp, 950 bp, 1000 bp, 1100 bp, 1200 bp, 1300 bp, 1400 bp, or 1500 bp.

In certain embodiments, a second nucleic acid may have a length of 10 nt to 10000 nt or 10 bp to 10000 bp, but is not limited thereto. In certain embodiments, a second nucleic acid may have a length of 100 nt to 5000 nt or 100 bp to 5000 bp. In certain embodiments, a second nucleic acid may have a length of about 100 nt, 200 nt, 300 nt, 400 nt, 500 nt, 600 nt, 700 nt, 800 nt, 900 nt, 1000 nt, 1100 nt, 1200 nt, 1300 nt, 1400 nt, 1500 nt, 1600 nt, 1700 nt, 1800 nt, 1900 nt, 2000 nt, 2100 nt, 2200 nt, 2300 nt, 2400 nt, 2500 nt, 2600 nt, 2700 nt, 2800 nt, 2900 nt, 3000 nt, 3100 nt, 3200 nt, 3300 nt, 3400 nt, 3500 nt, 3600 nt, 3700 nt, 3800 nt, 3900 nt, 4000 nt, 4100 nt, 4200 nt, 4300 nt, 4400 nt, 4500 nt, 4600 nt, 4700 nt, 4800 nt, 4900 nt, 5000 nt, 5100 nt, 5200 nt, 5300 nt, 5400 nt, 5500 nt, 5600 nt, 5700 nt, 5800 nt, 5900 nt, 6000 nt, 6100 nt, 6200 nt, 6300 nt, 6400 nt, 6500 nt, 6600 nt, 6700 nt, 6800 nt, 6900 nt, 7000 nt, 7100 nt, 7200 nt, 7300 nt, 7400 nt, 7500 nt, 7600 nt, 7700 nt, 7800 nt, 7900 nt, 8000 nt, 8100 nt, 8200 nt, 8300 nt, 8400 nt, 8500 nt, 8600 nt, 8700 nt, 8800 nt, 8900 nt, 9000 nt, 9100 nt, 9200 nt, 9300 nt, 9400 nt, 9500 nt, 9600 nt, 9700 nt, 9800 nt, 9900 nt, 10000 nt, 100 bp, 200 bp, 300 bp, 400 bp, 500 bp, 600 bp, 700 bp, 800 bp, 900 bp, 1000 bp, 1100 bp, 1200 bp, 1300 bp, 1400 bp, 1500 bp, 1600 bp, 1700 bp, 1800 bp, 1900 bp, 2000 bp, 2100 bp, 2200 bp, 2300 bp, 2400 bp, 2500 bp, 2600 bp, 2700 bp, 2800 bp, 2900 bp, 3000 bp, 3100 bp, 3200 bp, 3300 bp, 3400 bp, 3500 bp, 3600 bp, 3700 bp, 3800 bp, 3900 bp, 4000 bp, 4100 bp, 4200 bp, 4300 bp, 4400 bp, 4500 bp, 4600 bp, 4700 bp, 4800 bp, 4900 bp, 5000 bp, 5100 bp, 5200 bp, 5300 bp, 5400 bp, 5500 bp, 5600 bp, 5700 bp, 5800 bp, 5900 bp, 6000 bp, 6100 bp, 6200 bp, 6300 bp, 6400 bp, 6500 bp, 6600 bp, 6700 bp, 6800 bp, 6900 bp, 7000 bp, 7100 bp, 7200 bp, 7300 bp, 7400 bp, 7500 bp, 7600 bp, 7700 bp, 7800 bp, 7900 bp, 8000 bp, 8100 bp, 8200 bp, 8300 bp, 8400 bp, 8500 bp, 8600 bp, 8700 bp, 8800 bp, 8900 bp, 9000 bp, 9100 bp, 9200 bp, 9300 bp, 9400 bp, 9500 bp, 9600 bp, 9700 bp, 9800 bp, 9900 bp, or 10000 bp.

In certain embodiments, a nucleic acid construct may consist of a nucleotide sequence. A nucleic acid construct may consist of a DNA sequence. In certain embodiments, a nucleic acid construct may consist of an RNA sequence. In certain embodiments, a nucleic acid construct may consist of a DNA/RNA hybrid sequence.

In certain embodiments, a nucleic acid construct may have a length of 100 nt to 1000000 nt or 100 bp to 1000000 bp. In certain embodiments, a nucleic acid construct may have a length of 300 nt to 100000 nt or 300 bp to 100000 bp, but is not otherwise limited. In certain embodiments, a nucleic acid construct may have a length of 300 nt to 10000 nt or 300 bp to 10000 bp, but is not otherwise limited. In certain embodiments, a nucleic acid construct may have a length of 500 nt to 10000 nt or 500 bp to 10000 bp, but is not otherwise limited. In certain embodiments, a nucleic acid construct may have a length of 100000 nt or 100000 bp or less, but is not limited thereto. In certain embodiments, a nucleic acid construct may have a length of 50000 nt or 50000 bp or less, but is not limited thereto. In certain embodiments, a nucleic acid construct may have a length of 10000 nt or 10000 bp or less, but is not limited thereto. In certain embodiments, a nucleic acid construct may have a length of 5000 nt or 5000 bp or less, but is not limited thereto. In certain embodiments, a nucleic acid construct may have a length of 3000 nt or 3000 bp or less, but is not limited thereto.

In certain embodiments, a nucleic acid construct may be provided as a double strand. In certain embodiments, a nucleic acid construct may be provided as a single strand.

### Gene editing system

### Overview of gene editing system

Before disclosing other usage aspects of the promoter of the present application, a gene editing system that may be used in conjunction with a nucleic acid comprising the promoter of the present application and a transgene is disclosed in detail. In a situation where a gene editing system is used with a nucleic acid comprising the promoter of the present application and a transgene, the gene editing system may be used to insert any one or more of the promoter of the present application and a transgene into the genome of a cell. Gene editing systems well known to those of ordinary skill in the art may be used as a gene editing system and may be appropriately designed depending on needs. For example, an appropriate type of gene editing system for inserting a transgene may be selected. For example, the insertion site of the transgene may be appropriately selected. Examples of gene editing systems include ZFN (zinc-finger nucleases), TALEN (transcription activator-like effector nucleases), and CRISPR/Cas gene editing systems, etc. [see Khan, Sikandar Hayat. "Genome-editing technologies: concept, pros, and cons of various genome-editing techniques and bioethical concerns for clinical application." Molecular Therapy-Nucleic Acids 16 (2019): 326-334, the contents of which are incorporated herein by reference in its entirety]. Hereinafter, a CRISPR/Cas gene editing system, which is a gene editing system that may be used in conjunction with the promoter of the present application and a transgene, is disclosed in detail.

### CRISPR/Cas gene editing system

As described above, in order to insert a desired nucleic acid (for example, promoter and/or transgene) into the genome of a cell, a cleavage (for example, DSB or nick) should be made in the genome of a cell. Thereafter, during the process of repairing the cleaved site, the nucleic acid for insertion is inserted into the desired location (for example, a target site) within the genome of a cell. A CRISPR/Cas gene editing system may be used as a technology for editing the genome. A CRISPR/Cas gene editing system is originated from CRISPR (clustered regularly interspaced short palindromic repeats), an immune system discovered in prokaryotes. For example, a CRISPR/Cas gene editing system is disclosed in Ferretti, Joseph J., et al. "Complete genome sequence of an M1 strain of Streptococcus pyogenes." Proceedings of the National Academy of Sciences 98.8 (2001): 4658-4663.; Deltcheva, Elitza, et al. "CRISPR RNA maturation by trans-encoded small RNA and host factor RNase III." Nature 471.7340 (2011): 602-607.; and Jinek, Martin, et al. "A programmable dual-RNA-guided DNA endonuclease in adaptive bacterial immunity." science 337.6096 (2012): 816-821, and the contents of each of the above documents are incorporated herein by reference in their entireties. A CRISPR/Cas gene editing system uses a Cas protein and a guide RNA. In addition, an HDR template comprising any one or more of the promoters of the present application and a transgene may be used. Hereinafter, elements that may be used in the genome editing process using a CRISPR/Cas gene editing system are disclosed.

### Cas protein

As used herein, the term Cas protein is used to collectively refer to gene editing proteins that may generate DSBs or nicks in the target region used in a CRISPR/Cas gene editing system. Examples of Cas proteins may include Cas9, Cas9 variant, Cas9 nickase (nCas9), dead Cas9, Cpf1 (type-V CRISPR-Cas system), C2c1 (type V CRISPR-Cas system), C2c2 (type VI CRISPR-Cas system), and C2c3 (type V CRISPR-Cas system). Furthermore, it may include Cas proteins derived from any type of CRISPR systems (for example, types II, V, VI). Examples of additional Cas proteins are described in Abudayyeh, Omar O., et al. "C2c2 is a single-component programmable RNA-guided RNA-targeting CRISPR effector." Science 353.6299 (2016): aaf5573, the contents of which are incorporated herein by reference in its entirety. In one embodiment, Cas proteins may be Cas9 or Cpf1 derived from various microorganism such as Streptococcus pyogenes, Streptococcus thermophilus, Streptococcus sp., Staphylococcus aureus, Campylobacter jejuni, Nocardiopsis dassonvillei, Streptomyces pristinaespiralis, Streptomyces viridochromogenes, Streptomyces viridochromogenes, Streptosporangium roseum, Streptosporangium roseum, Alicyclobacillus acidocaldarius, Bacillus pseudomycoides, Bacillus selenitireducens, Exiguobacterium sibiricum, Lactobacillus delbrueckii, Lactobacillus salivarius, Microscilla marina, Burkholderiales bacterium, Polaromonas naphthalenivorans, Polaromonas sp., Crocosphaera watsonii, Cyanothece sp., Microcystis aeruginosa, Synechococcus sp., Acetohalobium arabaticum, Ammonifex degensii, Caldicelulosiruptor bescii, Candidatus Desulforudis, Clostridium botulinum, Clostridium difficile, Finegoldia magna, Natranaerobius thermophilus, Pelotomaculum thermopropionicum, Acidithiobacillus caldus, Acidithiobacillus ferrooxidans, Allochromatium vinosum, Marinobacter sp., Nitrosococcus halophilus, Nitrosococcus watsoni, Pseudoalteromonas haloplanktis, Ktedonobacter racemifer, Methanohalobium evestigatum, Anabaena variabilis, Nodularia spumigena, Nostoc sp., Arthrospira maxima, Arthrospira platensis, Arthrospira sp., Lyngbya sp., Microcoleus chthonoplastes, Oscillatoria sp., Petrotoga mobilis, Thermosipho africanus, or Acaryochloris marina. In certain embodiments, a Cas protein used in a CRISPR/Cas gene editing system may be Cas9 derived from Streptococcus pyogenes or Cas9 derived from Campylobacter jejuni. For example, the DNA cleavage domain of Cas9 is known to comprise two subdomains: the NHN nuclease subdomain and the RucC1 subdomain. The NHN subdomain cleaves a strand complementary to the gRNA, while the RuvC1 subdomain cleaves a non-complementary strand. Inactivation of these subdomains may silence the nuclease activity of Cas9. For example, both mutations D10A and H840A completely inactivate the nuclease activity of *S. pyogenes* Cas9 (see Jinek, Martin, et al. "A programmable dual-RNA-guided DNA endonuclease in adaptive bacterial immunity." science 337.6096 (2012): 816-821). For example, mutation H840A provides Cas9 nickase.

### Guide RNA

In a CRISPR/Cas gene editing system, a Cas protein associates with a guide RNA within a cell to form a Cas/gRNA complex (Ribonucleoprotein, RNP). A Cas/gRNA complex generates a double-strand break (DSB) or nick within a target region comprising a sequence that corresponds (for example, is complementary) to a spacer sequence of the guide RNA (gRNA). The location at which a DSB or nick occurs may be near a PAM sequence on the genome. Targeting of Cas/gRNA involves a protospacer adjacent motif (PAM) on the genome and a spacer sequence of the guide RNA. Cas proteins (for example, Cas9) guided to the target region by PAM and a spacer sequence of the guide RNA generate DSBs within the target region.

In a CRISPR/Cas gene editing system, RNA that has the function of guiding a Cas protein to a target region to recognize a specific sequence included in a target DNA molecule is referred to as a guide RNA. If the composition of the guide RNA is divided functionally, it may be roughly divided into 1) a scaffold sequence portion, and 2) a guide domain comprising a guide sequence. The scaffold sequence portion is a portion that interacts with a Cas protein (for example, Cas9 protein) and is a portion that allows it to bind with a Cas protein to form a complex. Generally, the scaffold sequence portion includes tracrRNA and crRNA repeat sequence portions, and the scaffold sequence is determined depending on which Cas9 protein is used. The guide sequence is a portion that may bind complementary to a nucleotide sequence portion of a certain length within a target nucleic acid (for example, target DNA molecule or the genome of a cell). The guide sequence may be artificially modified and is determined by the target nucleotide sequence of interest relevant to the desired gene editing. In one embodiment, a guide RNA may be provided in two strands. In one embodiment, a guide RNA may be provided in one strand. A single guide RNA (sgRNA) in which tracrRNA and crRNA are linked (see Jinek, Martin, et al. "A programmable dual-RNA-guided DNA endonuclease in adaptive bacterial immunity." science 337.6096 (2012): 816-821, the contents of which are incorporated herein by reference in its entirety) have been developed. In certain embodiments, a guide RNA may be sgRNA.

In one embodiment, a guide sequence included in the guide domain of a guide RNA may have a length of 5 nt to 40 nt. In one embodiment, a guide sequence included in the guide domain of a guide RNA may have a length of 10 nt to 30 nt. In one embodiment, a guide sequence may have a length of 15 nt to 25 nt. In one embodiment, a guide sequence may have a length of 18 nt to 22 nt. In one embodiment, a guide sequence may have a length of 20 nt. In one embodiment, a target sequence, which is a sequence in the genome that forms a complementary binding with a guide sequence (including both a target sequence present in a spacer binding strand and a target sequence present in a spacer non-binding strand), may have a length of 5 nt to 40 nt or 5 bp to 40 bp. In one embodiment, a target sequence, which is a sequence in the genome that forms a complementary binding with a guide sequence, may have a length of 10 nt to 30 nt or 10 bp to 30 bp. In one embodiment, a target sequence may have a length of 15 nt to 25 nt or a length of 15 bp to 25 bp. In one embodiment, a target sequence may have a length of 18 nt to 22 nt or a length of 18 bp to 22 bp. In one embodiment, a target sequence may have a length of 20 nt or 20 bp. In one embodiment, the complementary binding of a guide RNA to a target sequence may comprise 0 to 5 mismatches. In one embodiment, a guide RNA may be designed to comprise 0 to 5 mismatches to a target sequence. In certain embodiments, the complementary binding of a guide RNA to a target sequence may have 0, 1, 2, 3, 4, or 5 mismatches.

For example, when a CRISPR/Cas gene editing system (Cas protein and gRNA) is used in conjunction with a nucleic acid comprising the promoter of the present application and a transgene, a target nucleic acid may be used to mean a nucleic acid having a sequence at a target site in the genome of a cell (for example, hepatocyte) or a sequence adjacent thereto.

### PAM (protospacer adjacent motif)

As described above, in a CRISPR/Cas gene editing system, a Cas/gRNA complex is guided to a target region by a protospacer adjacent motif (PAM) sequence on a target DNA molecule (for example, the genome of a cell) and a guide sequence of the gRNA. In a target DNA molecule, a PAM sequence may be located on a guide sequence non-binding strand other than the strand to which a guide sequence of a guide RNA binds. A PAM sequence may be determined independently depending on the type of Cas proteins to be used. In one embodiment, a PAM sequence may be any one selected from the following: (disclosed in a 5' to 3' direction): NGG (SEQ ID NO: 18); NNNNRYAC (SEQ ID NO: 19); NNAGAAW (SEQ ID NO: 20); NNNNGATT (SEQ ID NO: 21); NNGRR(T) (SEQ ID NO: 22); TTN (SEQ ID NO: 23); and NNNVRYAC (SEQ ID NO: 24). Each N may independently be A, T, C, or G. Each R may independently be A or G. Each Y may independently be C or T. Each W may independently be A or T. For example, when spCas9 is used as a Cas protein, a PAM sequence may be NGG (SEQ ID NO: 18). For example, when Streptococcus thermophilus Cas9 (StCas9) is used as a Cas protein, a PAM sequence may be NNAGAAW (SEQ ID NO: 20). For example, when NmCas9 (Neisseria meningitides Cas9) is used, a PAM sequence may be NNNNGATT (SEQ ID NO: 21). For example, when CjCas9 (Campylobacter jejuni Cas9) is used, PAM may be NNNVRYAC (SEQ ID NO: 24). In one embodiment, a PAM sequence may be linked to the 3' terminus of a target sequence present in a spacer non-binding strand (wherein a target sequence present in a spacer non-binding strand refers to a sequence that does not bind to a guide RNA). In one embodiment, a PAM sequence may be located at the 3' terminus of a target sequence present in a spacer non-binding strand. A target sequence present in a spacer non-binding strand refers to a sequence that does not bind to a guide sequence of a guide RNA. A target sequence present in a spacer non-binding strand is complementary to a target sequence present in a spacer binding strand.

The location at which a DSB or nick occurs may be near a PAM sequence on the genome. In one embodiment, the location at which a DSB or nick occurs may be -0 to -20 or +0 to +20 relative to the 5' or 3' terminus of a PAM sequence present in a spacer non-binding strand. In one embodiment, the location at which a DSB or nick occurs may be -1 to -5 or +1 to +5 of a PAM sequence on a spacer non-binding strand. For example, in a CRISPR/Cas gene editing system using spCas9, spCas9 cleaves between the third and fourth nucleotides located upstream of a PAM sequence.

### Genome editing mechanism - DNA repair

Within a cell, a Cas protein and gRNA form a complex and are guided to the target region on the genome of a cell. A Cas/gRNA complex guided to the target region generates a DSB or nick at a site within the target region (for example, target site). Thereafter, through the DNA repair process, DNA in which a DSB has occurred (cleaved) is repaired, thereby achieving gene editing in the target region or target site. The two main pathways for repair of DSBs generated in DNA include homology-directed repair (HDR) and NHEJ (nonhomologous end joining). Among these, HDR is disclosed in detail.

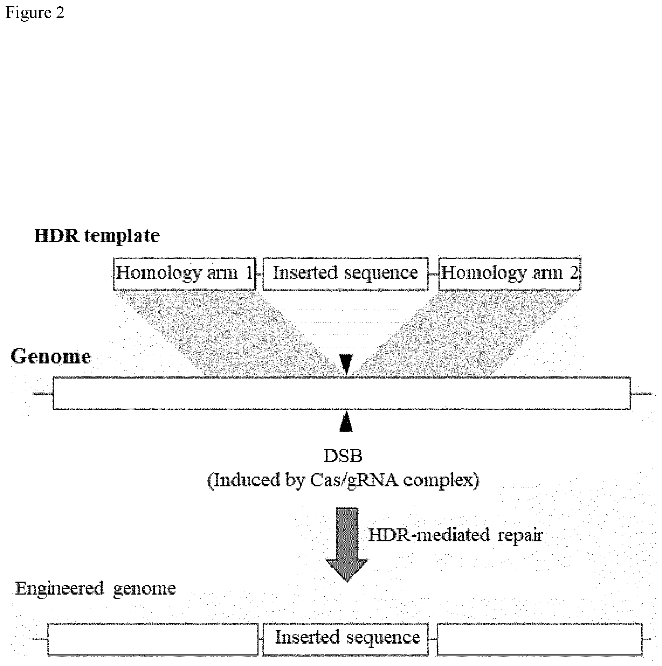

HDR, which is a naturally occurring DNA repair system, may be used to modify the genome in a variety of organisms, including humans. In the gene (for example, genome) editing process using a CRISPR/Cas gene editing system, HDR-mediated repair may be used to insert (e.g., knock-in) a desired sequence into a target region or target site or to induce a specific point mutation, etc. HDR-mediated repair is performed through HDR, a DNA repair system, and a donor template supplied from outside the cell. The term "donor template" may be used interchangeably with "HDR template." An example of an HDR-mediated repair process involving an HDR template is shown in Figure 02. As shown in Figure 02, a HDR template may comprise two homology arms and an insertion sequence. At this time, an upstream homology arm (UHA) of the two homology arms is designed to have homology or complementarity with the DNA sequence on the genome that is present upstream based on the location in which DSB occurs. A downstream homology arm (DHA) of the two homology arms is designed to have homology or complementarity with the DNA sequence on the genome that is present downstream based on the location in which DSB occurs. Ultimately, the two homology arms serve to assist in the process of integrating the insertion sequence into the genome of a cell. The HDR-mediated repair process is disclosed in detail in Sander, Jeffry D., and J. Keith Joung. "CRISPR-Cas systems for editing, regulating and targeting genomes." Nature biotechnology 32.4 (2014): 347-355, the contents of which are incorporated herein by reference in its entirety.

### Design of HDR template comprising the promoter of the present application and a transgene

The desired sequence may be inserted into a target site using HDR-mediated repair and an HDR template. Any one or more of the promoters of the present application and a transgene may be integrated into the genome of a cell through an HDR-mediated repair process. For example, if the nucleic acid of the present application comprises the promoter of the present application and a transgene and further comprises separate UHA and DHA, the region comprising the promoter of the present application and a transgene may be inserted into a target site related to the designed UHA and DHA. As another example, if the nucleic acid of the present application comprises the UHA and DHA of the present application and a transgene, and the UHA comprises the sequence of the promoter of the present application or a sequence complementary thereto, the transgene may be inserted near a region comprising a sequence having sequence identity to the promoter sequence of the present application present in the genome or a sequence complementary thereto. At this time, the target site will be designed downstream of a region comprising a sequence having sequence identity to the promoter sequence of the present application or a region comprising a sequence complementary thereto. For example, the target site may be designed into a region of intron 1 of the APOC3 gene on the genome. When designing UHA comprising the promoter sequence of the present application or a sequence complementary thereto, DHA may be designed based on a target site to be set. In the case of the two examples described above, a transgene may be expressed without being inserted into the genome of a cell, or it may be expressed by being inserted into the genome of a cell. In other words, the expression efficiency of a transgene within a cell may be increased by inducing the expression of a transgene outside and inside the genome of a cell with a single HDR template.

Hereinafter, the two usage aspects described above are specifically disclosed.

### Use (2) of the promoter of the present application

### Overview of use (2) of the promoter of the present application

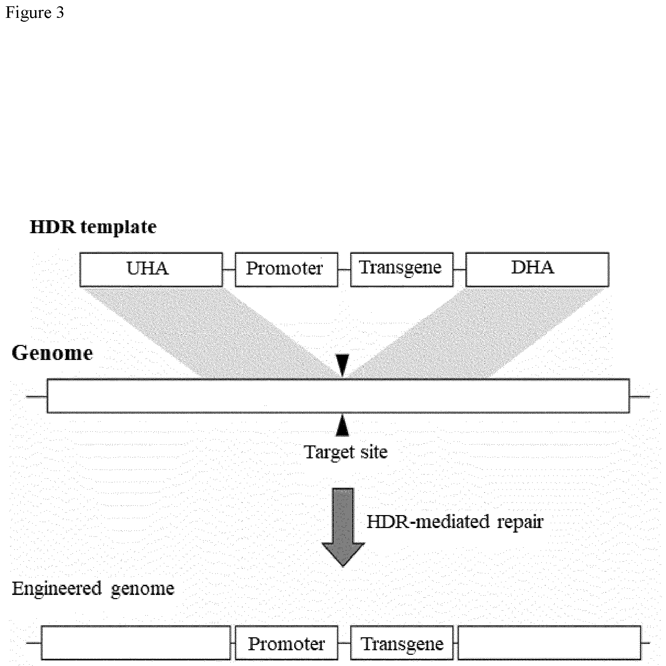

As described above, a nucleic acid comprising the promoter of the present application and a transgene may be used in conjunction with a gene editing system (for example, a gene editing system involving the use of a Cas protein and a guide RNA). In order to insert the promoter of the present application and a transgene into the genome of a cell, an HDR template may be provided. In one embodiment, the HDR template may comprise the promoter of the present application and a transgene. In addition to the promoter of the present application and a transgene, the HDR template may comprise an upstream homology arm (UHA) and a downstream homology arm (DHA) for inserting the promoter of the present application and a transgene into a target site within the genome of a cell. Through the HDR template provided according to one embodiment of the present application, a nucleic acid comprising the promoter of the present application and a transgene may be integrated into the genome of a cell (for example, a hepatocyte). A transgene inserted into the genome of a cell may be transcribed within the genome of a cell through the promoter of the present application to be inserted together. Furthermore, a transgene that is not integrated into the genome of a cell may also be transcribed through the operably linked promoter of the present application. A target site may be freely determined depending on the design of an upstream homology arm and a downstream homology arm. Of course, the PAM sequence located on the genome and the location in which DSB occurs will be considered together. An example of a use (2) of the promoter of the present application is shown in Figure 03. As shown in Figure 03, an engineered genome comprising the promoter of the present application and a transgene is formed by an HDR-mediated repair process involving an HDR template comprising UHA, the promoter of the present application, a transgene, and DHA.

### Promoter of the present application

The HDR template described above comprises the promoter of the present application. The promoter of the present application is disclosed in detail in the sections "Novel promoter provided by the present application" and "Nucleic acid construct comprising the novel promoter provided by the present application".

### Transgene

The HDR template described above comprises a transgene. A transgene is disclosed in detail in the section "Nucleic acid construct comprising the novel promoter provided by the present application".

### Nucleic acid intended for insertion into genome

In a usage aspect (2) of the promoter of the present application, the element intended for insertion into the genome is both the promoter of the present application and a transgene. The transgene inserted into the genome may be transcribed through a co-inserted promoter, and eventually a target protein may be produced through this.

### Design of target site or insertion site of transgene

In a usage aspect (2) of the promoter of the present application, since the transgene inserted into the genome may be transcribed through a co-inserted promoter, there is no need for the endogenous promoter present on the genome to be used for the expression of a transgene. Therefore, the insertion site of the transgene is not otherwise limited. In other words, a target site or target region that may be designed by a guide sequence of a guide RNA and a PAM sequence is not otherwise limited. In one embodiment, a target site may be set within a safe harbor region. A safe harbor region is a specific region in the genome where the insertion of a foreign gene, such as a transgene, does not cause serious side effects, such as cancer, and the transgene inserted within this specific region may be permanently or semi-permanently and safely expressed at a high level. A gene that may function as a safe harbor region may be, for example, ALB, FTL, FTH1, ACTB, HP, APOC3, SOD2, ORM1 AAVS1, Rosa, HPRT, or CCR5, but is not limited thereto. In one embodiment, a guide sequence of a guide RNA is capable of binding complementary to a sequence located within the target region of the spacer binding-strand, wherein the target region may be located within a safe harbor gene selected from ALB, FTL, FTH1, ACTB, HP, APOC3, SOD2, ORM1 AAVS1, Rosa, HPRT, and CCR5.

### Transgene expression process

In a usage aspect (2) of the promoter of the present application, a transgene may be used in conjunction with a gene editing system and inserted into the genome of a cell. However, a transgene that is still not inserted into the genome of a cell may also be present in the same cells described above or in other cells. A transgene that is inserted into the genome of a cell may be transcribed by a co-inserted promoter. Separately, when a transgene that is not inserted into the genome of a cell is present, a transgene that is not inserted into the genome of a cell may also be transcribed by an operably linked promoter. Ultimately, the expression efficiency of a transgene within a cell may be increased by inducing the expression of a transgene outside and inside the genome of a cell with a single HDR template.

### Design of HDR template - nucleic acid construct 2

Hereinafter, an HDR template that may be used in this usage aspect is disclosed. An HDR template may be used in conjunction with a gene editing system, or may be used alone without a gene editing system.

In one embodiment of the present application, there is provided a nucleic acid construct comprising:
a first nucleic acid comprising the promoter of the present application;
a second nucleic acid comprising a transgene;
a third nucleic acid comprising an upstream homology arm for inserting the promoter of the present application and a transgene into a target site within the genome of a cell; and
a fourth nucleic acid comprising a downstream homology arm for inserting the promoter of the present application and a transgene into a target site within the genome of a cell.

In certain embodiments, the promoter of the present application may be operably linked to a transgene. In certain embodiments, the promoter of the present application may be operably linked to the 5' terminus of a transgene. In certain embodiments, the promoter of the present application may be operably linked to the 3' terminus of a transgene. In certain embodiments, a first nucleic acid comprising the promoter of the present application, a second nucleic acid comprising a transgene, a third nucleic acid comprising an upstream homology arm, and a fourth nucleic acid comprising a downstream homology arm may be arranged in a 5' to 3' direction in the order of a third nucleic acid, a first nucleic acid, a second nucleic acid, and a fourth nucleic acid.

In certain embodiments, the nucleic acid may consist of a DNA sequence. In certain embodiments, the nucleic acid may consist of an RNA sequence. In certain embodiments, the nucleic acid may consist of a DNA/RNA hybrid sequence.

In certain embodiments, each of an upstream homology arm and a downstream homology arm may have a sequence that is identical to or complementary to a sequence of a target site or a region adjacent thereto. In certain embodiments, each of the upstream homology arm and the downstream homology arm may have a sequence that is homologous but not completely identical to a sequence of a target site or a region adjacent thereto, or may have a sequence that is complementary but not completely complementary. In certain embodiments, each of the upstream homology arm and the downstream homology arm may have a sequence having at least 80%, 85%, 90%, or 95% homology to a sequence of a target site or a region adjacent thereto, or a sequence having at least 80%, 85%, 90%, or 95% complementarity to a sequence of a target site or a region adjacent thereto.

In certain embodiments, each of an upstream homology arm and a downstream homology arm may have a length of 100 nt to 5000 nt or 100 bp to 5000 bp. In certain embodiments, each of an upstream homology arm and a downstream homology arm may have a length of 300 nt to 3000 nt or 300 bp to 3000 bp. In certain embodiments, each of an upstream homology arm and a downstream homology arm may have a length of 300 nt to 1500 nt or 300 bp to 1500 bp. In certain embodiments, each of an upstream homology arm and a downstream homology arm may have a length of 500 nt to 1500 nt or 500 bp to 1500 bp. In certain embodiments, each of an upstream homology arm and a downstream homology arm may have a length of about 500 nt, 550 nt, 600 nt, 650 nt, 700 nt, 750 nt, 800 nt, 850 nt, 900 nt, 950 nt, 1000 nt, 1100 nt, 1200 nt, 1300 nt, 1400 nt, 1500 nt, 500 bp, 550 bp, 600 bp, 650 bp, 700 bp, 750 bp, 800 bp, 850 bp, 900 bp, 950 bp, 1000 bp, 1100 bp, 1200 bp, 1300 bp, 1400 bp, or 1500 bp.

In certain embodiments, a first nucleic acid may have a length of 30 nt to 2000 nt or 30 bp to 2000 bp. In certain embodiments, a first nucleic acid may have a length of 100 nt to 1500 nt or 100 bp to 1500 bp. In certain embodiments, a first nucleic acid may have a length of 300 nt to 1000 nt or 300 bp to 1000 bp. In certain embodiments, a first nucleic acid may have a length of about 50 nt, 55 nt, 60 nt, 65 nt, 70 nt, 75 nt, 80 nt, 85 nt, 90 nt, 100 nt, 110 nt, 120 nt, 130 nt, 140 nt, 150 nt, 160 nt, 170 nt, 180 nt, 190 nt, 200 nt, 220 nt, 240 nt, 260 nt, 280 nt, 300 nt, 320 nt, 340 nt, 340 nt, 360 nt, 380 nt, 400 nt, 420 nt, 440 nt, 460 nt, 480 nt, 500 nt, 550 nt, 600 nt, 650 nt, 700 nt, 750 nt, 800 nt, 850 nt, 900 nt, 950 nt, 1000 nt, 1100 nt, 1200 nt, 1300 nt, 1400 nt, 1500 nt, 50 bp, 55 bp, 60 bp, 65 bp, 70 bp, 75 bp, 80 bp, 85 bp, 90 bp, 100 bp, 110 bp, 120 bp, 130 bp, 140 bp, 150 bp, 160 bp, 170 bp, 180 bp, 190 bp, 200 bp, 220 bp, 240 bp, 260 bp, 280 bp, 300 bp, 320 bp, 340 bp, 340 bp, 360 bp, 380 bp, 400 bp, 420 bp, 440 bp, 460 bp, 480 bp, 500 bp, 550 bp, 600 bp, 650 bp, 700 bp, 750 bp, 800 bp, 850 bp, 900 bp, 950 bp, 1000 bp, 1100 bp, 1200 bp, 1300 bp, 1400 bp, or 1500 bp.

In certain embodiments, a second nucleic acid may have a length of 10 nt to 10000 nt or 10 bp to 10000 bp, but is not limited thereto. In certain embodiments, a second nucleic acid may have a length of 100 nt to 5000 nt or 100 bp to 5000 bp. In certain embodiments, a second nucleic acid may have a length of about 100 nt, 200 nt, 300 nt, 400 nt, 500 nt, 600 nt, 700 nt, 800 nt, 900 nt, 1000 nt, 1100 nt, 1200 nt, 1300 nt, 1400 nt, 1500 nt, 1600 nt, 1700 nt, 1800 nt, 1900 nt, 2000 nt, 2100 nt, 2200 nt, 2300 nt, 2400 nt, 2500 nt, 2600 nt, 2700 nt, 2800 nt, 2900 nt, 3000 nt, 3100 nt, 3200 nt, 3300 nt, 3400 nt, 3500 nt, 3600 nt, 3700 nt, 3800 nt, 3900 nt, 4000 nt, 4100 nt, 4200 nt, 4300 nt, 4400 nt, 4500 nt, 4600 nt, 4700 nt, 4800 nt, 4900 nt, 5000 nt, 5100 nt, 5200 nt, 5300 nt, 5400 nt, 5500 nt, 5600 nt, 5700 nt, 5800 nt, 5900 nt, 6000 nt, 6100 nt, 6200 nt, 6300 nt, 6400 nt, 6500 nt, 6600 nt, 6700 nt, 6800 nt, 6900 nt, 7000 nt, 7100 nt, 7200 nt, 7300 nt, 7400 nt, 7500 nt, 7600 nt, 7700 nt, 7800 nt, 7900 nt, 8000 nt, 8100 nt, 8200 nt, 8300 nt, 8400 nt, 8500 nt, 8600 nt, 8700 nt, 8800 nt, 8900 nt, 9000 nt, 9100 nt, 9200 nt, 9300 nt, 9400 nt, 9500 nt, 9600 nt, 9700 nt, 9800 nt, 9900 nt, 10000 nt, 100 bp, 200 bp, 300 bp, 400 bp, 500 bp, 600 bp, 700 bp, 800 bp, 900 bp, 1000 bp, 1100 bp, 1200 bp, 1300 bp, 1400 bp, 1500 bp, 1600 bp, 1700 bp, 1800 bp, 1900 bp, 2000 bp, 2100 bp, 2200 bp, 2300 bp, 2400 bp, 2500 bp, 2600 bp, 2700 bp, 2800 bp, 2900 bp, 3000 bp, 3100 bp, 3200 bp, 3300 bp, 3400 bp, 3500 bp, 3600 bp, 3700 bp, 3800 bp, 3900 bp, 4000 bp, 4100 bp, 4200 bp, 4300 bp, 4400 bp, 4500 bp, 4600 bp, 4700 bp, 4800 bp, 4900 bp, 5000 bp, 5100 bp, 5200 bp, 5300 bp, 5400 bp, 5500 bp, 5600 bp, 5700 bp, 5800 bp, 5900 bp, 6000 bp, 6100 bp, 6200 bp, 6300 bp, 6400 bp, 6500 bp, 6600 bp, 6700 bp, 6800 bp, 6900 bp, 7000 bp, 7100 bp, 7200 bp, 7300 bp, 7400 bp, 7500 bp, 7600 bp, 7700 bp, 7800 bp, 7900 bp, 8000 bp, 8100 bp, 8200 bp, 8300 bp, 8400 bp, 8500 bp, 8600 bp, 8700 bp, 8800 bp, 8900 bp, 9000 bp, 9100 bp, 9200 bp, 9300 bp, 9400 bp, 9500 bp, 9600 bp, 9700 bp, 9800 bp, 9900 bp, or 10000 bp.

In certain embodiments, a nucleic acid construct may consist of a nucleotide sequence. A nucleic acid construct may consist of a DNA sequence. In certain embodiments, a nucleic acid construct may consist of an RNA sequence. In certain embodiments, a nucleic acid construct may consist of a DNA/RNA hybrid sequence.

In certain embodiments, a nucleic acid construct may have a length of 100 nt to 1000000 nt or 100 bp to 1000000 bp. In certain embodiments, a nucleic acid construct may have a length of 300 nt to 100000 nt or 300 bp to 100000 bp, but is not otherwise limited. In certain embodiments, a nucleic acid construct may have a length of 300 nt to 10000 nt or 300 bp to 10000 bp, but is not otherwise limited. In certain embodiments, a nucleic acid construct may have a length of 500 nt to 10000 nt or 500 bp to 10000 bp, but is not otherwise limited. In certain embodiments, a nucleic acid construct may have a length of 100000 nt or 100000 bp or less, but is not limited thereto. In certain embodiments, a nucleic acid construct may have a length of 50000 nt or 50000 bp or less, but is not limited thereto. In certain embodiments, a nucleic acid construct may have a length of 10000 nt or 10000 bp or less, but is not limited thereto. In certain embodiments, a nucleic acid construct may have a length of 5000 nt or 5000 bp or less, but is not limited thereto. In certain embodiments, a nucleic acid construct may have a length of 3000 nt or 3000 bp or less, but is not limited thereto.

In certain embodiments, a nucleic acid construct may be provided as a double strand. In certain embodiments, a nucleic acid construct may be provided as a single strand.

### Gene editing method

Hereinafter, a method for editing the genome of a cell using a CRISPR/Cas gene editing system and the HDR template described above is disclosed. In one embodiment of the present application, there is provided a method for editing the genome of a cell. In one embodiment of the present application, there is provided a method for producing an engineered cell. In one embodiment of the present application, there is provided a method for producing an engineered cell comprising an engineered genome. At this time, an engineered genome comprises the promoter of the present application and a transgene. A method for producing an engineered cell may be achieved by a method for editing the genome of a cell.

In one embodiment, a method for editing the genome of a cell may comprise:
introducing a Cas protein or a nucleic acid encoding the same, a guide RNA or a nucleic acid encoding the same, and an HDR template or a nucleic acid encoding the same into the cell,
wherein the HDR template comprises a first nucleic acid comprising the promoter of the present application, a second nucleic acid comprising a transgene, a third nucleic acid comprising an upstream homology arm for inserting the promoter of the present application and a transgene into a target site within the genome of a cell, and a fourth nucleic acid comprising a downstream homology arm for inserting the promoter of the present application and a transgene into a target site within the genome of a cell, and
wherein the guide RNA comprises a guide domain having a guide sequence, and wherein the guide sequence may consist of a sequence having complementarity to a sequence on the genome within the target region comprising a target site.

At this time, the Cas protein and the guide RNA form a complex within the cell, and the Cas protein may be localized to the target region by a guide sequence. At this time, the Cas protein may be localized to the target region by a guide sequence and may generate a DSB or nick at a target site within the target region.

At this time, the promoter of the present application and a transgene may be inserted into a target site within the target region on the genome of a cell through an HDR-mediated DNA repair process with an HDR template comprising an upstream homology arm and a downstream homology arm designed to insert the promoter of the present application and a transgene into a target site within the genome of a cell. At this time, the target site may be located within a gene related to a safe harbor.

### Gene editing composition

Hereinafter, a composition for editing the genome of a cell using a CRISPR/Cas gene editing system and the HDR template described above is disclosed. In one embodiment of the present application, there is provided a composition for editing the genome of a cell.

In one embodiment of the present application, there is provided a composition for editing the genome of a cell comprising:
a Cas protein or a nucleic acid encoding the same,
a guide RNA or a nucleic acid encoding the same, and
an HDR template or a nucleic acid encoding the same.

At this time, the HDR template may comprise a first nucleic acid comprising the promoter of the present application, a second nucleic acid comprising a transgene, a third nucleic acid comprising an upstream homology arm for inserting the promoter of the present application and a transgene into a target site within the genome of a cell, and a fourth nucleic acid comprising a downstream homology arm for inserting the promoter of the present application and a transgene into a target site within the genome of a cell.

### Use (3) of the promoter of the present application

### Overview of use (3) of the promoter of the present application

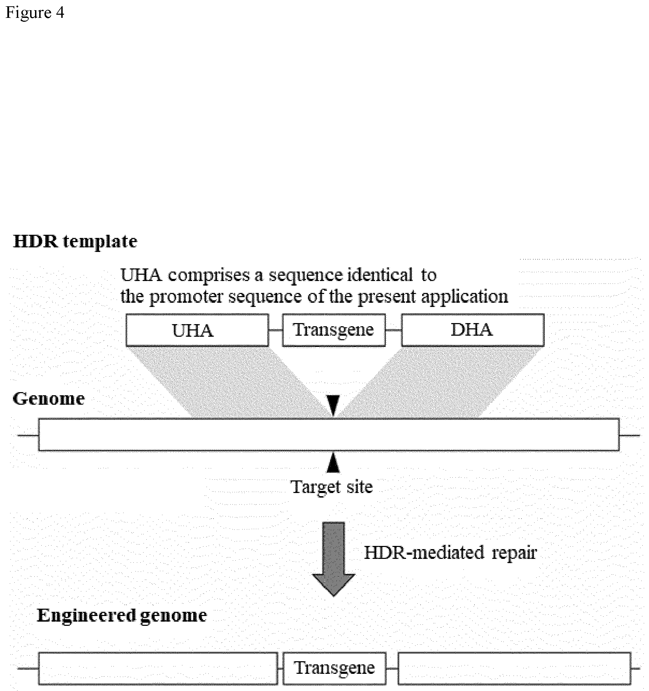

As described above, a nucleic acid comprising the promoter of the present application and a transgene may be used in conjunction with a gene editing system (for example, a gene editing system involving the use of a Cas protein and a guide RNA). In order to insert a transgene into the genome of a cell, an HDR template may be provided. In one embodiment, the HDR template may comprise the promoter of the present application and a transgene. In addition to the promoter of the present application and a transgene, the HDR template may further comprise one homology arm (for example, a downstream homology arm) for inserting the transgene of the present application into a target site within the genome of a cell. During the insertion of a transgene through HDR-mediated repair process using an HDR template, two homology arms are generally required. In a usage aspect (3) of the promoter of the present application, one homology arm of the two homology arms may be designed to comprise the promoter sequence of the present application or a sequence complementary thereto. In one embodiment, the homology arm designed to comprise the promoter sequence of the present application or a sequence complementary thereto may be an upstream homology arm. In one embodiment, the homology arm designed to comprise the promoter sequence of the present application or a sequence complementary thereto may be a downstream homology arm. One of the two homology arms is designed to comprise the promoter sequence of the present application or a sequence complementary thereto, such that the transgene may be inserted into or near a region on the genome having a sequence identical to the promoter sequence of the present application or a sequence complementary thereto. In this situation, it is noted that the promoter of the present application introduced from outside may not be inserted into the genome. In other words, a transgene is inserted near a region on the genome having a sequence identical to the promoter of the present application, and a region on the genome having a sequence identical to the promoter of the present application present in the genome is intended to be used as a promoter to initiate the transcription of a transgene. The transgene is integrated into the genome of a cell and may be transcribed through a newly invented, promoter-functioning region that has a sequence identical to the promoter of the present application present in the genome of a cell. Meanwhile, a transgene that is not integrated into the genome of a cell may also be present. A transgene that is not integrated into the genome of a cell may be transcribed through the operably linked promoter of the present application. An example of a use (3) of the promoter of the present application is shown in Figure 04. As shown in Figure 04, the transgene is integrated into a designated region on the genome by an HDR-mediated repair process involving an HDR template comprising UHA comprising the promoter of the present application, a transgene, and DHA, and an engineered genome comprising a transgene is formed.

### Promoter of the present application

The HDR template described above comprises the promoter of the present application. The promoter of the present application is disclosed in detail in the sections "Novel promoter provided by the present application" and "Nucleic acid construct comprising the novel promoter provided by the present application".

### Transgene

The HDR template described above comprises a transgene. A transgene is disclosed in detail in the section "Nucleic acid construct comprising the novel promoter provided by the present application".

### Nucleic acid intended for insertion into genome

In a usage aspect (3) of the promoter of the present application, the element intended for insertion into the genome is a transgene. An HDR template may be designed so that a region having a sequence identical to a sequence of the promoter of the present application present in the genome or a sequence complementary thereto may be used as a promoter for the transgene inserted into the genome. The transgene inserted into the genome may be transcribed through a region having a sequence identical to a sequence of the promoter of the present application present in the genome or a sequence complementary thereto, and eventually a target protein may be produced through this.

### Design of target site or insertion site of transgene

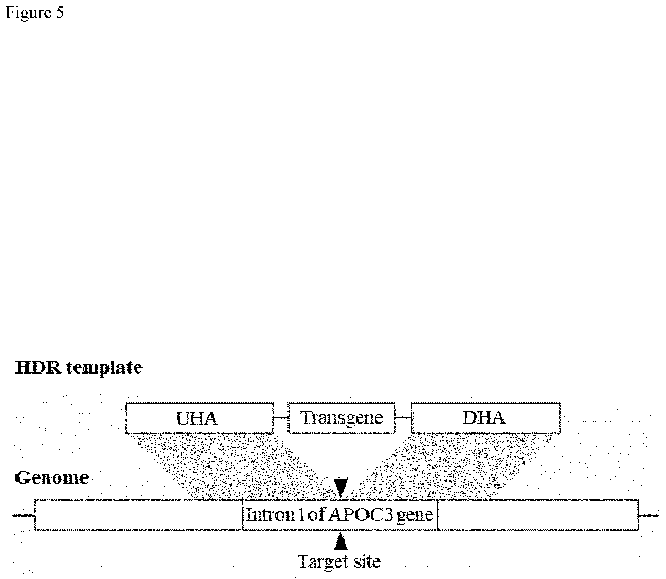

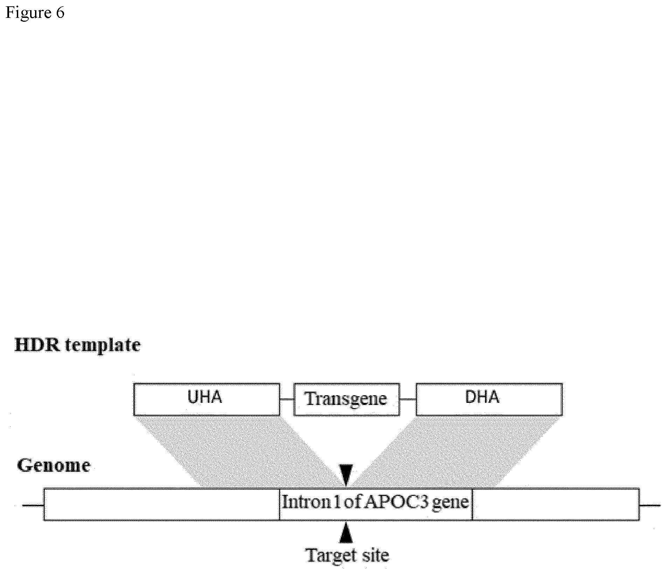

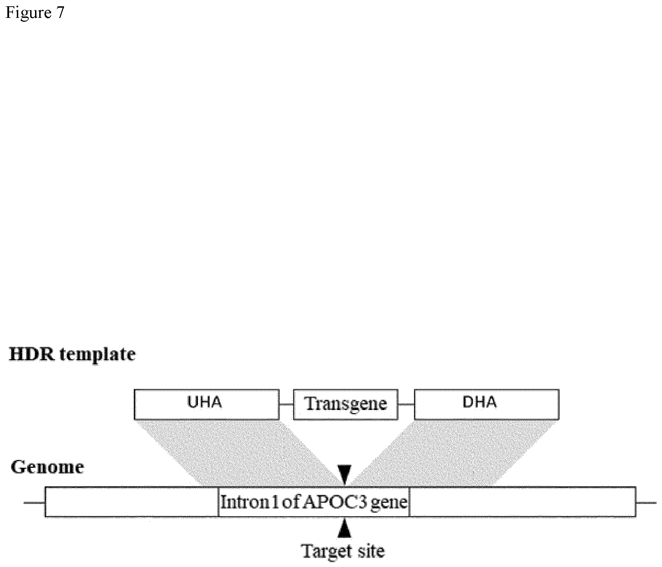

In a usage aspect (3) of the promoter of the present application, since the transgene inserted into the genome should be transcribed through a region having a sequence identical to a sequence of the promoter of the present application present on the genome, the insertion site of the transgene is determined within a designated region. In other words, a target site or target region that may be designed in a guide sequence of a guide RNA and a PAM sequence is designed to target a target sequence within a designated region. At this time, the designated region comprising a target site or target region may be the APOC3 gene (for example, the human APOC3 gene, the mouse APOC3 gene, or the rat APOC3 gene). At this time, the designated region comprising a target site or target region may be intron 1 of the APOC3 gene (for example, intron 1 of the human APOC3 gene, intron 1 of the mouse APOC3 gene, and intron 1 of the rat APOC3 gene). For example, an upstream homology arm included in an HDR template that may be used in a usage aspect (3) of the promoter of the present application (wherein an upstream homology arm comprises the promoter of the present application) may comprise a sequence of a region adjacent to the 5' of intron 1 of the APOC3 gene. At this time, the downstream homology arm included in an HDR template may comprise a sequence of a region adjacent to the 3' of APOC3 intron 1. Examples of a target site, an upstream homology arm, and a downstream homology arm that may be designed in a usage aspect (3) of the promoter of the present application are shown in Figures 05 to 07.

### Transgene expression process

In a usage aspect (3) of the promoter of the present application, a transgene may be used in conjunction with a gene editing system and inserted into the genome of a cell. However, a transgene that is still not inserted into the genome of a cell may also be present in the same cells described above or in other cells. A transgene that is inserted into the genome of a cell may be transcribed by a region having a sequence identical to the promoter of the present application present on the genome of a cell or by a different promoter. Separately, when a transgene that is not inserted into the genome of a cell is present, a transgene that is not inserted into the genome of a cell may also be transcribed by an operably linked promoter. Ultimately, the expression efficiency of a transgene within a cell may be increased by inducing the expression of a transgene outside and inside the genome of a cell with a single HDR template.

### Design of HDR template - nucleic acid construct 3

Hereinafter, an HDR template that may be used in this usage aspect is disclosed. An HDR template may be used in conjunction with a gene editing system, or may be used alone without a gene editing system.

In one embodiment of the present application, there is provided a nucleic acid construct comprising:
a first nucleic acid comprising the promoter of the present application;
a second nucleic acid comprising a transgene; and
a third nucleic acid comprising a first region.

In certain embodiments, a first region included in a third nucleic acid has a sequence identical to the sequence of a portion of intron 1 of the APOC3 gene, and may be different from a sequence of the promoter of the present application.

In certain embodiments, a first nucleic acid comprises a second region, wherein the second region has a sequence identical to the sequence of a portion of intron 1 of the APOC3 gene, and wherein a first region included in a third nucleic acid has a sequence identical to the sequence of a portion of intron 1 of the APOC3 gene, and wherein the sequence of a second region may be different from the sequence of a first region.

In certain embodiments, the promoter of the present application may be operably linked to a transgene. In certain embodiments, the promoter of the present application may be operably linked to the 5' terminus of a transgene. In certain embodiments, the promoter of the present application may be operably linked to the 3' terminus of a transgene.

In certain embodiments, a first nucleic acid comprising the promoter of the present application, a second nucleic acid comprising a transgene, a third nucleic acid comprising a first region may be arranged in a 5' to 3' direction in the order of a first nucleic acid, a second nucleic acid, and a third nucleic acid.

In certain embodiments, the nucleic acid may consist of a DNA sequence. In certain embodiments, the nucleic acid may consist of an RNA sequence. In certain embodiments, the nucleic acid may consist of a DNA/RNA hybrid sequence.

In certain embodiments, a first nucleic acid may comprise an upstream homology arm for inserting a transgene into a target site within the genome of a cell.

In certain embodiments, a third nucleic acid may comprise a downstream homology arm for inserting a transgene into a target site within the genome of a cell.

In certain embodiments, a target site may be located within intron 1 of the APOC3 gene. In certain embodiments, a target site may be present within the sequence GAGAGGGCCAGAAATCACCCAA (SEQ ID NO: 40) on the genome.

In certain embodiments, each of an upstream homology arm and a downstream homology arm may have a sequence that is identical to or complementary to a sequence of a target site or a region adjacent thereto. In certain embodiments, each of the upstream homology arm and the downstream homology arm may have a sequence that is homologous but not completely identical to a sequence of a target site or a region adjacent thereto, or may have a sequence that is complementary but not completely complementary. In certain embodiments, each of the upstream homology arm and the downstream homology arm may have a sequence having at least 80%, 85%, 90%, or 95% homology to a sequence of a target site or a region adjacent thereto, or a sequence having at least 80%, 85%, 90%, or 95% complementarity to a sequence of a target site or a region adjacent thereto.

In certain embodiments, each of an upstream homology arm and a downstream homology arm may have a length of 100 nt to 5000 nt or 100 bp to 5000 bp. In certain embodiments, each of an upstream homology arm and a downstream homology arm may have a length of 300 nt to 3000 nt or 300 bp to 3000 bp. In certain embodiments, each of an upstream homology arm and a downstream homology arm may have a length of 500 nt to 1500 nt or 500 bp to 1500 bp. In certain embodiments, each of an upstream homology arm and a downstream homology arm may have a length of about 100 nt, 110 nt, 120 nt, 130 nt, 140 nt, 150 nt, 160 nt, 170 nt, 180 nt, 190 nt, 200 nt, 220 nt, 240 nt, 260 nt, 280 nt, 300 nt, 320 nt, 340 nt, 340 nt, 360 nt, 380 nt, 400 nt, 420 nt, 440 nt, 460 nt, 480 nt, 500 nt, 550 nt, 600 nt, 650 nt, 700 nt, 750 nt, 800 nt, 850 nt, 900 nt, 950 nt, 1000 nt, 1100 nt, 1200 nt, 1300 nt, 1400 nt, 1500 nt, 100 bp, 110 bp, 120 bp, 130 bp, 140 bp, 150 bp, 160 bp, 170 bp, 180 bp, 190 bp, 200 bp, 220 bp, 240 bp, 260 bp, 280 bp, 300 bp, 320 bp, 340 bp, 340 bp, 360 bp, 380 bp, 400 bp, 420 bp, 440 bp, 460 bp, 480 bp, 500 bp, 550 bp, 600 bp, 650 bp, 700 bp, 750 bp, 800 bp, 850 bp, 900 bp, 950 bp, 1000 bp, 1100 bp, 1200 bp, 1300 bp, 1400 bp, or 1500 bp.

In certain embodiments, a second nucleic acid may have a length of 10 nt to 10000 nt or 10 bp to 10000 bp, but is not limited thereto. In certain embodiments, a second nucleic acid may have a length of 100 nt to 5000 nt or 100 bp to 5000 bp. In certain embodiments, a second nucleic acid may have a length of about 100 nt, 200 nt, 300 nt, 400 nt, 500 nt, 600 nt, 700 nt, 800 nt, 900 nt, 1000 nt, 1100 nt, 1200 nt, 1300 nt, 1400 nt, 1500 nt, 1600 nt, 1700 nt, 1800 nt, 1900 nt, 2000 nt, 2100 nt, 2200 nt, 2300 nt, 2400 nt, 2500 nt, 2600 nt, 2700 nt, 2800 nt, 2900 nt, 3000 nt, 3100 nt, 3200 nt, 3300 nt, 3400 nt, 3500 nt, 3600 nt, 3700 nt, 3800 nt, 3900 nt, 4000 nt, 4100 nt, 4200 nt, 4300 nt, 4400 nt, 4500 nt, 4600 nt, 4700 nt, 4800 nt, 4900 nt, 5000 nt, 5100 nt, 5200 nt, 5300 nt, 5400 nt, 5500 nt, 5600 nt, 5700 nt, 5800 nt, 5900 nt, 6000 nt, 6100 nt, 6200 nt, 6300 nt, 6400 nt, 6500 nt, 6600 nt, 6700 nt, 6800 nt, 6900 nt, 7000 nt, 7100 nt, 7200 nt, 7300 nt, 7400 nt, 7500 nt, 7600 nt, 7700 nt, 7800 nt, 7900 nt, 8000 nt, 8100 nt, 8200 nt, 8300 nt, 8400 nt, 8500 nt, 8600 nt, 8700 nt, 8800 nt, 8900 nt, 9000 nt, 9100 nt, 9200 nt, 9300 nt, 9400 nt, 9500 nt, 9600 nt, 9700 nt, 9800 nt, 9900 nt, 10000 nt, 100 bp, 200 bp, 300 bp, 400 bp, 500 bp, 600 bp, 700 bp, 800 bp, 900 bp, 1000 bp, 1100 bp, 1200 bp, 1300 bp, 1400 bp, 1500 bp, 1600 bp, 1700 bp, 1800 bp, 1900 bp, 2000 bp, 2100 bp, 2200 bp, 2300 bp, 2400 bp, 2500 bp, 2600 bp, 2700 bp, 2800 bp, 2900 bp, 3000 bp, 3100 bp, 3200 bp, 3300 bp, 3400 bp, 3500 bp, 3600 bp, 3700 bp, 3800 bp, 3900 bp, 4000 bp, 4100 bp, 4200 bp, 4300 bp, 4400 bp, 4500 bp, 4600 bp, 4700 bp, 4800 bp, 4900 bp, 5000 bp, 5100 bp, 5200 bp, 5300 bp, 5400 bp, 5500 bp, 5600 bp, 5700 bp, 5800 bp, 5900 bp, 6000 bp, 6100 bp, 6200 bp, 6300 bp, 6400 bp, 6500 bp, 6600 bp, 6700 bp, 6800 bp, 6900 bp, 7000 bp, 7100 bp, 7200 bp, 7300 bp, 7400 bp, 7500 bp, 7600 bp, 7700 bp, 7800 bp, 7900 bp, 8000 bp, 8100 bp, 8200 bp, 8300 bp, 8400 bp, 8500 bp, 8600 bp, 8700 bp, 8800 bp, 8900 bp, 9000 bp, 9100 bp, 9200 bp, 9300 bp, 9400 bp, 9500 bp, 9600 bp, 9700 bp, 9800 bp, 9900 bp, or 10000 bp.

In certain embodiments, a nucleic acid construct may consist of a nucleotide sequence. A nucleic acid construct may consist of a DNA sequence. In certain embodiments, a nucleic acid construct may consist of an RNA sequence. In certain embodiments, a nucleic acid construct may consist of a DNA/RNA hybrid sequence.

In certain embodiments, a nucleic acid construct may have a length of 100 nt to 1000000 nt or 100 bp to 1000000 bp. In certain embodiments, a nucleic acid construct may have a length of 300 nt to 100000 nt or 300 bp to 100000 bp, but is not otherwise limited. In certain embodiments, a nucleic acid construct may have a length of 300 nt to 10000 nt or 300 bp to 10000 bp, but is not otherwise limited. In certain embodiments, a nucleic acid construct may have a length of 500 nt to 10000 nt or 500 bp to 10000 bp, but is not otherwise limited. In certain embodiments, a nucleic acid construct may have a length of 100000 nt or 100000 bp or less, but is not limited thereto. In certain embodiments, a nucleic acid construct may have a length of 50000 nt or 50000 bp or less, but is not limited thereto. In certain embodiments, a nucleic acid construct may have a length of 10000 nt or 10000 bp or less, but is not limited thereto. In certain embodiments, a nucleic acid construct may have a length of 5000 nt or 5000 bp or less, but is not limited thereto. In certain embodiments, a nucleic acid construct may have a length of 3000 nt or 3000 bp or less, but is not limited thereto.

In certain embodiments, a nucleic acid construct may be provided as a double strand. In certain embodiments, a nucleic acid construct may be provided as a single strand.

### Homology arm (sequence) of nucleic acid construct 3

Hereinafter, sequences that may be included in a homology arm (for example, a downstream homology arm) of the HDR template described above are disclosed. A downstream homology arm may comprise any one of the sequences of SEQ ID NO: 07 to SEQ ID NO: 08.

### Gene editing method

Hereinafter, a method for editing the genome of a cell using a CRISPR/Cas gene editing system and the HDR template described above is disclosed. In one embodiment of the present application, there is provided a method for editing the genome of a cell. In one embodiment of the present application, there is provided a method for producing an engineered cell. In one embodiment of the present application, there is provided a method for producing an engineered cell comprising an engineered genome. At this time, an engineered genome comprises a transgene. At this time, the transgene may be located within the engineered intron 1 of the APOC3 gene. A method for producing an engineered cell may be achieved by a method for editing the genome of a cell.

In one embodiment, a method for editing the genome of a cell may comprise:
introducing a Cas protein or a nucleic acid encoding the same, a guide RNA or a nucleic acid encoding the same, and an HDR template or a nucleic acid encoding the same into the cell,
wherein the HDR template comprises a first nucleic acid comprising the promoter of the present application, a second nucleic acid comprising a transgene, and a third nucleic acid comprising a first region, and
wherein the guide RNA comprises a guide domain having a guide sequence, and wherein the guide sequence may consist of a sequence having complementarity to a sequence in the target region on the genome where a target site is present.

At this time, the guide sequence of a guide RNA may be designed to target the sequence GAGAGGGCCAGAAATCACCCAA (SEQ ID NO: 40).

At this time, the target region or target site may be located within intron 1 of the APOC3 gene (for example, intron 1 of the human APOC3 gene, intron 1 of the mouse APOC3 gene, and intron 1 of the rat APOC3 gene).

At this time, the Cas protein and the guide RNA form a complex within the cell, and the Cas protein may be localized to the target region by a guide sequence. At this time, the Cas protein may be localized to the target region by a guide sequence and may generate a DSB or nick at a target site within the target region.

At this time, the first nucleic acid may be a homology arm for inserting the transgene into the target region on the genome. For example, the first nucleic acid may be an upstream homology arm.

At this time, the third nucleic acid may be a homology arm for inserting the transgene into the target region on the genome. For example, the third nucleic acid may be a downstream homology arm.

At this time, the transgene may be inserted into a target site within the target region on the genome of a cell through an HDR-mediated DNA repair process with an HDR template comprising an upstream homology arm and a downstream homology arm designed to insert a transgene into a target site within the genome of a cell. At this time, the target site may be located within intron 1 of the APOC3 gene.

### Gene editing composition

Hereinafter, a composition for editing the genome of a cell using a CRISPR/Cas gene editing system and the HDR template described above is disclosed. In one embodiment of the present application, there is provided a composition for editing the genome of a cell.

In one embodiment of the present application, there is provided a composition for editing the genome of a cell comprising:
a Cas protein or a nucleic acid encoding the same,
a guide RNA or a nucleic acid encoding the same, and
an HDR template or a nucleic acid encoding the same.

At this time, the HDR template may comprise a first nucleic acid comprising the promoter of the present application, a second nucleic acid comprising a transgene, and a third nucleic acid comprising a first region.

### Introduction of nucleic acid or nucleic acid construct

### Overview of introduction of nucleic acid or nucleic acid construct

A nucleic acid or nucleic acid construct (for example, HDR template) comprising the promoter of the present application may be introduced into a cell and function. For example, a nucleic acid or nucleic acid construct comprising the promoter of the present application and a transgene may be introduced into a cell, and a transgene may be expressed within a cell. Furthermore, in order to manipulate the genome of a cell, it is necessary to introduce the elements used in a gene editing system into a cell (for example, Cas protein, guide RNA, and HDR template, etc.) or produce them within a cell (for example, expression). Hereinafter, a method for introducing the elements disclosed in the present application into a cell is disclosed.

For example, a nucleic acid or nucleic acid construct comprising the promoter of the present application may be included in a vector and introduced into a cell. For example, it may be introduced into a cell by vector-based introduction. In another embodiment, a nucleic acid or nucleic acid construct comprising the promoter of the present application may be introduced into a cell in the form of a non-vector. For example, it may be introduced into a cell in the form of a non-vector.

The nucleic acid or nucleic acid construct of the present application (for example, nucleic acid construct 1, nucleic acid construct 2, or nucleic acid construct 3) or a nucleic acid encoding the same, a Cas protein or a nucleic acid encoding the same, and/or a guide RNA or a nucleic acid encoding the same may be delivered or introduced into a cell by methods known in the art. Alternatively, the nucleic acid or nucleic acid construct of the present application (for example, nucleic acid construct 1, nucleic acid construct 2, or nucleic acid construct 3) or a nucleic acid encoding the same, a Cas protein or a nucleic acid encoding the same, and/or a guide RNA or a nucleic acid encoding the same may be delivered into a subject (for example, a cell) by a vector, a non-vector, or a combination thereof. The vector may be a viral vector or a non-viral vector (for example, a plasmid). The non-vector may be naked DNA, DNA complex, or mRNA.

### Vector-based introduction

The nucleic acid or nucleic acid construct (for example, HDR template) comprising the promoter of the present application or a nucleic acid encoding the same, a Cas protein or a nucleic acid encoding the same, and/or a guide RNA or a nucleic acid encoding the same may be introduced into a cell by vector-based introduction.

For example, a nucleic acid or nucleic acid construct comprising the promoter of the present application may be included in a vector and introduced into a cell.

In one embodiment, the vector may comprise the promoter of the present application or a nucleic acid encoding the same.

In one embodiment, the vector may comprise the promoter of the present application or a nucleic acid encoding the same and a transgene or a nucleic acid encoding the same.

In one embodiment, the vector may comprise any one or more selected from a nucleic acid or nucleic acid construct comprising the promoter of the present application (for example, nucleic acid construct 1, nucleic acid construct 2, or nucleic acid construct 3) or a nucleic acid encoding the same, a Cas protein or a nucleic acid encoding the same, and a guide RNA or a nucleic acid encoding the same. In one embodiment, the vector may comprise a nucleic acid or nucleic acid construct comprising the promoter of the present application, or a nucleic acid encoding the same. In one embodiment, the vector may comprise a Cas protein or a nucleic acid encoding the same. In one embodiment, the vector may comprise a guide RNA or a nucleic acid encoding the same.

In one embodiment, a nucleic acid or nucleic acid construct comprising the promoter of the present application (for example, nucleic acid construct 1, nucleic acid construct 2, or nucleic acid construct 3) or a nucleic acid encoding the same, a Cas protein or a nucleic acid encoding the same, and a guide RNA or a nucleic acid encoding the same may be introduced or delivered into a cell through one or more vectors. For example, a nucleic acid or nucleic acid construct comprising the promoter of the present application or a nucleic acid encoding the same, a Cas protein or a nucleic acid encoding the same, and a guide RNA or a nucleic acid encoding the same may be introduced or delivered into a cell through one, two, three, four, or five vectors.

In one embodiment, the vector may comprise one or more regulatory/control components. At this time, the regulatory/control component may be any one or more selected from promoter, enhancer, intron, polyadenylation signal, Kozak consensus sequence, internal ribosome entry site (IRES), NLS (nuclear localization signal) or a nucleic acid encoding the same, Poly A, a splicing acceptor and 2A sequence. The promoter may be a promoter recognized by RNA polymerase II. The promoter may be a promoter recognized by RNA polymerase III. The promoter may be an inducible promoter. The promoter may be a target-specific promoter. The promoter may be a viral or non-viral promoter. The promoter may be selected as a suitable promoter depending on the control region.

In one embodiment, the vector may be a viral vector or a recombinant viral vector. The virus may be a DNA virus or an RNA virus. At this time, the DNA virus may be a double-stranded DNA (dsDNA) virus or a single-stranded DNA (ssDNA) virus. At this time, the RNA virus may be a single-stranded RNA (ssRNA) virus. The virus may be a retrovirus, lentivirus, adenovirus, adeno-associated virus (AAV), vaccinia virus, pox virus, or herpes simplex virus, but is not limited thereto. For example, the AAV vector may be any one selected from AAV1, AAV2, AAV5, AAV6, AAV8, AAV9, AAVrh. 10, AAVrh.74, and AAVhu.37, but is not limited thereto. Examples of AAV vectors used in research or clinical practice are disclosed in detail in Wang, Dan, Phillip WL Tai, and Guangping Gao. "Adeno-associated viral vector as a platform for gene therapy delivery." Nature reviews Drug discovery 18.5 (2019): 358-378, the contents of which are incorporated herein by reference in its entirety. Generally, a virus may infect a host (for example, a cell) and introduce a nucleic acid encoding the genetic information of the virus into the host or insert a nucleic acid encoding the genetic information into the genome of a host. A virus having these characteristics may be used to introduce a target sequence or a nucleic acid encoding a target protein into a subject (for example, a cell). Furthermore, the target sequence or target protein may be expressed within a host.

### Non-vector-based introduction

A nucleic acid or nucleic acid construct (for example, HDR template) comprising the promoter of the present application or a nucleic acid encoding the same, a Cas protein or a nucleic acid encoding the same, and/or a guide RNA or a nucleic acid encoding the same may be introduced into a cell by non-vector-based introduction.

In one embodiment, any one or more of a nucleic acid or nucleic acid construct comprising the promoter of the present application (for example, nucleic acid construct 1, nucleic acid construct 2, or nucleic acid construct 3) or a nucleic acid encoding the same, a Cas protein or a nucleic acid encoding the same, and a guide RNA or a nucleic acid encoding the same may be introduced into a cell by non-vector-based introduction.

In one embodiment, a nucleic acid or nucleic acid construct comprising the promoter of the present application (for example, nucleic acid construct 1, nucleic acid construct 2, or nucleic acid construct 3) or a nucleic acid encoding the same, a Cas protein or a nucleic acid encoding the same, and a guide RNA or a nucleic acid encoding the same may be introduced or delivered into a cell through one or more non-vectors. For example, a nucleic acid or nucleic acid construct comprising the promoter of the present application or a nucleic acid encoding the same, a Cas protein or a nucleic acid encoding the same, and a guide RNA or a nucleic acid encoding the same may be introduced or delivered into a cell through one, two, three, four, or five non-vectors.

A non-vector may comprise any one or more of a nucleic acid or nucleic acid construct (for example, HDR template) comprising the promoter of the present application or a nucleic acid encoding the same, a Cas protein or a nucleic acid encoding the same, and a guide RNA or a nucleic acid encoding the same. The non-vector may be naked DNA, DNA complex, mRNA, or a combination thereof. The non-vector may be delivered or introduced into a cell by electroporation, gene gun, ultrasonic poration, magnetofection, transient cell compression or squeezing (as disclosed in Lee, et al, (2012) Nano Lett., 12, 6322-6327), lipid-mediated transfection, dendrimers, nanoparticles, calcium phosphate, silica, silicate (ormosil), or a combination thereof. As another example, the non-vector may be delivered using nanoparticles. The nanoparticles may be inorganic nanoparticles (for example, magnetic nanoparticles, silica, etc.) or organic nanoparticles (for example, lipids coated with polyethylene glycol (PEG), etc.). The outer surface of the nanoparticle may be conjugated with a positively charged polymer (for example, polyethyleneimine, polylysine, polyserine, etc.) to enable attachment.

### Delivery or introduction in the form of a peptide, polypeptide, or protein

In one embodiment, an element that has activity in the form of a peptide, polypeptide, or protein within a cell may be delivered or introduced in the form of a peptide, polypeptide, or protein. For example, a Cas protein may be delivered or introduced into a subject by methods known in the art. The form of a peptide, polypeptide, or protein may be delivered or introduced into a cell by electroporation, microinjection, transient cell compression or squeezing (as disclosed in Lee, et al, (2012) Nano Lett., 12, 6322-6327), lipid-mediated transfection, nanoparticles, liposomes, peptide-mediated delivery, or a combination thereof.

### Administration to subject

In one embodiment, a nucleic acid or nucleic acid construct comprising the promoter of the present application (for example, nucleic acid construct 1, nucleic acid construct 2, nucleic acid construct 3) may be administered to a subject. In one embodiment, a composition comprising a nucleic acid or nucleic acid construct comprising the promoter of the present application may be administered to a subject. In one embodiment, the composition may be a composition for expressing transgene. In one embodiment, the composition for expressing transgene may further comprise one or more of the elements used in a gene editing system (for example, a Cas protein or a nucleic acid encoding the same, a guide RNA or a nucleic acid encoding the same). At this time, the nucleic acid or nucleic acid construct may be included in a vector (for example, an AAV vector) (i.e., the vector is used) and administered to a subject. At this time, the subject used with the term "administration" may refer to, for example, a human or a non-human animal. Non-human animals may be, for example, murines such as mice or rats, bovines, canines, equines, felines, ovines, pigs, or primates (non-human primates).

In one embodiment, the composition provided by the present application may further comprise a pharmaceutically acceptable additive (excipient). At this time, the pharmaceutically acceptable additive should be compatible with the active ingredient of the present invention, and saline, sterile water, Ringer's solution, buffered saline, dextrose solution, maltodextrin solution, glycerol, ethanol, and one or two or more of these ingredients may be used in combination, and other conventional additives such as antioxidants, buffers, and bacteriostatic agents may be added as needed. The additive may be appropriately selected from materials well known in the art depending on the purpose of use.

In one embodiment, a composition comprising the nucleic acid or nucleic acid construct of the present application may be administered to a subject and used to produce a transgene in the subject's body.

In one embodiment, a composition comprising the nucleic acid or nucleic acid construct of the present application may be administered in various formulations according to oral and/or parenteral methods when administered to a subject.

In one embodiment, the composition of the present application may be administered to a subject by parenteral administration. At this time, a formulation for parenteral administration comprising the composition of the present application may be prepared and/or used.

The parenteral administration method may be any one selected from intrathecal (IT) injection, intracerebroventricular (ICV) injection, intracranial injection, subcutaneous injection, intravenous injection, intramuscular injection, injection within the substantia nigra pars compacta and the ventral tegmental area, intraparenchymal injection, cisterna magna injection, and intrathoracic injection, or a method injected by a combination thereof.

The formulation for parenteral administration may be prepared as a solution or suspension by mixing in water with a stabilizer or a buffering agent, and it may be prepared in a unit dosage form such as an ampoule or a vial. In addition, the formulation for parenteral administration may comprise auxiliaries such as preservatives, stabilizers, wetting agents or emulsification accelerators, salts and/or buffering agents for osmotic pressure adjustment, and other therapeutically useful substances, and may be formulated by mixing, granulation, or coating in a conventional manner.

In another embodiment, a composition according to one embodiment of the present application may be administered to a subject by oral administration. In one embodiment, a formulation for oral administration comprising the pharmaceutical composition of the present application may be prepared and/or used.

### Exemplary embodiments

Hereinafter, examples of embodiments that may be provided by the present application are disclosed. The embodiments disclosed below are examples of the invention that can be provided by the present application, and the scope of the present application is not limited by the following content.

### Nucleic acid construct comprising the novel promoter of the present application and nucleic acid construct 1

Hereinafter, exemplary embodiments of a nucleic acid construct comprising a promoter according to one embodiment of the present application are disclosed. Some or all of the exemplary embodiments disclosed in this section encompass exemplary embodiments of nucleic acid construct 2 and exemplary embodiments of nucleic acid construct 3, described below.

A01. A nucleic acid construct comprising:
a promoter comprising any one of the sequences of SEQ ID NOs: 01 to 08 and sequences having at least 80% sequence identity thereto.

A02. The nucleic acid construct according to A01, wherein the promoter comprises any one of the sequences of SEQ ID NO: 02 and a sequence having at least 90% sequence identity thereto.

A03. The nucleic acid construct according to any one of A01 to A02, wherein the promoter has a length of 100 bp to 1000 bp.

A04. The nucleic acid construct according to any one of A01 to A03, wherein the nucleic acid construct further comprises a transgene.

A05. The nucleic acid construct according to A04, wherein the transgene is a nucleic acid encoding a FIX protein.

A06. The nucleic acid construct according to any one of A04 to A05, wherein the promoter is operably linked to the 5' terminus of the transgene.

A07. The nucleic acid construct according to any one of A01 to A06, wherein the promoter is a hepatocyte-specific promoter.

A08. The nucleic acid construct according to any one of A01 to A07, wherein the nucleic acid construct has a length of 10000 bp or less.

A09. The nucleic acid construct according to any one of A01 to A08, wherein the nucleic acid construct further comprises any one or more selected from an upstream homology arm, a downstream homology arm, a splicing acceptor, and a Poly A sequence.

A10. A vector comprising the nucleic acid construct according to any one of A01 to A09.

A11. The vector according to A10, wherein the vector is an AAV vector.

A12. A composition for expressing transgene, comprising the nucleic acid construct according to any one of A01 to A09 or the vector according to any one of A10 to A11.

A13. A method of expressing a transgene in a cell, comprising introducing the nucleic acid construct according to any one of A01 to A09 into the cell.

A14. The method of expressing a transgene in a cell according to A13, wherein the nucleic acid construct is introduced into a cell through one or more vectors.

A15. The method of expressing a transgene in a cell according to any one of A13 to A14, wherein the vector is an AAV vector.

A16. The method of expressing a transgene in a cell according to A13, wherein the nucleic acid construct is introduced into a cell by a non-vector-based introduction method.

### Nucleic acid construct 2

Hereinafter, exemplary embodiments of a nucleic acid construct comprising a promoter according to one embodiment of the present application are disclosed. Exemplary embodiments of a nucleic acid construct disclosed in this section relate to the section "Use (2) of the promoter of the present application". In other words, exemplary embodiments of a nucleic acid construct disclosed in this section may be used alone or in conjunction with a gene editing system (for example, a CRISPR/Cas gene editing system).

B01. A nucleic acid construct comprising:
a promoter comprising any one of the sequences of SEQ ID NOs: 01 to 08 and sequences having at least 80% sequence identity thereto;
a transgene;
an upstream homology arm designed to insert the promoter and the transgene into a target site within the genome of a cell; and
a downstream homology arm designed to insert the promoter and the transgene into a target site within the genome of a cell,
wherein the promoter is operably linked to the 5' terminus of the transgene.

B02. The nucleic acid construct according to B01, wherein the promoter has a length of 100 bp to 1000 bp.

B03. The nucleic acid construct according to any one of B01 to B02, wherein the transgene is a nucleic acid encoding a FIX protein.

B04. The nucleic acid construct according to any one of B01 to B03, wherein on the nucleic acid construct, the promoter, the transgene, the upstream homology arm, and the downstream homology arm are arranged in a 5' to 3' direction in the order of the upstream homology arm, the promoter, the transgene, and the downstream homology arm.

B05. The nucleic acid construct according to any one of B01 to B04, wherein the nucleic acid construct comprises the following structure:
[upstream homology arm]-[promoter]-[transgene]-[downstream homology arm].

B06. The nucleic acid construct according to any one of B01 to B05, wherein each of the upstream homology arm and the downstream homology arm has a length of 300 bp to 1500 bp.

B07. The nucleic acid construct according to any one of B01 to B06, wherein the target site is located within a safe harbor gene on the genome.

B08. The nucleic acid construct according to B07, wherein the safe harbor gene is selected from ALB gene, FTL gene, FTH1 gene, ACTB gene, HP gene, APOC3 gene, SOD2 gene, ORM1 gene, AAVS1 gene, Rosa gene, HPRT gene, and CCR5 gene.

B09. The nucleic acid construct according to any one of B01 to B09, wherein the upstream homology arm has a sequence identical to the sequence of a region located upstream of the target site on the genome, and the downstream homology arm has a sequence identical to the sequence of a region located downstream of the target site on the genome.

B10. The nucleic acid construct according to any one of B01 to B09, wherein the nucleic acid construct has a length of 10000 bp or less.

B11. A method for editing the genome of a cell, comprising:
introducing a Cas protein or a nucleic acid encoding the same, a guide RNA or a nucleic acid encoding the same, and the nucleic acid construct according to any one of B01 to B10 or a nucleic acid encoding the same into the cell,
wherein the guide RNA comprises a guide domain having a guide sequence, and wherein the guide sequence consists of a sequence having complementarity to a sequence in the target region on the genome where a target site is present.

B12. The method for editing the genome of a cell according to B11, wherein a promoter comprising any one of the sequences of SEQ ID NOs: 01 to 08 and sequences having at least 80% sequence identity thereto and a transgene are inserted into a target region on the genome of a cell through an HDR-mediated DNA repair process with the upstream homology arm and the downstream homology arm designed to insert the promoter and the transgene into a target site within the genome of a cell.

B13. The method for editing the genome of a cell according to any one of B11 to B12, wherein the guide sequence has a length of 18 nt to 22 nt.

B14. The method for editing the genome of a cell according to any one of B11 to B13, wherein the Cas protein or a nucleic acid encoding the same, the guide RNA or a nucleic acid encoding the same, and the nucleic acid construct or a nucleic acid encoding the same are introduced into a cell through one or more vectors.

B15. The method for editing the genome of a cell according to any one of B11 to B14, wherein the vector is an AAV vector.

B16. The method for editing the genome of a cell according to any one of B11 to B13, wherein the Cas protein or a nucleic acid encoding the same, the guide RNA or a nucleic acid encoding the same, and the nucleic acid construct or a nucleic acid encoding the same are introduced into a cell by a non-vector-based introduction method.

B17. A composition for editing the genome of a cell, comprising:
a Cas protein or a nucleic acid encoding the same, a guide RNA or a nucleic acid encoding the same, and the nucleic acid construct according to any one of B01 to B10 or a nucleic acid encoding the same,
wherein the guide RNA comprises a guide domain having a guide sequence, and wherein the guide sequence consists of a sequence having complementarity to a sequence in the target region on the genome where a target site is present.

B18. The composition for editing the genome of a cell according to B17, wherein the guide sequence has a length of 18 nt to 22 nt.

### Nucleic acid construct 3

Hereinafter, exemplary embodiments of a nucleic acid construct comprising a promoter according to one embodiment of the present application are disclosed. Exemplary embodiments of a nucleic acid construct disclosed in this section relate to the section "Use (3) of the promoter of the present application". In other words, exemplary embodiments of a nucleic acid construct disclosed in this section may be used alone or in conjunction with a gene editing system (for example, a CRISPR/Cas gene editing system).

C01. A nucleic acid construct comprising:
an upstream homology arm comprising a promoter comprising any one of the sequences of SEQ ID NOs: 01 to 08 and sequences having at least 80% sequence identity thereto;
a transgene; and
a downstream homology arm designed to insert the transgene into a target site on the genome of a cell, wherein the downstream homology arm comprises a first region, and wherein the first region has a sequence that is at least 90% identical to the sequence of a portion of intron 1 of the APOC3 gene, and
wherein the promoter is operably linked to the 5' terminus of the transgene.

C02. The nucleic acid construct according to C01, wherein the promoter has a length of 100 bp to 1000 bp.

C03. The nucleic acid construct according to any one of C01 to C02, wherein the transgene is a nucleic acid encoding a FIX protein.

C04. The nucleic acid construct according to any one of C01 to C03, wherein
the upstream homology arm comprises a promoter comprising any one of the sequences of SEQ ID NOs: 01 to 06 and sequences having at least 90% sequence identity thereto, and
wherein the downstream homology arm comprises any one of the sequences of SEQ ID NOs: 07 to 08 and sequences having at least 90% sequence identity thereto.

C05. The nucleic acid construct according to any one of C01 to C04, wherein on the nucleic acid construct, the upstream homology arm, the transgene, and the downstream homology arm are arranged in a 5' to 3' direction in the order of the upstream homology arm, the transgene, and the downstream homology arm.

C06. The nucleic acid construct according to any one of C01 to C05, wherein the nucleic acid construct comprises the following structure:
[upstream homology arm]-[transgene]-[downstream homology arm].

C07. The nucleic acid construct according to any one of C01 to C06, wherein the first region has a sequence identical to the sequence of a portion of intron 1 of the APOC3 gene and has a sequence different from the sequence of the promoter.

C08. The nucleic acid construct according to any one of C01 to C07, wherein the target site is located within the APOC3 gene.

C09. The nucleic acid construct according to any one of C01 to C08, wherein the target site is located within intron 1 of the APOC3 gene.

C10. The nucleic acid construct according to any one of C01 to C09, wherein the upstream homology arm has a sequence identical to the sequence of a region located upstream of the target site on the genome, and the downstream homology arm has a sequence identical to the sequence of a region located downstream of the target site on the genome.

C11. The nucleic acid construct according to any one of C01 to C10, wherein the nucleic acid construct has a length of 10000 bp or less.

C12. A method for editing the genome of a cell, comprising:
introducing a Cas protein or a nucleic acid encoding the same, a guide RNA or a nucleic acid encoding the same, and the nucleic acid construct according to any one of C01 to C11 or a nucleic acid encoding the same into the cell,
wherein the guide RNA comprises a guide domain having a guide sequence, and wherein the guide sequence consists of a sequence having complementarity to a sequence in the target region on the genome where a target site is present.

C13. The method for editing the genome of a cell according to C12, wherein a transgene is inserted into a target region on the genome of a cell through an HDR-mediated DNA repair process with the upstream homology arm and the downstream homology arm designed to insert the transgene into a target site within the genome of a cell.

C14. The method for editing the genome of a cell according to any one of C12 to C13, wherein the guide sequence has a length of 18 nt to 22 nt.

C15. The method for editing the genome of a cell according to any one of C12 to C14, wherein the Cas protein or a nucleic acid encoding the same, the guide RNA or a nucleic acid encoding the same, and the nucleic acid construct or a nucleic acid encoding the same are introduced into a cell through one or more vectors.

C16. The method for editing the genome of a cell according to any one of C12 to C15, wherein the vector is an AAV vector.

C17. The method for editing the genome of a cell according to any one of C12 to C14, wherein the Cas protein or a nucleic acid encoding the same, the guide RNA or a nucleic acid encoding the same, and the nucleic acid construct or a nucleic acid encoding the same are introduced into a cell by a non-vector-based introduction method.

C18. A composition for editing the genome of a cell, comprising:
a Cas protein or a nucleic acid encoding the same, a guide RNA or a nucleic acid encoding the same, and the nucleic acid construct according to any one of C01 to C11 or a nucleic acid encoding the same,
wherein the guide RNA comprises a guide domain having a guide sequence, and wherein the guide sequence consists of a sequence having complementarity to a sequence in the target region on the genome where a target site is present.

C19. The composition for editing the genome of a cell according to C18, wherein the guide sequence has a length of 18 nt to 22 nt.

### Mode for Carrying out the Invention

Hereinafter, the invention provided by the present application will be described in more detail through experimental examples or examples. These experimental examples are solely for illustrating the content disclosed by the present application, and it will be apparent to those of ordinary skill in the art that the scope of the content disclosed by the present specification is not to be construed as limited by these experimental examples.

### Experimental Example

### Experimental Example 1. Confirmation of function of sequence derived from human APOC3 gene as promoter

### Experimental Example 1-1. Construction of plasmid comprising sequence derived from hAPOC3 gene and transgene

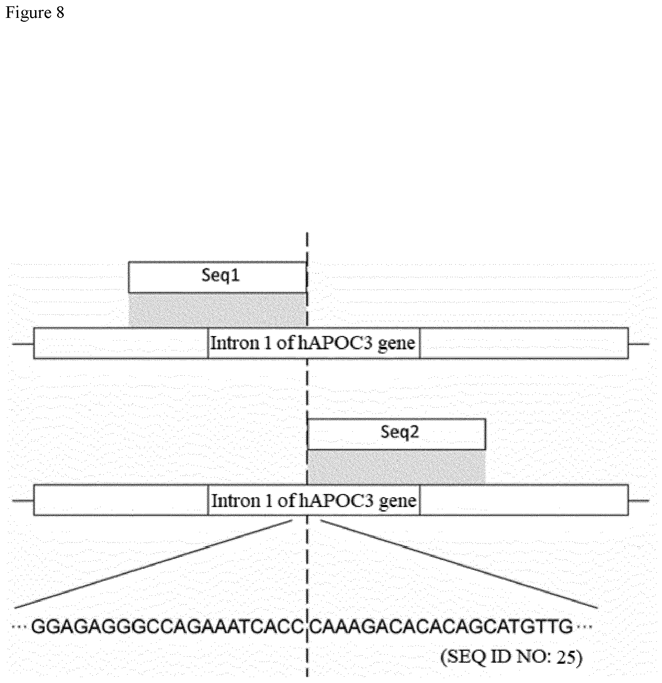

UHA (seq 1) and DHA (seq 2) were extracted based on the specific position of intron 1 of the human APOC3 gene. In other words, seq1 comprises a sequence having sequence identity to the sequence of a portion of intron 1 of the hAPOC3 gene and a sequence of the region linked to the 5' terminus of intron 1 of the hAPOC3 gene. seq2 comprises a sequence having sequence identity to the sequence of a portion of intron 1 of the hAPOC3 gene (at this time, a different sequence from the sequence of seq1) and a sequence of the region linked to the 3' terminus of intron 1 of the hAPOC3 gene. In order to aid the understanding of those of ordinary skill in the art, the region on the genome corresponding to seq1 and the region on the genome corresponding to seq2 are shown in Figure 08. The sequence of SEQ ID NO: 25 disclosed in Figure 08 discloses the sequence of a portion of intron 1 of the hAPOC3 gene. seq1 comprises the sequence of a portion of intron 1 of the hAPOC3 gene, and thus it is referred to as a sequence derived from the hAPOC3 gene.

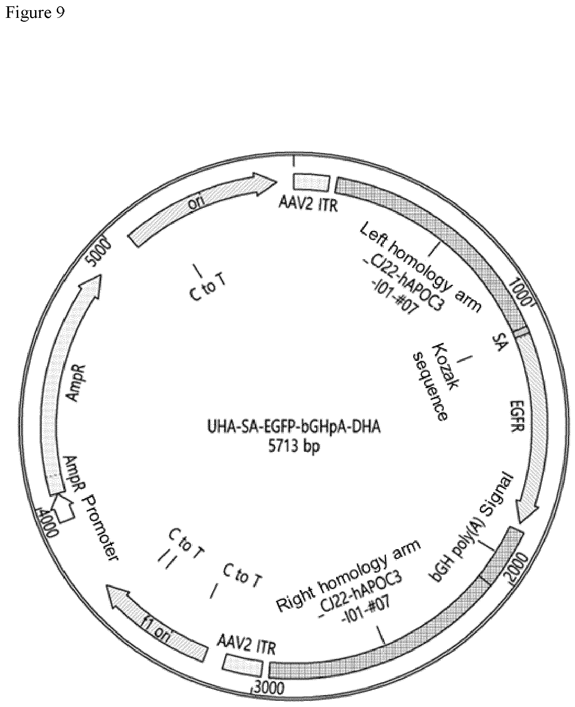

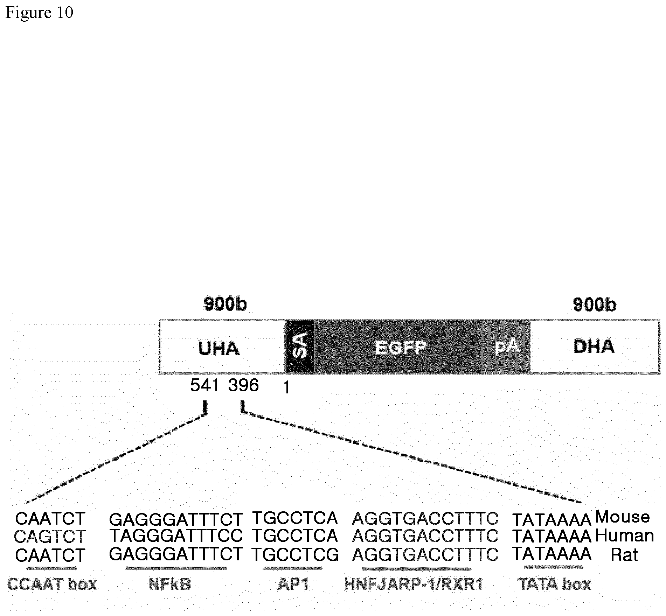

The AAV-ITR plasmid (plasmid 1, see Figure 09) comprising the nucleic acid of UHA (seq1)-SA-EGFP-bGHpA-DHA (seq2) was constructed by linking seq1 upstream and linking seq2 downstream of the DNA sequence of SA-EGFP-bGHpA (in a 5' to 3' direction) comprising a transgene encoding the EGFP protein. The constructed AAV-ITR plasmid was produced by cloning method using Gibson assembly. An exemplary structure of the nucleic acid seq1-SA-EGFP-bGHpA-seq2 is disclosed in Figure 10. As shown in Figure 10, seq1 comprises various transcription binding sites such as a CCAAT box, NFkB, AP1, a HNFJARP-1/RXR1, and a TATA box.

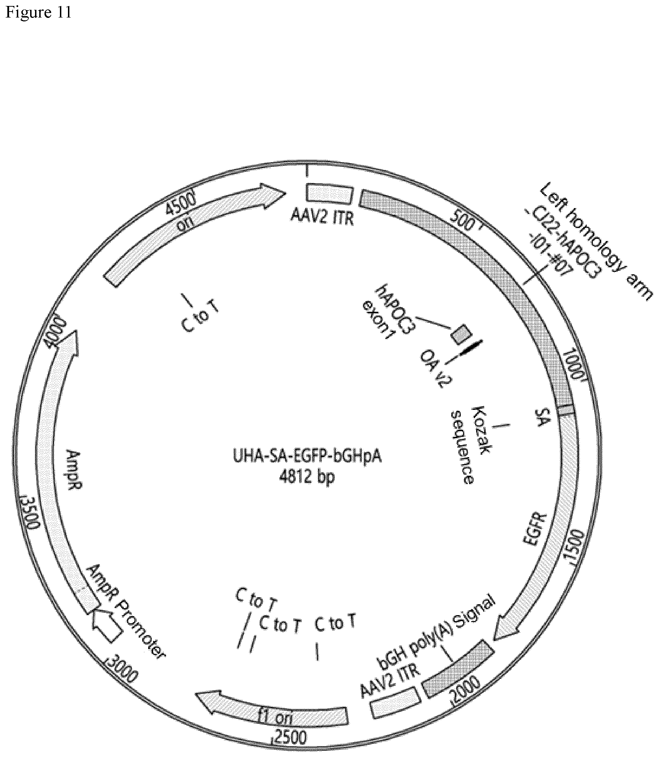

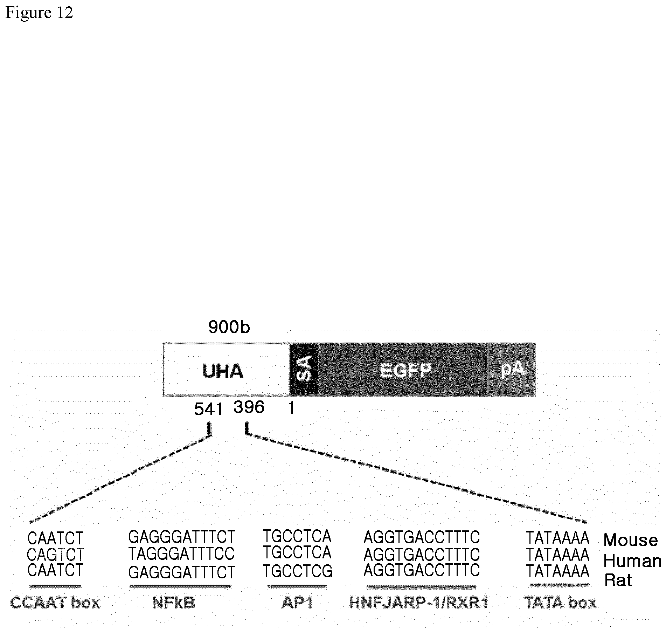

In addition, the AAV-ITR plasmid (plasmid 2, see Figure 11) comprising seq1-SA-EGFP-bGHpA was constructed in a similar manner to the above. An exemplary structure of the nucleic acid seq1-SA-EGFP-bGHA is disclosed in Figure 12.

Hereinafter, the sequences of the nucleic acid seq1-SA-EGFP-bGHpA-seq2, the nucleic acid seq1-SA-EGFP-bGHpA, and elements included in the nucleic acids are disclosed.

### SA (splicing acceptor)

CTGACCTCTTCTCTTCCTCCCACAG (SEQ ID NO: 26)
bGHpA (pA) 
CTGTGCCTTCTAGTTGCCAGCCATCTGTTGTTTGCCCCTCCCCCGTGCCTTC
CTTGACCCTGGAAGGTGCCACTCCCACTGTCCTTTCCTAATAAAATGAGGAAATTG
CATCGCATTGTCTGAGTAGGTGTCATTCTATTCTGGGGGGTGGGGTGGGGCAGGAC
AGCAAGGGGGAGGATTGGGAAGACAATAGCAGGCATGCTGGGGATGCGGTGGGC
TCTATGG (SEQ ID NO: 27)
Sequence of EGFP gene encoding GFP protein 
ATGGTGAGCAAGGGCGAGGAGCTGTTCACCGGGGTGGTGCCCATCCTGGT
CGAGCTGGACGGCGACGTAAACGGCCACAAGTTCAGCGTGTCCGGCGAGGGCGA
GGGCGATGCCACCTACGGCAAGCTGACCCTGAAGTTCATCTGCACCACCGGCAAG
CTGCCCGTGCCCTGGCCCACCCTCGTGACCACCCTGACCTACGGCGTGCAGTGCT
TCAGCCGCTACCCCGACCACATGAAGCAGCACGACTTCTTCAAGTCCGCCATGCC
CGAAGGCTACGTCCAGGAGCGCACCATCTTCTTCAAGGACGACGGCAACTACAA
GACCCGCGCCGAGGTGAAGTTCGAGGGCGACACCCTGGTGAACCGCATCGAGCT
GAAGGGCATCGACTTCAAGGAGGACGGCAACATCCTGGGGCACAAGCTGGAGTA
CAACTACAACAGCCACAACGTCTATATCATGGCCGACAAGCAGAAGAACGGCATC
AAGGTGAACTTCAAGATCCGCCACAACATCGAGGACGGCAGCGTGCAGCTCGCC
GACCACTACCAGCAGAACACCCCCATCGGCGACGGCCCCGTGCTGCTGCCCGACA
ACCACTACCTGAGCACCCAGTCCGCCCTGAGCAAAGACCCCAACGAGAAGCGCG
ATCACATGGTCCTGCTGGAGTTCGTGACCGCCGCCGGGATCACTCTCGGCATGGA
CGAGCTGTACAAGTCCGGATAA (SEQ ID NO: 28)
Seq1 
TGAGAGCTCAGCCCTGTAACCAGGCCTTGCCGGAGCCACTGATGCCTGGT
CTTCTGTGCCTTTACTCCAAACACCCCCCAGCCCAAGCCACCCACTTGTTCTCAAG
TCTGAAGAAGCCCCTCACCCCTCTACTCCAGGCTGTGTTCAGGGCTTGGGGCTGG
TGGAGGGAGGGGCCTGAAATTCCAGTGTGAAAGGCTGAGATGGGCCCGAGGCCC
 
CTGGCCTATGTCCAAGCCATTTCCCCTCTCACCAGCCTCTCCCTGGGGAGCCAGTC
AGCTAGGAAGGAATGAGGGCTCCCCAGGCCCACCCCCAGTTCCTGAGCTCATCTG
GGCTGCAGGGCTGGCGGGACAGCAGCGTGGACTCAGTCTCCTAGGGATTTCCCAA
CTCTCCCGCCCGCTTGCTGCATCTGGACACCCTGCCTCAGGCCCTCATCTCCACTG
GTCAGCAGGTGACCTTTGCCCAGCGCCCTGGGTCCTCAGTGCCTGCTGCCCTGGA
GATGATATAAAACAGGTCAGAACCCTCCTGCCTGTCTGCTCAGTTCATCCCTAGAG
GCAGCTGCTCCAGGTAATGCCCTCTGGGGAGGGGAAAGAGGAGGGGAGGAGGAT
GAAGAGGGGCAAGAGGAGCTCCCTGCCCAGCCCAGCCAGCAAGCCTGGAGAAG
CACTTGCTAGAGCTAAGGAAGCCTCGGAGCTGGACGGGTGCCCCCCACCCCTCAT
CATAACCTGAAGAACATGGAGGCCCGGGAGGGGTGTCACTTGCCCAAAGCTACA
CAGGGGGTGGGGCTGGAAGTGGCTCCAAGTGCAGGTTCCCCCCTCATTCTTCAGG
CTTAGGGCTGGAGGAAGCCTTAGACAGCCCAGTCCTACCCCAGACAGGGAAACT
GAGGCCTGGAGAGGGCCAGAAATCACC (SEQ ID NO: 01)
Seq2 
CAAAGACACACAGCATGTTGGCTGGACTGGACGGAGATCAGTCCAGACC
GCAGGTGCCTTGATGTTCAGTCTGGTGGGTTTTCTGCTCCATCCCACCCACCTCCC
TTTGGGCCTCGATCCCTCGCCCCTCACCAGTCCCCCTTCTGAGAGCCCGTATTAGC
AGGGAGCCGGCCCCTACTCCTTCTGGCAGACCCAGCTAAGGTTCTACCTTAGGGG
CCACGCCACCTCCCCAGGGAGGGGTCCAGAGGCATGGGGACCTGGGGTGCCCCT
CACAGGACACTTCCTTGCAGGAACAGAGGTGCCATGCAGCCCCGGGTACTCCTTG
TTGTTGCCCTCCTGGCGCTCCTGGCCTCTGCCCGTAAGCACTTGGTGGGACTGGG
CTGGGGGCAGGGTGGAGGCAACTTGGGGATCCCAGTCCCAATGGGTGGTCAAGC
AGGAGCCCAGGGCTCGTCCAGAGGCCGATCCACCCCACTCAGCCCTGCTCTTTCC
TCAGGAGCTTCAGAGGCCGAGGATGCCTCCCTTCTCAGCTTCATGCAGGGTTACAT
GAAGCACGCCACCAAGACCGCCAAGGATGCACTGAGCAGCGTGCAGGAGTCCCA
GGTGGCCCAGCAGGCCAGGTACACCCGCTGGCCTCCCTCCCCATCCCCCCTGCCA
GCTGCCTCCATTCCCACCCGCCCCTGCCCTGGTGAGATCCCAACAATGGAATGGA
GGTGCTCCAGCCTCCCCTGGGCCTGTGCCTCTTCAGCCTCCTCTTTCCTCACAGGG
CCTTTGTCAGGCTGCTGCGGGAGAGATGACAGAGTTGAGACTGCATTCCTCCCAG
GTCCCTCCTTTCTCCCCGGAGCAGTCCTAGGGCGTGCCGTTTTAGCCCTCATTTCC
ATTTTCCTTTCCTTTCCCTTTCTT (SEQ ID NO: 07)
seq1-SA-EGFP-bGHpA-seq2 
CGTATTAGCAGGGAGCCGGCCCCTACTCCTTCTGGCAGACCCAGCTAAGG
TTCTACCTTAGGGGCCACGCCACCTCCCCAGGGAGGGGTCCAGAGGCATGGGGAC
CTGGGGTGCCCCTCACAGGACACTTCCTTGCAGGAACAGAGGTGCCATGCAGCCC
CGGGTACTCCTTGTTGTTGCCCTCCTGGCGCTCCTGGCCTCTGCCCGTAAGCACTT
GGTGGGACTGGGCTGGGGGCAGGGTGGAGGCAACTTGGGGATCCCAGTCCCAAT
 
GGGTGGTCAAGCAGGAGCCCAGGGCTCGTCCAGAGGCCGATCCACCCCACTCAG
CCCTGCTCTTTCCTCAGGAGCTTCAGAGGCCGAGGATGCCTCCCTTCTCAGCTTCA
TGCAGGGTTACATGAAGCACGCCACCAAGACCGCCAAGGATGCACTGAGCAGCG
TGCAGGAGTCCCAGGTGGCCCAGCAGGCCAGGTACACCCGCTGGCCTCCCTCCCC
ATCCCCCCTGCCAGCTGCCTCCATTCCCACCCGCCCCTGCCCTGGTGAGATCCCAA
CAATGGAATGGAGGTGCTCCAGCCTCCCCTGGGCCTGTGCCTCTTCAGCCTCCTCT
TTCCTCACAGGGCCTTTGTCAGGCTGCTGCGGGAGAGATGACAGAGTTGAGACTG
CATTCCTCCCAGGTCCCTCCTTTCTCCCCGGAGCAGTCCTAGGGCGTGCCGTTTTA
GCCCTCATTTCCATTTTCCTTTCCTTTCCCTTTCTT (SEQ ID NO: 29)
seq1-SA-EGFP-bGHpA

CGAGGGCGAGGGCGATGCCACCTACGGCAAGCTGACCCTGAAGTTCATCT
GCACCACCGGCAAGCTGCCCGTGCCCTGGCCCACCCTCGTGACCACCCTGACCTA
CGGCGTGCAGTGCTTCAGCCGCTACCCCGACCACATGAAGCAGCACGACTTCTTC
AAGTCCGCCATGCCCGAAGGCTACGTCCAGGAGCGCACCATCTTCTTCAAGGACG
ACGGCAACTACAAGACCCGCGCCGAGGTGAAGTTCGAGGGCGACACCCTGGTGA
ACCGCATCGAGCTGAAGGGCATCGACTTCAAGGAGGACGGCAACATCCTGGGGC
ACAAGCTGGAGTACAACTACAACAGCCACAACGTCTATATCATGGCCGACAAGCA
GAAGAACGGCATCAAGGTGAACTTCAAGATCCGCCACAACATCGAGGACGGCAG
CGTGCAGCTCGCCGACCACTACCAGCAGAACACCCCCATCGGCGACGGCCCCGTG
CTGCTGCCCGACAACCACTACCTGAGCACCCAGTCCGCCCTGAGCAAAGACCCCA
ACGAGAAGCGCGATCACATGGTCCTGCTGGAGTTCGTGACCGCCGCCGGGATCAC
TCTCGGCATGGACGAGCTGTACAAGTCCGGATAACTCAGATCTCGAgCTGTGCCTT
CTAGttgccagccatctgttgtttgcccctcccccgtgccttccttgaccctggaaggtgccactcccactgtcctttcctaataaaatg
aggaaattgcatcgcattgtctgagtaggtgtcattctattctggggggtggggtggggcaggacagcaagggggaggattgggaag
acaatagcaggcatgctggggatgcgGTGGGCTCTATGG (SEQ ID NO: 30)

### Experimental Example 1-2. Production of AAV vector comprising seq1-SA-EGFP-bGHpA-seq2 and AAV vector comprising seq1-SA-EGFP-bGHpA

10 µg of plasmid 1 (pAAV-UHA-SA-EGFP-bGHpA-DHA) or 2 (pAAV-UHA-SA-EGFP-bGHpA) constructed in Experimental Example 1-1, 20 µg of helper plasmid, 10 µg of Rep/Cap plasmid and LipoGene (100 µL) were mixed, and then plasmid 1 or 2, helper plasmid, and Rep/Cap plasmid were co-transfected into HEK293. At this time, the helper plasmid is a plasmid that encodes genes (E4, E2a, VA) required for AAV viral genome replication when producing an AAV vector. The Rep/Cap plasmid is a plasmid that encodes genes (Rep78/Rep68/Rep52/Rep40) required for replication and packaging of the AAV viral genome and genes (VP1/VP2/VP3) required for viral capsid formation when producing an AAV vector. After 3 days, virus particles were obtained. Through iodixanol gradient purification, an AAV vector comprising the nucleic acid seq1-SA-EGFP-bGHA-seq2 and an AAV vector comprising the nucleic acid seq1-SA-EGFP-bGHA were finally obtained, respectively. Thereafter, the titer was quantified through qPCR on the ITR genome. Quant Studio3 Real-Time PCR Instrument (ThermoFisher) was used as a qPCR equipment.

Specifically, the quantification of titer was performed as follows:

### (1) Dnase1 treatment

After the AAV vector was obtained, it was treated with DNase1 to remove poorly formed AAV.

The produced AAV (2 µL from a 1/20 diluted sample of the final produced AAV), Nuclease Free Water (NFW) (15.6 µL), 10X DNase1 buffer (2 µL), and DNase1 (70 U/µL) (0.4 µL) were mixed and incubated at 37 °C for 30 minutes.

### (2) Preparation of standard sample

2E+9 GC(genome copy)/µL was diluted with NFW by 1/10 to prepare samples at concentrations of 2E+8 GC/µL, 2E+7 GC/µL, 2E+6 GC/µL, 2E+5 GC/µL, 2E+4 GC/µL, and 2E+3 GC/µL.

(3) SYBR Green Master mix (Thermo, Cat# 4344463) (10 µL), 50X ROX (0.4 µL), F/R ITR primer (50 µM) (0.2 µL), and Nuclease Free Water (4.4 µL) were first made as a master mix as needed, and samples were dispensed at 5 µL each.

Information on the sequences of the primers used is as follows:
Forward ITR primer: 5'-GGAACCCCTAGTGATGGAGTT-3' (SEQ ID NO: 31)
Reverse ITR primer: 5'-CGGCCTCAGTGAGCGA-3' (SEQ ID NO: 32)

### (4) Performance of qPCR

qPCR was performed under the following qPCR conditions: pre-denaturation at 95 °C (10 min), [95 °C (15 sec), 60 °C (1 min)] X 40 cycles.

### Experimental Example 1-3. Introduction of each of nucleic acid seq1-SA-EGFP-bGHpA-seq2 and nucleic acid seq1-SA-EGFP-bGHpA into hepatocyte, and confirmation of GFP expression in hepatocyte

The AAV vector comprising the nucleic acid seq1-SA-EGFP-bGHpA-seq2 prepared in Experimental Example 1-2 was transfected into HepG2 cells. In addition, the AAV vector comprising the nucleic acid seq1-SA-EGFP-bGHpA prepared in Experimental Example 1-2 was transfected into HepG2 cells. Specifically, the well was treated with the cells in a 24 well plate, 5E+4 cells/tip, 3 tips/well (total 1.5E+5 cells per well (=1.5E+5 cells/well)). They were treated with the AAV vector by converting the amount of DNA (ng) corresponding to 6.02E+10 AAV copy number. 5E+4 cells were treated with 353 ng of the AAV vector comprising the nucleic acid seq1-SA-EGFP-bGHpA-seq2. 5E+4 cells were treated with 301.1 ng of the AAV vector comprising the nucleic acid seq1-SA-EGFP-bGHpA. Transfection was performed using the electroporation-type NEON equipment. Transfection was performed under the conditions of 1350 V (voltage)/20 ms (width)/2 pulses (pulse).

After 3 days, cell pellets were obtained. The pellet was resuspended in 200 µL of FACS buffer (1X PBS supplemented with 2% FBS). Thereafter, GFP-positive cells were distinguished through primary gating of FSC/SSC and additional gating on a GFP fluorescence filter using FACS equipment (Attune Life technologies). The percentage of GFP-expressing cells for a total of 20,000 cells was calculated.

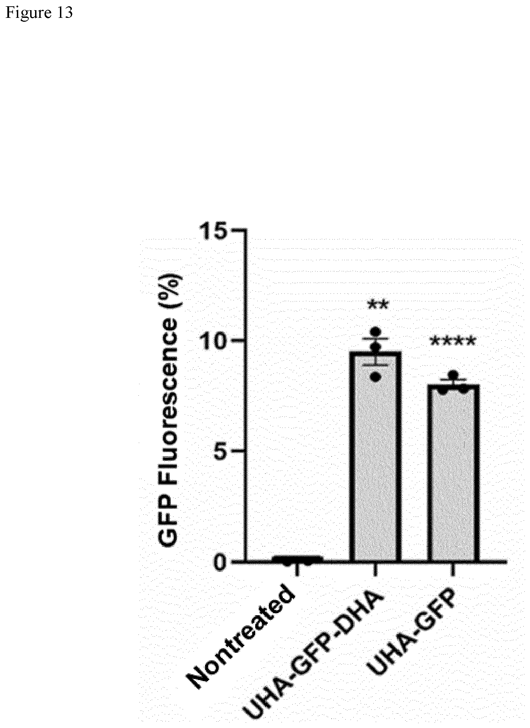

The results obtained by confirming GFP expression in HepG2 cells are shown in Figure 13. As shown in Figure 13, GFP fluorescence was confirmed in HepG2 (UHA-GFP-DHA) into which the nucleic acid seq1-SA-EGFP-bGHpA-seq2 was introduced and HepG2 (UHA-GFP) into which the nucleic acid seq1-SA-EGFP-bGHpA was introduced. In other words, it was confirmed that GFP was expressed in HepG2 into which the nucleic acid was introduced, regardless of the presence of a gene editing tool. This is interpreted to mean that GFP was transcribed and/or expressed without using the endogenous promoter present in hepatocytes, and that part or all of the regions of seq1 and/or seq2 have promoter activity for transcription of EGFP.

### Experimental Example 2. Optimization of human APOC3 intron 1 derived promoter

### Experimental Example 2-1. Confirmation of promoter function of seq1

In order to confirm the promoter function of seq1 used in Experimental Example 1, GFP fluorescence was confirmed and compared in HepG2 (UHA-GFP-DHA) into which the nucleic acid seq1-SA-EGFP-bGHpA-seq2 was introduced and HepG2 (UHA-GFP) into which the nucleic acid seq1-SA-EGFP-bGHpA was introduced (n=3). Introduction of nucleic acid into HepG2 and confirmation of fluorescence were performed using the same method as disclosed in Experimental Example 1.

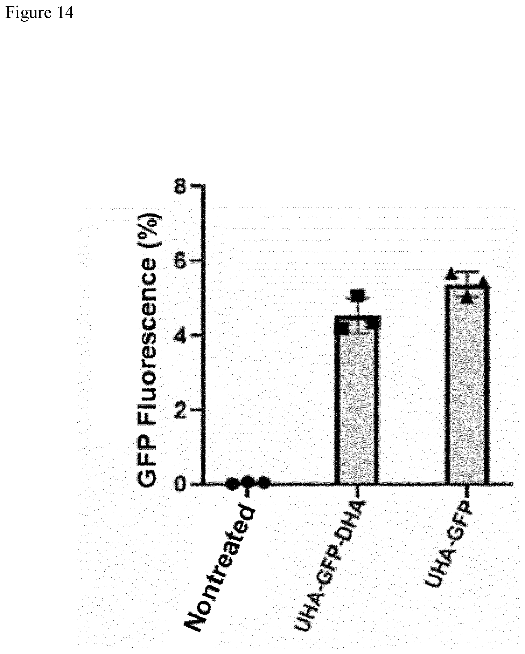

The results are shown in Figure 14. The results disclosed in Figure 14 show similar results to the results of Figure 13 described in Experimental Example 1-3. It was confirmed that there was no significant difference between the GFP level observed in HepG2 into which the vector (the nucleic acid seq1-SA-EGFP-bGHpA-seq2) was introduced, in which UHA (seq1) and DHA (seq2) were linked to the upstream and downstream of the transgene (here, EGFP), respectively, and the GFP level observed in HepG2 into which the nucleic acid seq1-SA-E
- bGHpA was introduced. Most promoter activities were found to be in part or all of seq1.

### Experimental Example 2-2. Sequence extraction to identify the region that plays a major role in promoter activity in seq1, and construction of vector

The inventors of the present application confirmed that most of the promoter activity in Experimental Example 2-1 was due to part or all of the sequence seq1. To order to compare the promoter activity of each region within the sequences seq1, seq3 and seq4 were extracted from seq1.
Seq3: Sequence of the region comprising the TATA box

CGCCCTGGGTCCTCAGTGCCTGCTGCCCTGGAGATGA TATAAAACAGGTC
AGAACCCTCCTGCCTGTCTGCTCAGTTCATCCCTAGAGGCAGCTGCTCCAGGTAAT
GCCCTCTGGGGAGGGGAAAGAGGAGGGGAGGAGGATGAAGAGGGGCAAGAGGA
GCTCCCTGCCCAGCCCAGCCAGCAAGCCTGGAGAAGCACTTGCTAGAGCTAAGG
AAGCCTCGGAGCTGGACGGGTGCCCCCCACCCCTCATCATAACCTGAAGAACATG
GAGGCCCGGGAGGGGTGTCACTTGCCCAAAGCTACACAGGGGGTGGGGCTGGAA
GTGGCTCCAAGTGCAGGTTCCCCCCTCATTCTTCAGGCTTAGGGCTGGAGGAAGC
CTTAGACAGCCCAGTCCTACCCCAGACAGGGAAACTGAGGCCTGGAGAGGGCCA
GAAATCACC (SEQ ID NO: 02)

The underlined part in seq3 indicates the TATA box.
Seq4: Sequence of the region comprising both the TATA box and the CAAT box

TCCTGAGCTCATCTGGGCTGCAGGGCTGGCGGGACAGCAGCGTGGACT C
AGTCTCCTAGGGATTTCCCAACTCTCCCGCCCGCTTGCTGCATCTGGACACCCTGC
CTCAGGCCCTCATCTCCACTGGTCAGCAGGTGACCTTTGCCCAGCGCCCTGGGTC
CTCAGTGCCTGCTGCCCTGGAGATGA TATAAAACAGGTCAGAACCCTCCTGCCTG
TCTGCTCAGTTCATCCCTAGAGGCAGCTGCTCCAGGTAATGCCCTCTGGGGAGGG
GAAAGAGGAGGGGAGGAGGATGAAGAGGGGCAAGAGGAGCTCCCTGCCCAGCC
CAGCCAGCAAGCCTGGAGAAGCACTTGCTAGAGCTAAGGAAGCCTCGGAGCTGG
ACGGGTGCCCCCCACCCCTCATCATAACCTGAAGAACATGGAGGCCCGGGAGGGG
TGTCACTTGCCCAAAGCTACACAGGGGGTGGGGCTGGAAGTGGCTCCAAGTGCA
GGTTCCCCCCTCATTCTTCAGGCTTAGGGCTGGAGGAAGCCTTAGACAGCCCAGT
CCTACCCCAGACAGGGAAACTGAGGCCTGGAGAGGGCCAGAAATCACC (SEQ ID
NO: 03).

In seq4, the italicized part indicates the CAAT box, and the underlined part indicates the TATA box.

The inventors of the present application constructed each AAV vector comprising nucleic acids 1 to 5 in order to compare the promoter activity of each sequence. Nucleic acid 1 has a structure in which seq1 and seq2 are linked upstream and downstream of the hF9 transgene, respectively. Nucleic acid 2 has a structure in which seq1 is linked upstream of the hF9 transgene. Nucleic acid 3 has a structure in which seq3 comprising the TATA box is linked upstream of the hF9 transgene. Nucleic acid 4 has a structure in which seq4 comprising both the TATA box and the CAAT box is linked upstream of the hF9 transgene. Nucleic acid 5 has a structure in which seq2 is linked downstream of the hF9 transgene. Information about nucleic acids 1 to 5 is disclosed below. At this time, hF9 refers to the sequence encoding the human F9 protein as a transgene.
(Nucleic acid 1) seq1-SA-hF9-bGHpA-seq2;
(Nucleic acid 2) seq1-SA-hF9;
(Nucleic acid 3) seq3-SA-hF9-bGHpA;
(Nucleic acid 4) seq4-SA-hF9-bGHpA;
(Nucleic acid 5) hF9-bGHpA-seq2;

The AAV vectors comprising each of nucleic acids 1 to 5 were constructed through a method similar to that disclosed in Experimental Example 1. Specifically, it was constructed using the following method: 10 µg of plasmids for each of nucleic acids 1 to 5, 20 µg of helper plasmid, 10 µg of Rep/Cap plasmid, and 100 µL of LipoGene were mixed in HEK293 cells. Thereafter, HEK293 cells were co-transfected with each of the plasmids for nucleic acids 1 to 5, a helper plasmid, and a Rep/Cap plasmid. Three days after transfection, virus particles were obtained. Through iodixanol gradient purification, five AAV vectors comprising each of nucleic acids 1 to 5 were finally obtained, respectively. Thereafter, the titer for the ITR genome was quantified using the same method as in Experimental Example 1.
Sequence of hF9 gene encoding hF9 protein 
ACACCAACATCTTTCTGAAGTTCGGCAGCGGCTACGTGTCCGGCTGGGGC
AGAGTGTTTCACAAGGGCAGATCCGCTCTGGTGCTGCAGTACCTGAGAGTGCCTC
TGGTGGACCGGGCCACCTGTCTGAGAAGCACCAAGTTCACCATCTACAACAACAT
GTTCTGCGCCGGCTTTCACGAGGGCGGCAGAGATAGCTGTCAGGGCGATTCTGGC
GGCCCTCACGTGACAGAGGTGGAAGGCACCAGCTTTCTGACCGGCATCATCAGCT
GGGGCGAGGAATGCGCCATGAAGGGGAAGTACGGCATCTACACCAAGGTGTCCA
GATACGTGAACTGGATCAAAGAAAAGACCAAGCTGACATGA (SEQ ID NO: 33)
Sequence of nucleic acid 1 (seq1-SA-hF9-bGHpA-seq2) 
CTTCATGCAGGGTTACATGAAGCACGCCACCAAGACCGCCAAGGATGCAC
TGAGCAGCGTGCAGGAGTCCCAGGTGGCCCAGCAGGCCAGGTACACCCGCTGGC
CTCCCTCCCCATCCCCCCTGCCAGCTGCCTCCATTCCCACCCGCCCCTGCCCTGGT
GAGATCCCAACAATGGAATGGAGGTGCTCCAGCCTCCCCTGGGCCTGTGCCTCTT
CAGCCTCCTCTTTCCTCACAGGGCCTTTGTCAGGCTGCTGCGGGAGAGATGACAG
AGTTGAGACTGCATTCCTCCCAGGTCCCTCCTTTCTCCCCGGAGCAGTCCTAGGGC
GTGCCGTTTTAGCCCTCATTTCCATTTTCCTTTCCTTTCCCTTTCTT (SEQ ID NO: 34)
Sequence of nucleic acid 2 (seq1-SA-hF9-bGHpA) 
GAGTGCCTCTGGTGGACCGGGCCACCTGTCTGAGAAGCACCAAGTTCACC
ATCTACAACAACATGTTCTGCGCCGGCTTTCACGAGGGCGGCAGAGATAGCTGTC
AGGGCGATTCTGGCGGCCCTCACGTGACAGAGGTGGAAGGCACCAGCTTTCTGA
 
CCGGCATCATCAGCTGGGGCGAGGAATGCGCCATGAAGGGGAAGTACGGCATCTA
CACCAAGGTGTCCAGATACGTGAACTGGATCAAAGAAAAGACCAAGCTGACATG
ACTGTGCCTTCTAGTTGCCAGCCATCTGTTGTTTGCCCCTCCCCCGTGCCTTCCTTG
ACCCTGGAAGGTGCCACTCCCACTGTCCTTTCCTAATAAAATGAGGAAATTGCATC
GCATTGTCTGAGTAGGTGTCATTCTATTCTGGGGGGTGGGGTGGGGCAGGACAGC
AAGGGGGAGGATTGGGAAGACAATAGCAGGCATGCTGGGGATGCGGTGGGCTCT
ATGG (SEQ ID NO: 35)
Sequence of nucleic acid 3 (seq3-SA-hF9-bGHpA)
   AGCAGGCATGCTGGGGATGCGGTGGGCTCTATGG (SEQ ID NO: 36)
Sequence of nucleic acid 4 (seq4-SA-hF9-bGHpA) 
CCGTGCCTTCCTTGACCCTGGAAGGTGCCACTCCCACTGTCCTTTCCTAAT
AAAATGAGGAAATTGCATCGCATTGTCTGAGTAGGTGTCATTCTATTCTGGGGGGT
GGGGTGGGGCAGGACAGCAAGGGGGAGGATTGGGAAGACAATAGCAGGCATGCT
GGGGATGCGGTGGGCTCTATGG (SEQ ID NO: 37)
Sequence of nucleic acid 5 (hF9-bGHpA-seq2)

AGCAGGAGCCCAGGGCTCGTCCAGAGGCCGATCCACCCCACTCAGCCCT
GCTCTTTCCTCAGGAGCTTCAGAGGCCGAGGATGCCTCCCTTCTCAGCTTCATGCA
GGGTTACATGAAGCACGCCACCAAGACCGCCAAGGATGCACTGAGCAGCGTGCA
GGAGTCCCAGGTGGCCCAGCAGGCCAGGTACACCCGCTGGCCTCCCTCCCCATCC
CCCCTGCCAGCTGCCTCCATTCCCACCCGCCCCTGCCCTGGTGAGATCCCAACAAT
GGAATGGAGGTGCTCCAGCCTCCCCTGGGCCTGTGCCTCTTCAGCCTCCTCTTTCC
TCACAGGGCCTTTGTCAGGCTGCTGCGGGAGAGATGACAGAGTTGAGACTGCATT
CCTCCCAGGTCCCTCCTTTCTCCCCGGAGCAGTCCTAGGGCGTGCCGTTTTAGCCC
TCATTTCCATTTTCCTTTCCTTTCCCTTTCTT (SEQ ID NO: 38)

### Experimental Example 2-3. Introduction of nucleic acids 1 to 5 into hepatocytes and confirmation of hF9 expression

Primary human hepatocytes were treated with the AAV vectors comprising each of nucleic acids 1 to 5 constructed in Experimental Example 2-2. Specifically, the primary human hepatocytes were dispensed at 5E+5 cells per well (using a 24 well plate). Thereafter, in order to treat under the condition of 5E+5 multiplicity of infection (MOI), the cells were treated with the AAV vectors comprising each of nucleic acids 1 to 5 under the condition of 2.5E+11GC (genome copy).

After 48 hours of treatment with the AAV vector, the expression level of the hF9 protein present in the cell supernatant was compared through ELISA analysis (n=3). In order to quantitatively analyze human Factor 9 (hF9) protein, the hF9 ELISA kit (abcam, 108831) was used. First, all cell supernatants were prepared by diluting them by 2/5. hF9 protein standard was prepared as follows. 120 µL was taken from hF9 (100 ng/ml, 1 ml) and diluted with 120 µL of diluent M solution to prepare each sample (240 µL) at concentrations of 50 ng/ml, 25 ng/ml, 12.5 ng/ml, 6.25 ng/ml, 3.13 ng/ml, and 1.56 ng/ml.

Thereafter, 50 µL of cell supernatant and hF9 standard were dispensed onto an ELISA plate coated with hF9 antibody and then incubated for 2 hours. Thereafter, it was washed three times using PBS (200 µL/well). Thereafter, biotin-conjugated Factor IX detector antibody was dispensed at 50 µL/well and then incubated for 1 hour. Thereafter, the cells were washed three times with PBS (200 µL/well), and streptavidin, which binds to biotin, was dispensed at 50 µL/well, and then incubated for 30 minutes. Thereafter, the cells were washed three times with PBS (200 µL/well), chromogen substrate was dispensed at 50 µL/well, and then incubated for 15 minutes in a place where light was blocked. Thereafter, the stop solution was dispensed at 50 µL/well, and after 1 minute, the absorbance was measured at a wavelength of 450 nm using a microplate reader equipment (Multiskan GO Microplate spectrophotometer, ThermoFisher).

The results obtained by analyzing hF9 by ELISA are disclosed in Figure 15. In Figure 15, the sample treated with the AAV vector related to nucleic acid 1 was denoted as sample 1, the sample treated with the AAV vector related to nucleic acid 2 was denoted as sample 2, the sample treated with the AAV vector related to nucleic acid 3 was denoted as sample 3, the sample treated with the AAV vector related to nucleic acid 4 was denoted as sample 4, and the sample treated with the AAV vector related to nucleic acid 5 was denoted as sample 5. The group denoted as non-treated represents the results for the group in which primary human hepatocytes were not treated with the AAV vector.

### Experimental Example 3. Confirmation of function of sequence derived from mouse APOC3 gene as promoter

### Experimental Example 3-1. Construction of plasmid comprising sequence derived from mAPOC3 gene and transgene

UHA (seq5) and DHA (seq6) were extracted based on the specific position of intron 1 of the mouse APOC3 gene (mAPOC3 intron 1). In other words, seq5 comprises a sequence having sequence identity to the sequence of a portion of intron 1 of the mAPOC3 gene and a sequence of the region linked to the 5' terminus of intron 1 of the mAPOC3 gene. seq6 comprises a sequence having sequence identity to the sequence of a portion of intron 1 of the mAPOC3 gene (at this time, a different sequence from the sequence of seq5) and a sequence of the region linked to the 3' terminus of intron 1 of the mAPOC3 gene.

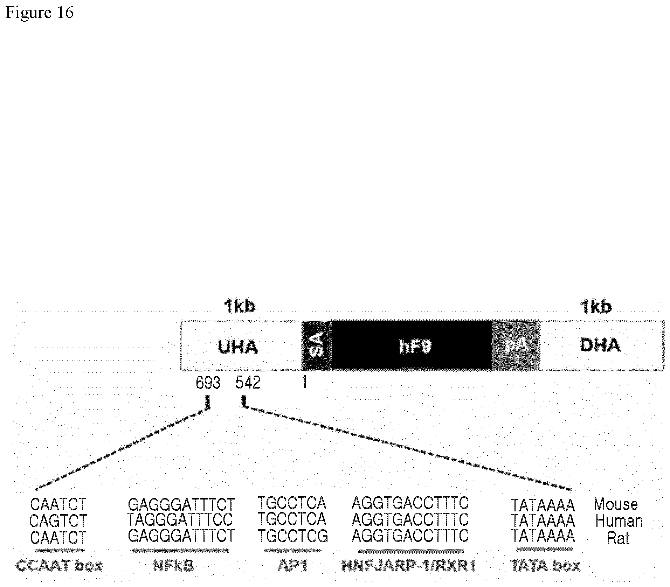

The AAV-ITR plasmid comprising the nucleic acid of seq5-SA-hF9-pA-seq6 was constructed by linking seq5 upstream and linking seq6 downstream of the DNA sequence of SA-hF9-pA comprising a transgene encoding the hF9 protein. The AAV-ITR plasmid was produced by cloning method using Gibson assembly. An exemplary structure of the nucleic acid seq5-SA-hF9-pA-seq6 is disclosed in Figure 16. As shown in Figure 16, seq5 comprises various transcription binding sites such as a CCAAT box, NFkB, AP1, a HNFJARP-1/RXR1, and a TATA box.

Hereinafter, the sequences of the nucleic acid seq5-SA-hF9-pA-seq6 and elements included in the nucleic acid are disclosed.

### SA (splicing acceptor)

CTGACCTCTTCTCTTCCTCCCACAG (SEQ ID NO: 26)
pA (poly A) 
CTGTGCCTTCTAGTTGCCAGCCATCTGTTGTTTGCCCCTCCCCCGTGCCTTC
CTTGACCCTGGAAGGTGCCACTCCCACTGTCCTTTCCTAATAAAATGAGGAAATTG
CATCGCATTGTCTGAGTAGGTGTCATTCTATTCTGGGGGGTGGGGTGGGGCAGGAC
AGCAAGGGGGAGGATTGGGAAGACAATAGCAGGCATGCTGGGGATGCGGTGGGC
TCTATGG (SEQ ID NO: 27)
Seq5 
TGCCCAGCTACGGCAGAGGAGACTGTTGGGATCACAGGTGGAGGTCAGT
GTGGGGGCATGTAACCTTCACTCTGCCCCCTCCCACCCCACTTCCTCCGTTGCTCC
ACAGTGGCACCACAGATTCCCCATTCCATTCTACTGGAAGGCTTTCAGGTGGCTTC
TGTGAAGGAGCCTGTCACTGGCTCTAGGCTGTAAAGCAGGCCTGAGGTCCAAGG
CTTGTCCCCTGTCTTTTCCTGTCTCACCGACCTCATCTAGGTGAGCCGGTAGCTGA
TCCCTTGGTGGCGGAGGTGGCCGTGAAAAGCATGGGCAATCTCAGAGGGATTTCT
CAACTCCTCTGGCAGATGGCTGCATGGCCTTCCTGCCTCAGGCTCTGGTCTGGACT
GCTCAGCAGGTGACCTTTGCCCAGCTCACTGGGCCTTCTGTGCCCGCTGTCCCATC
CTGGAGCCAATATAAAACAGGTCACAGCCCTCCCCGCTTGCCTGCTCAGTTTTATC
CCTAGAAGCAGCTAGCTACTCCAGGTAATGCCCCTGGGGAGGAGAGGAAGGAAG
GGAAGAAACAAAGAGCTGGAGGGAGAAGCTCTCACCACCCAGCCATCTAGCCCA
CAGAAGGCTTGGGACTCATGGTCTCCACCCTTGGGTTCCTGGTGTGGGGGGGGCT
AGAAGCTGGGGAAAGGGAGGCTATGGGCCAAAAGCCTTGCACTGGGTAGTGGCA
GAGCTGCCTGCAATTGGGTCATGCTGTCCCTTGTTTAGCTTGCTCTGAGCTATTAG
AAGCCTTTGGTATCCATGCCTACCCAGGACTGAAAAAACTGAGTCCTAGCGAGGA
AAAGAAACCTATTGTTTCTCAGCATATTAGAGTAGATGTCCCGTTCTGCAGAGTATT
TCTATACTCCACCTTCCAGGTTCCCCCCTCATTCTTCAGGCTTAGGGCTGGAGGAA
GCCTTAGACAGCCCAGTCCTACCCCAGACAGGGAAACTGAGGCCTGGAGAGGGC
CAGAAATCACC (SEQ ID NO: 06)
Seq6 
CAAAGACACACAGACCACTTGGGGGGCTGGGTCTACTGTAGTTCTCTATC
TAATATCAGTGGGAGAAAGGCTTGGGATTCATGACCCAGGAGTCATGGCATTCTCA
AGGGGAGGACCTAGATGTGGCAGATCCACAGTACCTTCTCACGAGATGCTTTCCC
 
ACAGGTACGTAGGTGCCATGCAGCCCCGGACGCTCCTCACTGTGGCCCTCTTGGC
TCTCCTGGCATCTGCCCGTAAGATGGTGGGAGTGGGGCTGGGTTTCAGTGGGAGC
TGGTCAGGGGGCCCTGCCCTGAGAAGTGGTGGCTGGGCAGGAGCTGAGGGCTGG
CGTGGCCCAACTCTGCTCTCTCGCAGGAGCTGAAGAGGTAGAGGGATCCTTGCTG
CTGGGCTCTGTACAGGGCTACATGGAACAAGCCTCCAAGACGGTCCAGGATGCGC
TAAGTAGCGTGCAGGAGTCCGATATAGCTGTGGTGGCCAGGTAGGCACACTGGCC
TCCTTTCCTGTGTCTTCTCCTCTCATGCCCTGGTGCTAAGACCCTAGCACAGAGAC
TGTGGTCCACCAAGTTTACCAGGGCCTGGGACCCGCCCGAGCCTCTGGTCCCTCA
TGAGGCCTTTCGGTACTGTGACCAAGATGACAGGCAGGGCAAGCAGCCTTCTCCT
ATCTTCTCTGCTTTTCCTCAGAGCAGCCTGAGGGGCATTTAGCCTCGCTGCAGGTT
TTCTCCTCTGGGTCCTGCGCACACGATGGGAGGCCTACCTGCTCTGGTTTCTGCCT
GTATGCAGGGGGTAGTGGTGGGAAGTGAGGTGGGAAGTTGGGGGTTAGAGGGTG
GGAGTCTGCCCTGTTTCTATCCCTGGGCTCAGTCTGAGGTAGGCATCTTGCCCACT
ATGAAATATCACCCCCTGGCCAATGGCTTTGGCTGACATTTGATAGTTTAGTTGCCT
ATGTAGCAAAAGATGTTGACAGTCTCTATCCCCAGAACAGGCTGTGGGGAGGATA
TTAGATACTC (SEQ ID NO: 08)
seq5-SA-hF9-pA-seq6

GGATGCGCTAAGTAGCGTGCAGGAGTCCGATATAGCTGTGGTGGCCAGGT
AGGCACACTGGCCTCCTTTCCTGTGTCTTCTCCTCTCATGCCCTGGTGCTAAGACC
CTAGCACAGAGACTGTGGTCCACCAAGTTTACCAGGGCCTGGGACCCGCCCGAG
CCTCTGGTCCCTCATGAGGCCTTTCGGTACTGTGACCAAGATGACAGGCAGGGCA
AGCAGCCTTCTCCTATCTTCTCTGCTTTTCCTCAGAGCAGCCTGAGGGGCATTTAG
CCTCGCTGCAGGTTTTCTCCTCTGGGTCCTGCGCACACGATGGGAGGCCTACCTGC
TCTGGTTTCTGCCTGTATGCAGGGGGTAGTGGTGGGAAGTGAGGTGGGAAGTTGG
GGGTTAGAGGGTGGGAGTCTGCCCTGTTTCTATCCCTGGGCTCAGTCTGAGGTAG
GCATCTTGCCCACTATGAAATATCACCCCCTGGCCAATGGCTTTGGCTGACATTTG
ATAGTTTAGTTGCCTATGTAGCAAAAGATGTTGACAGTCTCTATCCCCAGAACAGG
CTGTGGGGAGGATATTAGATACTC (SEQ ID NO: 39)

### Experimental Example 3-2. Production of AAV vector comprising nucleic acid seq5-SA-hF9-pA-seq6

The AAV-ITR plasmid constructed in Experimental Example 3-1, a helper, and a Rep/Cap plasmid were co-transfected into HEK293. After 3 days, virus particles were obtained. Through iodixanol gradient purification, a vector (AAV8) comprising the nucleic acid UHA (seq5)-SA-hF9-DHA (seq6) was finally obtained. Thereafter, the titer was quantified through qPCR on the ITR genome.

### Experimental Example 3-3. Confirmation of hF9 protein expression in mice

The AAV vector comprising the nucleic acid seq5-SA-hF9-pA-seq6 prepared in Experimental Example 3-2 was administered to mice. Specifically, the AAV vector (1×10 ¹¹ vg) was mixed in warmed saline, and 200 µl (5E+8 vg/µL) (2E+13 vg/kg) of the mixture was injected to 8-week-old adult mice through intravenous or intraperitoneal route. From 2 weeks to 14 weeks after injection, plasma of mice was extracted using a heparinized tube through the tail vein or inferior vena cava. The concentration of hFIX expressed in mouse blood was calculated using a 200-fold diluted sample of extracted plasma and an hFIX ELISA kit (Abcam, Cambridge, UK).

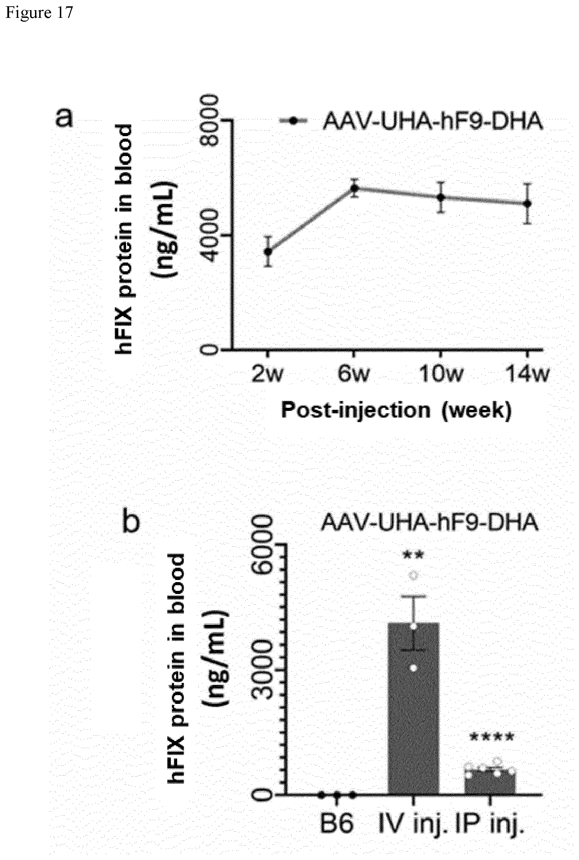

The results obtained by measuring the hFIX concentration in the blood of mice are shown in Figure 17. Figure 17a shows a result obtained by observing the hFIX concentration in blood for a certain period (14 weeks) after injection of the AAV vector (AAV-UHA-hF9-DHA) comprising the nucleic acid (seq5-SA-hF9-pA-seq6) through the intravenous route in mice.

When a vector comprising the nucleic acid (AAV-UHA-hF9-DHA) was injected into mice through the intravenous route, hFIX was observed in the mouse blood (i.e., hFIX was expressed), and it was confirmed that hF9 at a concentration of approximately 5000 ng/ml was maintained until 14 weeks after injection (Figures 17a and 17b).

In addition, even when a vector comprising the nucleic acid (AAV-UHA-hF9-DHA) was injected into mice through the intraperitoneal route, hFIX was observed in the mouse blood (Figure 17b).

## Claims

1. A nucleic acid construct for expressing transgene comprising:
a promoter comprising any one of the sequences of SEQ ID NOs: 01 to 08 and sequences having at least 80% sequence identity thereto; and
a transgene,
wherein the promoter is operably linked to the 5' terminus of the transgene.

2. The nucleic acid construct according to claim 1, wherein the promoter comprises any one of the sequences of SEQ ID NO: 02 and a sequence having at least 80% sequence identity thereto.

3. The nucleic acid construct according to claim 1, wherein the promoter comprises any one of the sequences of SEQ ID NO: 03 and a sequence having at least 80% sequence identity thereto.

4. The nucleic acid construct according to claim 1, wherein the promoter has a length of 1000 nt or 1000 bp or less.

5. The nucleic acid construct according to claim 1, wherein the nucleic acid construct has a length of 10000 nt or 10000 bp or less.

6. The nucleic acid construct according to claim 1, wherein the transgene is a nucleic acid encoding the human FIX protein.

7. The nucleic acid construct according to claim 1, wherein the nucleic acid construct further comprises any one of an upstream homology arm, a downstream homology arm, a splicing acceptor, and a Poly A sequence.

8. A nucleic acid construct for expressing transgene comprising:
a promoter comprising any one of the sequences of SEQ ID NOs: 01 to 08 and sequences having at least 80% sequence identity thereto;
a transgene; and
an upstream homology arm designed to insert the promoter and the transgene into a target site within the genome of a cell; and
a downstream homology arm designed to insert the promoter and the transgene into a target site within the genome of a cell,
wherein the promoter is operably linked to the 5' terminus of the transgene.

9. The nucleic acid construct according to claim 8, wherein on the nucleic acid construct, the promoter, the transgene, the upstream homology arm, and the downstream homology arm are arranged in a 5' to 3' direction in the order of the upstream homology arm, the promoter, the transgene, and the downstream homology arm.

10. The nucleic acid construct according to claim 8, wherein each of the upstream homology arm and the downstream homology arm has a length of 300 nt to 1500 nt or a length of 300 bp to 1500 bp.

11. The nucleic acid construct according to claim 8, wherein the target site is located within a safe harbor gene on the genome.

12. The nucleic acid construct according to claim 11, wherein the safe harbor gene is any one selected from ALB gene, FTL gene, FTH1 gene, ACTB gene, HP gene, APOC3 gene, SOD2 gene, ORM1 gene, AAVS1 gene, Rosa gene, HPRT gene, and CCR5 gene.

13. A nucleic acid construct for expressing transgene comprising:
an upstream homology arm comprising a promoter comprising any one of the sequences of SEQ ID NOs: 01 to 08 and sequences having at least 80% sequence identity thereto;
a transgene; and
a downstream homology arm designed to insert the transgene into a target site on the genome of a cell,
wherein the promoter is operably linked to the 5' terminus of the transgene.

14. The nucleic acid construct according to claim 13, wherein
the upstream homology arm comprises a promoter comprising any one of the sequences of SEQ ID NOs: 01 to 06 and sequences having at least 80% sequence identity thereto, and
the downstream homology arm comprises any one of the sequences of SEQ ID NOs: 07 to 08 and sequences having at least 80% sequence identity thereto.

15. The nucleic acid construct according to claim 13, wherein on the nucleic acid construct, the upstream homology arm, the transgene, and the downstream homology arm are arranged in a 5' to 3' direction in the order of the upstream homology arm, the transgene, and the downstream homology arm.

16. The nucleic acid construct according to claim 13, wherein the target site is located within intron 1 of the APOC3 gene.
